# EUROPÄISCHE PATENTSCHRIFT

(11) **EP 1 320 520 B1**
(45) Veröffentlichungstag und Bekanntmachung des Hinweises auf die Patenterteilung: **11.05.2011**
(21) Anmeldenummer: 01974309.5
(22) Anmeldetag: 01.10.2001
(51) Int. Cl.: C07C 311/64, A61K 31/18

(54) **SULFONYLGUANIDINE**
SULFONYLGUANIDINE
SULFONYLGUANIDINES

(30) Priorität: 30.09.2000 DE 10048716; 12.03.2001 DE 10112068
(43) Veröffentlichungstag der Anmeldung: 25.06.2003
(73) Patentinhaber: Grünenthal GmbH, 52078 Aachen (DE)
(72) Erfinder: GERLACH, Matthias, 63636 Brachttal (DE); URAGG, Heinz, 52223 Stolberg (DE); HAURAND, Michael, 52078 Aachen (DE); PÜTZ, Claudia, Katharina, 52349 Düren (DE); MAUL, Corinna, 52066 Aachen (DE); CHIZH, Boris, Whittlesford, Cambridge CB2 4NW (GB)
(86) Internationale Anmeldenummer: PCT/EP2001/011245
(87) Internationale Veröffentlichungsnummer: WO 2002/030881

(56) Entgegenhaltungen:
- EP-A- 0 195 673
- WO-A-00/15611
- WO-A-99/20599
- US-A- 4 900 740
- CHEMICAL ABSTRACTS, vol. 53, no. 8, 25. April 1959 (1959-04-25) Columbus, Ohio, US; abstract no. 7062i, YU. A. BASKAKOV ET AL.: "Dervatives of hydroxy phenylamines as new herbicides" XP002185314 & KHIM, NAUKA I PROM., Bd. 3, 1958, Seiten 683-4,
- CHEMICAL ABSTRACTS, vol. 53, no. 8, 25. April 1959 (1959-04-25) Columbus, Ohio, US; abstract no. 7062f, A. BARHON ET AL.: "N4-acylated derivatives of sulfaguanidine and of its N1'-acylated derivatives " XP002185315 & ANN. SCI. UNIV. BESANÇON, CHIM. [2], NO. 1, 16 PP. (1955),
- DATABASE CROSSFIRE BEILSTEIN [Online] Beilstein Institut zur Förderung der Chemischen Wissenschaften, Frankfurt am Main, DE; Database accession no. BRN 2899488, 2951283, 2903865 XP002185322 & SUCHORUTSCHKIN ET AL.: J. GEN. CHEM. USSR (ENGL. TRANSL.), Bd. 34, 1964, Seite 1333
- DATABASE CA [Online] CHEMICAL ABSTRACTS SERVICE, COLUMBUS, OHIO, US; Database accession no. 129:175973 XP002185323 & WO 98 34113 A (TREGA BIOSCIENCES INC.) 6. August 1998 (1998-08-06)
- CHEMICAL ABSTRACTS, vol. 121, no. 15, 10. Oktober 1994 (1994-10-10) Columbus, Ohio, US; abstract no. 180208, KIMURA, YUMIKO ET AL.: "Preparation of .alpha.-guanidic acid derivatives " XP002185316 & JP 06 009541 A (SUMITOMO PHARMA) 18. Januar 1994 (1994-01-18)
- CHEMICAL ABSTRACTS, vol. 119, no. 3, 19. Juli 1993 (1993-07-19) Columbus, Ohio, US; abstract no. 27802, KOZAKIEWICZ, IRENA: "Synthesis N-(2,4-dichloro-5-methylbenzenesulfonyl)-S -methylisothiourea and its use for preparation of some novel guanidine derivatives" XP002185317 & ANN. ACAD. MED. GEDANENSIS, Bd. 22, 1992, Seiten 83-8,
- CHEMICAL ABSTRACTS, vol. 115, no. 23, 9. Dezember 1991 (1991-12-09) Columbus, Ohio, US; abstract no. 256577, EGGLESTON, IAN M. ET AL.: "The acid lability of some arginine side-chain sbstitutents related to the 2,2,5,7,8-pentamethylchroman-6-sulfonyl(Pm c) protecting group" XP002185318 & J. CHEM. RES., SYNOP, Nr. 10, 1991, Seiten 286-7,
- CHEMICAL ABSTRACTS, vol. 101, no. 11, 10. September 1984 (1984-09-10) Columbus, Ohio, US; abstract no. 90880, FISCHER, E. ET AL.: "Synthesis and properties of l.lambda4.2,4,6-thiatriazine-1-oxides. Structure of the hydrolysis product of 1-arenesulfonylimino-l.lambda4.,2,4,6-thia triazines" XP002185319 & TETRAHEDRON , Bd. 40, Nr. 2, 1984, Seiten 385-90,
- DATABASE CROSSFIRE BEILSTEIN [Online] Beilstein Institut zur Förderung der Chemischen Wissenschaften, Frankfurt am Main, DE; Database accession no. BRN 4170152, 867787 XP002185324 & SUGIURA ET AL.: YAKUGAKU ZASSHI, Bd. 90, 1970, Seite 711, 714
- CHEMICAL ABSTRACTS, vol. 53, no. 21, 10. November 1959 (1959-11-10) Columbus, Ohio, US; abstract no. 21887h, ROBERT G. GALBREATH ET AL.: "Synthesis of 2-chlorophenothiazine via a Smiles rearrangement" XP002185320 & J. ORG. CHEM., Bd. 23, 1958, Seiten 1804-6,
- CHEMICAL ABSTRACTS, vol. 55, no. 5, 6. März 1961 (1961-03-06) Columbus, Ohio, US; abstract no. 4396e, ALFREDA DANSI ET AL.: "Reactions of N1-(.beta.-phenylethyl)biguanide" XP002185321 & GAZZ. CHIM. ITAL., Bd. 89, 1959, Seiten 1681-6,

## Beschreibung

Die vorliegende Erfindung betrifft Sulfonylguanidine, Verfahren zu deren Herstellung, Arzneimittel enthaltend diese Verbindungen und die Verwendung von Sulfonylguanidinen zur Herstellung von Arzneimitteln zur Behandlung von Schmerz und in anderen Indikationen.

Das cyclische GABA Analoge Gabapentin ist ein klinisch erprobtes Antiepileptikum. Gabapentin zeigt zudem weitere interessante, medizinische relevante Eigenschaften, insbesondere als Analgetikum. Interessant sind deshalb neue Strukturklassen, die Affinität zur Gabapentin-Bindungsstelle aufweisen. Es besteht bei den genannten Indikationen weiterer Bedarf an Substanzen, die in ihren Eigenschaften Übereinstimmungen mit Gabapentin zeigen, beispielsweise in der analgetischen Wirkung.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

Aufgabe der Erfindung war es daher, Strukturen, vorzugsweise neue Strukturen, die Affinität zur Gabapentin-Bindungsstelle und/oder entsprechende physiologische Wirksamkeiten, beispielsweise in Hinblick auf Analgesie, aber auch andere GBP-Indikationen aufweisen, aufzufinden.

Gegenstand der Erfindung ist daher die Verwendung eines Sulfonylguanidins gemäß den allgemeinen tautomeren Formeln I und la, 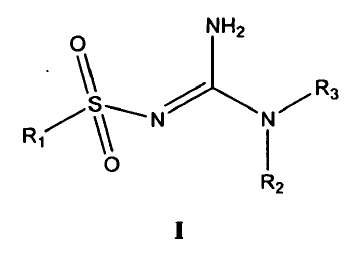 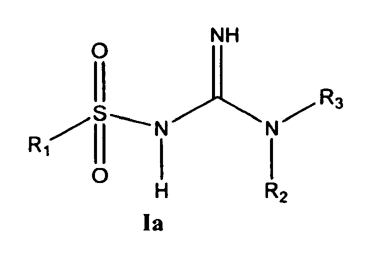 , worin
R¹ ausgewählt ist aus Aryl oder Heteroaryl, jeweils unsubstituiert oder einfach oder mehrfach substituiert
und
R² ausgewählt ist aus C₁₋₁₀-Alkyl, verzweigt oder unverzweigt, gesättigt oder ungesättigt, unsubstituiert; C₃-C₈-Cycloalkyl, gesättigt oder ungesättigt, unsubstituiert; Aryl oder Heteroaryl, jeweils unsubstituiert oder einfach oder mehrfach substituiert; oder über gesättigtes oder ungesättigtes C₁₋₃-Alkyl, S(O)₂ oder NH gebundenem Aryl, C₃₋₉-Cycloalkyl oder Heteroaryl, jeweils unsubstituiert; oder
NR⁴R⁵,
mit R⁴, R⁵ unabhängig voneinander ausgewählt aus H; C₁₋₁₈-Alkyl, verzweigt oder unverzweigt, gesättigt oder ungesättigt, unsubstituiert; C₃-C₈-Cycloalkyl, gesättigt oder ungesättigt, unsubstituiert; Aryl oder Heteroaryl, jeweils unsubstituiert; oder über gesättigtes oder ungesättigtes C₁₋₃-Alkyl gebundenem Aryl, C₃₋₉-Cycloalkyl oder Heteroaryl, jeweils unsubstituiert; oder
R⁴ und R⁵ zusammen: -CH₂CH₂OCH₂CH₂-; -CH₂CH₂N(R⁷)C_{H}2C_{H}2-bilden mit R⁷ ausgewählt aus C₁₋₁₈-Alkyl, verzweigt oder unverzweigt, gesättigt oder ungesättigt, unsubstituiert; C₃-C₈-Cycloalkyl, gesättigt oder ungesättigt, unsubstituiert; Aryl oder Heteroaryl, jeweils unsubstituiert; oder über gesättigtes oder ungesättigtes C₁₋₃-Alkyl gebundenem Aryl, C₃₋₉-Cycloalkyl oder Heteroaryl, jeweils unsubstituiert;
oder
SO₂R⁶
mit R⁶ ausgewählt aus C₁₋₁₈-Alkyl, verzweigt oder unverzweigt, gesättigt oder ungesättigt, unsubstituiert; C₃-C₈-Cycloalkyl, gesättigt oder ungesättigt, unsubstituiert; Aryl oder Heteroaryl, jeweils unsubstituiert; oder über gesättigtes oder ungesättigtes C₁₋₃-Alkyl gebundenem Aryl, C₃₋₉-Cycloalkyl oder Heteroaryl, jeweils unsubstituiert;
und
R³ ausgewählt ist aus H, oder CH₃;
oder
R² und R³ zusammen
-CH₂(CH₂)ₙCH₂- n=2, 3
-CH₂CH₂OCH₂CH₂- oder
-CH₂CH₂N(R⁸)CH₂CH₂- bilden
mit R⁸ ausgewählt aus C₁₋₁₈-Alkyl, verzweigt oder unverzweigt, gesättigt oder ungesättigt, unsubstituiert;
gegebenenfalls in Form ihrer Racemate, ihrer reinen Stereoisomeren insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate; in beiden tautomeren Formen nach Formeln I und Ia, ausschließlich in einer der tautomeren Formen nach Formeln I oder Ia oder auch in Mischungen beider Formen nach Formeln I und Ia, wobei die bevorzugte tautomere Form von Verbindung zu Verbindung z.B. in Abhängigkeit vom Aggregatzustand oder vom gewählten Lösungsmittel variieren kann;
zur Herstellung eines Arzneimittels zur Behandlung von Schmerz, insbesondere von neuropathischem, chronischem oder akutem Schmerz, von Epilepsie und/oder von Migräne
oder
zur Herstellung eines Arzneimittels zur Behandlung von Hyperalgesie und Allodynie, insbesondere thermischer Hyperalgesie, mechanischer Hyperalgesie und Allodynie und Kälte-Allodynie, oder von inflammatorischem oder postoperativem Schmerz
oder
zur Herstellung eines Arzneimittels zur Behandlung von Hitzewallungen, Beschwerden in der Postmenopause, Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Morbus Alzheimer, Morbus Huntington, Morbus Parkinson und Epilepsie; gastrointestinaler Schädigung; von erythromelalgischem oder postpoliomyelitischem Schmerz, trigeminaler oder postherpetischer Neuralgie; oder als Antikonvulsivum, Analgetikum oder Anxiolytikum.

In einer Ausführungsform der Erfindung gilt für die verwendeten Sulfonylguanidine gemäß den allgemeinen tautomeren Formeln I und Ia, dass
R² ausgewählt ist aus C₁₋₁₀-Alkyl, verzweigt oder unverzweigt, gesättigt oder ungesättigt, unsubstituiert; C₃-C₈-Cycloalkyl, gesättigt oder ungesättigt, unsubstituiert; Aryl oder Heteroaryl, jeweils unsubstituiert; oder über gesättigtes oder ungesättigtes C₁₋₃-Alkyl gebundenem Aryl, C₃₋₉-Cycloalkyl oder Heteroaryl, jeweils unsubstituiert.

Diese Substanzen binden an die Gabapentin-Bindungsstelle und zeigen eine ausgeprägte analgetische Wirkung.

Im Sinne dieser Erfindung versteht man unter Alkyl- bzw. Cykloalkyl-Resten gesättigte und ungesättigte (aber nicht aromatische), verzweigt, unverzweigte und cyclische Kohlenwasserstoffe, die unsubstituiert oder ein- oder mehrfach substituiert sein können. Dabei steht C₁₋₂-Alkyl für C1- oder C2-Alkyl, C₁₋₃-Alkyl für C1-, C2- oder C3-Alkyl, C₁₋₄-Alkyl für C1-, C2-, C3- oder C4-Alkyl, C₁₋₅-Alkyl für C1-, C2-, C3-, C4- oder C5-Alkyl, C₁₋₆-Alkyl für C1-, C2-, C3-, C4-, C5- oder C6-Alkyl, C₁₋₇-Alkyl für C1-, C2-, C3-, C4-, C5-, C6- oder C7-Alkyl, C₁₋₈-Alkyl für C1-, C2-, C3-, C4-, C5-, C6-, C7- oder C8-Alkyl, C₁₋₁₀-Alkyl für C1-, C2-, C3-, C4-, C5-, C6-, C7-, C8,- C9- oder C10-Alkyl und C₁₋₈-Alkyl für C1-, C2-, C3-, C4-, C5-, C6-, C7-, C8,- C9-, C10-, C11-, C12-C13-, C14-, C15-, C16-, C17- oder C18-Alkyl Weiter steht C₃₋₄-Cycloalkyl für C3- oder C4-Cycloalkyl, C₃₋₅-Cycloalkyl für C3-, C4- oder C5-Cycloalkyl, C₃₋₆-Cycloalkyl für C3-, C4-, C5- oder C6-Cycloalkyl, C₃₋₇-Cycloalkyl für C3-, C4-, C5-, C6- oder C7-Cycloalkyl, C₃₋₈-Cycloalkyl für C3-, C4-, C5-, C6-, C7- oder C8-Cycloalkyl, C₄₋₅-Cycloalkyl für C4- oder C5-Cycloalkyl, C₄₋₆-Cycloalkyl für C4-, C5- oder C6-Cycloalkyl, C₄₋₇-CYcloalkyl für C4-, C5-, C6- oder C7-Cycloalkyl, C₅₋₆-Cycloalkyl für C5- oder C6-Cycloalkyl und C₅₋₇-Cycloalkyl für C5-, C6- oder C7-Cycloalkyl. Unter den Begriff Cycloalkyl fallen insbesondere ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethytethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, CHF₂, CF₃ oder CH₂OH sowie Pyrazolinon, Oxopyrazolinon, [1,4]Dioxan oder Dioxolan.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen, unter (CH₂)₁₋₄ ist -CH₂-, -CH₂-CH₂-, -CH₂-CH₂-CH₂- und -CH₂-CH₂-CH₂-CH₂- zu verstehen, unter (CH₂)₄₋₅ ist -CH₂-CH₂-CH₂-CH₂- und -CH₂-CH₂-CH₂-CH₂-CH₂- zu verstehen, etc.

Unter einem Aryl-Rest werden Ringsysteme mit mindestens einem armomatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5]thiadiazol, Benzothiazol, Indol, Benzotriazol, Benzodioxolan, Benzodioxan, Carbazol und Chinazolin aufgeführt.

Dabei versteht man im Zusammenhang mit Aryl und Heteroaryl unter substituiert die Substitution des Aryls oder Heteroaryls mit R²³, OR²³ einem Halogen, vorzugsweise F und/oder Cl, einem CF₃, einem CN, einem NO₂, einem NR²⁴R²⁵, einem C₁₋₆-Alkyl (gesättigt), einem C₁₋₆-Alkoxy, einem C₃₋₈-Cycloalkoxy, einem C₃₋₈-Cycloalkyl oder einem C₂₋₆-Alkylen.

Dabei steht der Rest R²³ für H, einen C₁₋₁₀-Alkyl-, vorzugsweise einen C₁₋₆-Alkyl-, einen Aryl- oder Heteroaryl- oder für einen über C₁₋₃-Alkyl, gesättigt oder ungesättigt, oder eine C₁₋₃-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen,
die Reste R²⁴ und R²⁵, gleich oder verschieden, für H, einen C₁₋₁₀-Alkyl-, vorzugsweise einen C₁₋₆-Alkyl-, einen Aryl-, einen Heteroaryl- oder einen über C₁₋₃-Alkyl, gesättigt oder ungesättigt, oder eine C₁₋₃-Alkylen-Gruppe gebundenen Aryl- oder Heteroaryl-Rest stehen, wobei diese Aryl- und Heteroarylreste nicht selbst mit Aryl- oder Heteroaryl-Resten substituiert sein dürfen,
oder die Reste R²⁴ und R²⁵ bedeuten zusammen CH₂CH₂OCH₂CH₂, CH₂CH₂NR²⁶CH₂CH₂ oder (CH₂)₃₋₆, und
der Rest R²⁶ für H, einen C₁₋₁₀-Alkyl-, vorzugsweise einen C₁₋₆-Alkyl-, einen Aryl-, oder Heteroaryl- Rest oder für einen über C₁₋₃-Alkyl, gesättigt oder ungesättigt, oder eine C₁₋₃-Alkylen-Gruppe gebundenen Aryl- oder Heteroaryl-Rest steht, wobei diese Aryl- und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter physiologisch verträgliche Salze mit Kationen oder Basen und physiologisch verträgliche Salze mit Anionen oder Säuren.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit NH₄⁺, insbesondere aber (Mono-) oder (Di-) Natrium-, (Mono-) oder (Di-) Kalium-, Magnesium- oder Calzium-Salze.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure. Besonders bevorzugt ist das Hydrochlorid-Salz.

Alle hier vorangehend aufgeführten und für die Verwendung definierten Substanzen verdrängen Gabapentin von seiner - auch in der Wissenschaft bisher noch unbekannten - Bindungsstelle. Das impliziert aber, daß die erfindungsgemäßen Substanzen an der gleichen Bindungsstelle binden und über sie physiologisch wirken werden, vermutlich mit dem gleichen Wirkungsprofil wie Gabapentin. Daß diese Annahme der gleichen Wirkung bei gleicher Bindungsstelle auch zutrifft, wird durch die analgetische Wirkung bewiesen. So verdrängen die erfindungsgemäßen Verbindungen nicht nur Gabapentin von seiner Bindungsstelle sondern wirken auch - wie Gabapentin - deutlich analgetisch. Entsprechend ist der Gegenstand der Erfindung die Verwendung der genannten und definierten Thioaminosäuren in den vorangehend genannten Indikationen, in denen Gabapentin wirkt, also insbesondere in der Schmerztherapie, bei Epilepsie oder Migräne, aber speziell auch im neuropathischen Schmerz also Hyperalgesie und Allodynie und den anderen Gabapentin Indikationen.

Gabapentin ist ein bekanntes Antiepileptikum mit antikonvulsiver Wirkung. Neben dieser wird Gabapentin auch in verschiedenen anderen Indikation eingesetzt, unter anderem von behandelnden Ärzten bei Migräne und bipolaren Störungen sowie Hitzewallungen (z.B. in der Postmenopause) verschrieben (M. Schrope, Modern Drug Discovery, September 2000, S. 11). Andere Indikationenen, in denen Gabapentin ein therapeutisches Potential zeigt, wurden während der Humanstudien und im klinischen Gebrauch identifiziert (J.S. Bryans, D.J. Wustrow; "3-Substituted GABA Analogs with Central Nervous System Activity: A Review" in Med. Res. Rev. (1999), S. 149-177). In diesem Übersichtsartikel wird detailliert die Wirkung von Gabapentin aufgelistet. So ist Gabapentin wirksam in der Behandlung chronischer Schmerzen und Verhaltensstörungen. Insbesondere sind aufgeführt: Antikonvulsive und antiepileptische Wirkungen, der Einsatz gegen chronischen, neuropathischen Schmerz, insbesondere thermische Hyperalgesie, mechanische Allodynie, Kälte-Allodynie. Weiter wirkt es gegen durch Nervenschädigungen ausgelöste Neuropathie, insbesondere eben neuropathischen Schmerz, wie auch inflammatorischen und postoperativen Schmerz erfolgreich. Gabapentin ist auch erfolgreich bei antipsychotischen Effekten insbesondere als Anxiolytikum. Weitere überprüfte Indikationen umfassen: Amyotropische Laterale Sklerose (ALS), Reflex Sympasthetic Dystrophy (RSD), Spastische Lähmung, Restless Leg Syndrom, Behandlung von Symptomen und Schmerz aufgrund von Multipler Sklerose, erworbener Nystagmus, Behandlung der Symptome der Parkinsonschen Krankheit, der schmerzvollen diabetischen Neuropathie und psychatrischer Störungen, z.B. bipolare Störungen, Stimmungsschwankungen, manisches Verhalten. Weiter erfolgreich war der Einsatz von Gabapentin bei erythromelalgischem Schmerz, postpoliomyelitisem Schmerz, trigeminaler Neuralgie und postherpetischer Neuralgie (Bryans und Wustrow (1999), a.a.O.). Allgemein bekannt und auch dem genannten Übersichtsartikel anhand der Beispiele zu entnehmen ist auch die allgemeine Wirksamkeit in neurodegenerativen Erkrankungen. Solche Neurodegenerativen Erkrankungen sind z.B. Morbus Alzheimer, Morbus Huntington, Morbus Parkinson und Epilepsie. Bekannt ist auch die Wirksamkeit von Gabapentin bei gastrointestinalen Schädigungen.

In einer bevorzugten Ausführungsform der Erfindung gilt für die verwendeten Sulfonylguanidine gemäß den allgemeinen tautomeren Formeln I und la, dass
R² ausgewählt ist aus über gesättigtes oder ungesättigtes C₁₋₃-Alkyl, , gebundenem Aryl oder Heteroaryl, jeweils unsubstituiert, wobei diese vorzugsweise über eine -CH₂-Gruppe gebunden sind.
Dabei ist das in R² über C₁₋₃-Alkyl gebundene Heteroaryl vorzugsweise ausgewählt aus Pyridinyl, Thiophenyl, Furanyl oder Pyrimidinyl, vorzugsweise Pyridinyl, jeweils unsubstituiert.

In einer bevorzugten Ausführungsform der Erfindung sind die erfindungsgemäß verwendeten Sulfonylguanidine ausgewählt aus der folgenden Gruppe:
- *N*-{Amino-[pyridin-2ylmethyl)-amino]-methylen}-4-methylbenzolsulfonamid **(1)**
- *N*-[Amino-(benzylamino-methylen)]-4-methylbenzolsulfonamid **(2)**
- *N*-(Amino-morpholin-4yl-methylen)-4-methylbenzolsulfonamid **(3)**
- *N*-(Amino-cyclohexylaminomethylen)-4-methylbenzolsulfonamid **(4)**
- *N*-(Amino-phenylaminomethylen)-4-methylbenzolsulfonamid **(5)**
- *N*-[(Amino-4-methoxybenzylamino)methylen]-4-methylbenzolsulfonamid **(6)**
- *N*-[Amino-(naphthalin-2-ylamino)methylen]-4-methylbenzolsulfonamid **(7)**
- *N*-[Amino-(4-methyl-piperazin-1-ylamino)-methylen]-4-methylbenzolsulfonamid **(8)**
- *N*-[Amino-(*N*'-pyridin-2-yl-hydrazino)-methylen]-4-methylbenzolsulfonamid **(9)**
- *N*-[Amino-propylamino-methylen]-4-methylbenzolsulfonamid **(10)**
- *N*-(Amino-butylamino-methylen)-4-methylbenzolsulfonamid **(11)**
- *N*-[Amino-piperidin-methylen]-4-methylbenzolsulfonamid **(12)**
- *N*-[Amino-(N-methyl-N-phenyl-amino)-methylen]-4-methylbenzolsulfonamid **(13)**
- *N*-[Amino-phenethylamino-methylen]-4-methylbenzolsulfonamid **(14)**
- *N*-[Amino-sec-butylamino-methylen]-4-methylbenzolsulfonamid **(15)**
- *N*-[Amino-(*N*'-tosyl-2-yl-hydrazino)-methylen]-4-methylbenzolsulfonamid **(16)**
- *N*-[Amino-(2H-pyrazol-3-ylamino)-methylen]-4-methylbenzolsulfonamid **(17)**
- *N*-(Amino-pyrrolidin-1-yl-methylen)-4-methylbenzolsulfonamid **(18)**
- *N*-[Amino-(naphthalin-1-ylamino)-methylen]-4-methylbenzolsulfonamid **(19)**
- *N*-[Amino-(morpholin-4-ylamino)-methylen]-4-methylbenzolsulfonamid **(20)**
- N-{Amino-[pyridin-2ylmethyl)-amino]-methylen}-chlorbenzolsulfonamid **(21)**
- N-{Amino-[pyridin-4ylmethyl)-amino]-methylen}-4-methylbenzolsulfonamid **(22)**
- N-{Amino-[pyridin-3ylmethyl)-amino]-methylen}-4-methylbenzolsulfonamid **(23)**
- N-[Amino-(pyridin-3ylamino)-methylen]-4-methylbenzolsulfonamid **(24)**
- *N*-(Amino-pyrrolidin-1-yl-methylen)-4-chlorbenzolsulfonamid **(25)**
- *N*-(Amino-pyrrolidin-1-yl-methylen)-benzolsulfonamid **(26)**
- *N*-(Amino-pyrrolidin-1-yl-methylen)-2-nitrobenzolsutfonamid **(27)**
- *N*-[Amino-(morpholin-4-ylamino)-methylen]-4-chlorbenzolsulfonamid **(28)**
- *N*-[Amino-(morpholin-4-ylamino)-methylen]-benzolsulfonamid **(29)**
- Naphthalin-1-sulfonsäureamino-(morpholin-4-ylamino)-methylenamid **(30)**
- N-{Amino-[(pyridin-2-ylmethyl)-amino]-methylen}-4-brom-benzolsulfonamid **(32)**
- N-{Amino-[(pyridin-2-ylmethyl)-amino]-methylen}-3,4-dichlor-benzolsulfonamid **(33)**
- N-{Amino-[(pyridin-2-ylmethyl)-amino]-methylen}-4-isopropyl-benzolsulfonamid **(34)**
- N-{Amino-[(pyridin-2-ylmethyl)-amino]-methylen}-4-iod-benzolsulfonamid **(35)**
- N-{Amino-[(pyridin-2-ylmethyl)-amino]-methylene}-benzolsulfonamid **(36)**
- Naphthalin-2-sulfonsäure-amino-[(pyridin-2-ylmethyl)-amino]-methylenamid **(37)**
- 1-Methyl-1H-imidazol-4-sulfonsäure-amino-[(pyridin-2-ylmethyl)-amino]-methylenamid **(38)**
- N-[4-({Amino-[(pyridin-2-ylmethyl)-amino]-methylen}-sulfamoyl)-phenyl]-acetamid **(39)**
- N-{Amino-[(pyridin-2-yimethyl)-amino]-methylen}-4-fluor-benzolsulfonamid **(40)**
- N-{Amino-[(pyridin-2-ylmethyl)-amino]-methylen}-2,4,6-trimethyl-benzolsulfonamid **(41)**
- N-{Amino-[(pyridin-2-ylmethyl)-amino]-methylen}-4-propyl-benzolsulfonamid **(42)**
- N-{Amino-[(pyridin-2-ylmethyl)-amino]-methylen}-4-methoxy-benzolsulfonamid **(43)**
- Naphthalin-1-sulfonsäure-amino-[(pyridin-2-ylmethyl)-amino]-methylenamid **(44)**
- N-{Amino-[(pyridin-2-ylmethyl)-amino]-methylen}-3-methyl-benzolsulfonamid **(45)**
- Thiophen-2-sulfonsäure-amino-[(pyridin-2-ylmethyl)-amino]-methylenamid **(46)**
- Quinolin-8-sulfonsäure-amino-[(pyridin-2-ylmethyl)-amino]-methylenamid **(47)**
- 5-Chlor-3-methyl-benzo[b]thiophen-2-sulfonsäure-amino-[(pyridin-2-ylmethyl)-amino]-methylenamid **(48)**
- N-{Amina-[(pyridin-2-ylmethyl)-amino]-methylen}-4-tert-butyl-benzolsulfonamid **(49)**
- N-{Amino-[(pyridin-2-ylmethyl)-amino]-methylen}-4-butyl-benzolsulfonamid **(50)**
- N-{Amino-[(pyrimidin-2-ylmethyl)-amino]-methylen}-4-tert-butyl-benzolsulfonamid **(51)**
- N-{Amino-[(furan-2-ylmethyl)-amino]-methylen}-4-butyl-benzolsutfonamid **(52)**
- N-{Amino-[(furan-2-ylmethyl)-amino]-methylen}-4-methyl-benzolsulfonamid **(53)**
- N-{Amino-[(furan-2-ylmethyl)-amino]-methylen}-4-isopropyl-benzolsulfonamid **(54)**
- N-{Amino-[(furan-2-ylmethyl)-amino]-methylen}-4-propyl-benzolsulfonamid **(55)**
- N-{Amino-[(furan-2-ylmethyl)-amino]-methylen}-4-tert-butyl-benzolsulfonamid **(56)**
- N-{Amino-[(thiophen-2-ylmethyl)-amino]-methylen}-4-butyl-benzolsulfonamid **(57)**
- N-{Amino-[(thiophen-2-ylmethyl)-amino]-methylen}-4-propyl-benzolsulfonamid **(58)**
- N-{Amino-[(thiophen-2-ylmethyl)-amino]-methylene)-4-isopropyl-benzolsulfonamid **(59)**
- N-{Amino-[(thiophen-2-ylmethyl)-amino]-methylen}-4-tert-butyl-benzolsulfanamid **(60)**
- N-{Amino-[(thiophen-2-ylmethyl)-amino]-methylen}-4-chlor-benzolsulfonamid **(61)**
- N-{Amino-[(thiophen-2-ylmethyl)-amino]-methylen}-4-methyl-benzolsulfonamid **(62)**
- N-{Amino-[(furan-2-ylmethyl)-amino]-methylen}-4-chlor-benzolsulfonamid **(63)**
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen; oder in Form ihrer Salze, insbesondere der physiologisch verträglichen Salze, vorzugsweise des Hydrochlorid-oder Natriumsalzes; oder in Form ihrer Solvate, insbesondere der Hydrate; in beiden tautomeren Formen nach Formeln I und la, ausschließlich in einer der tautomeren Formen nach Formeln I oder Ia oder auch in Mischungen beider Formen nach Formeln I und la, wobei die bevorzugte tautomere Form von Verbindung zu Verbindung z.B. in Abhängigkeit vom Aggregatzustand oder vom gewählten Lösungsmittel variieren kann.

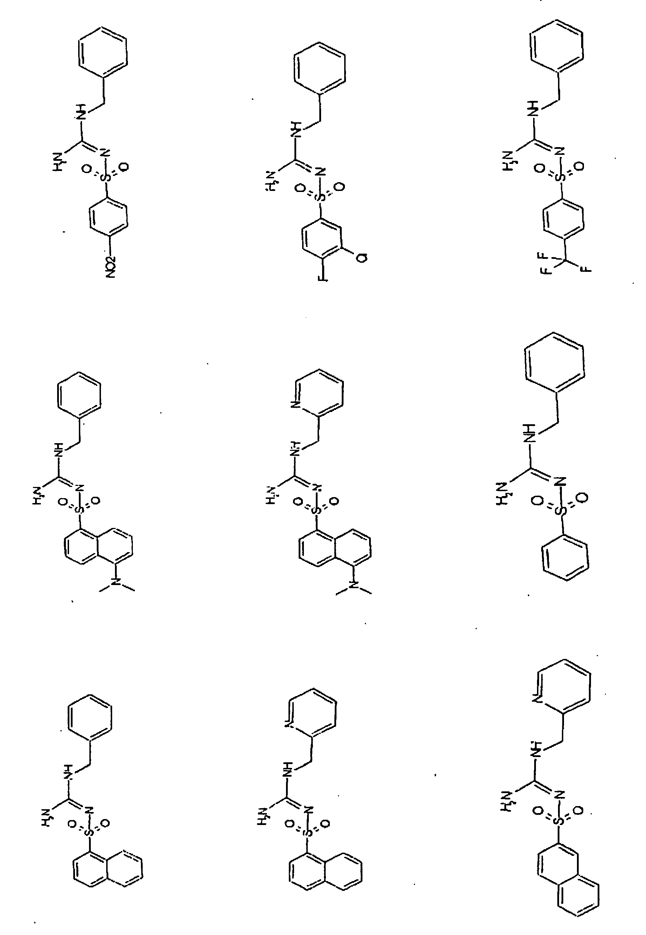

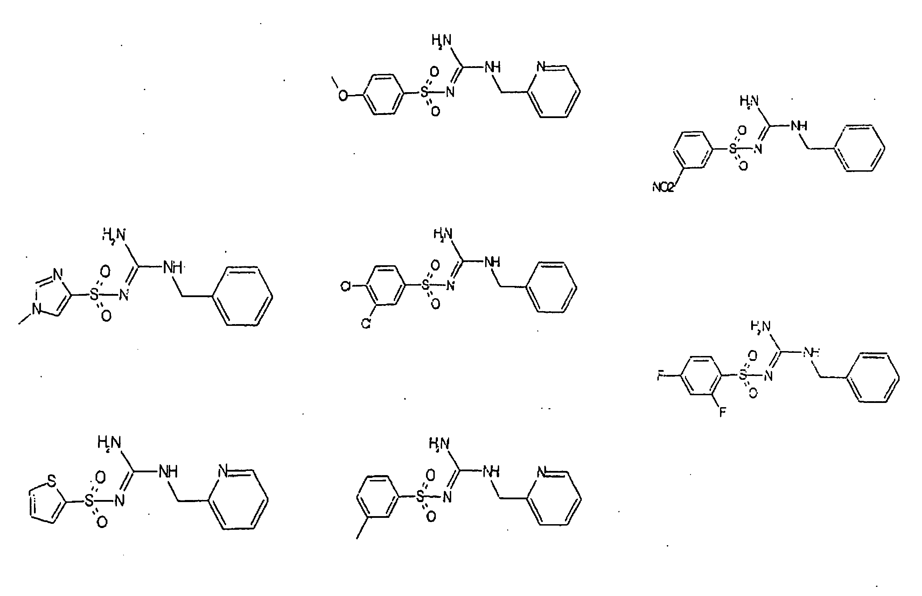

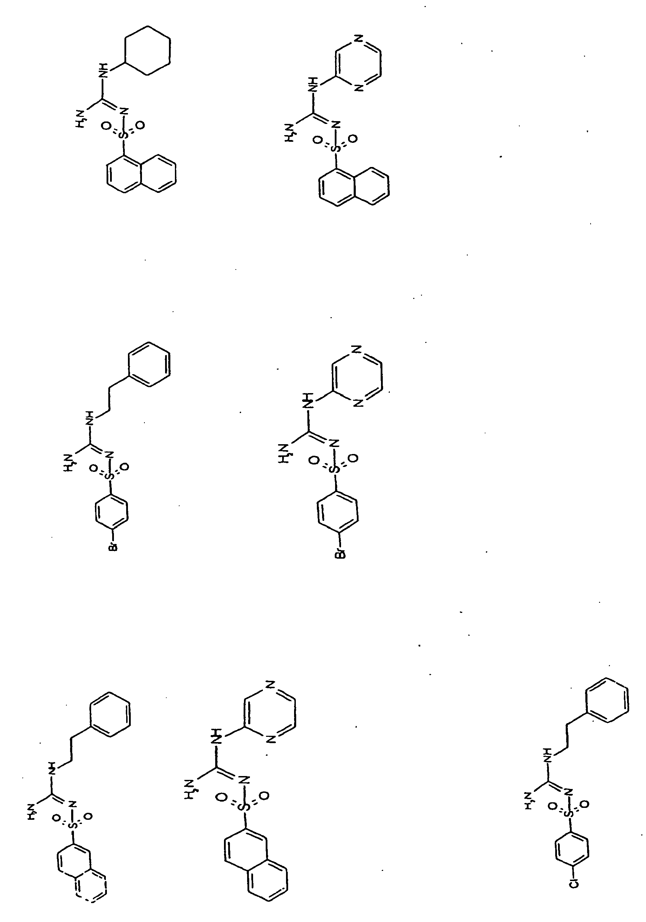

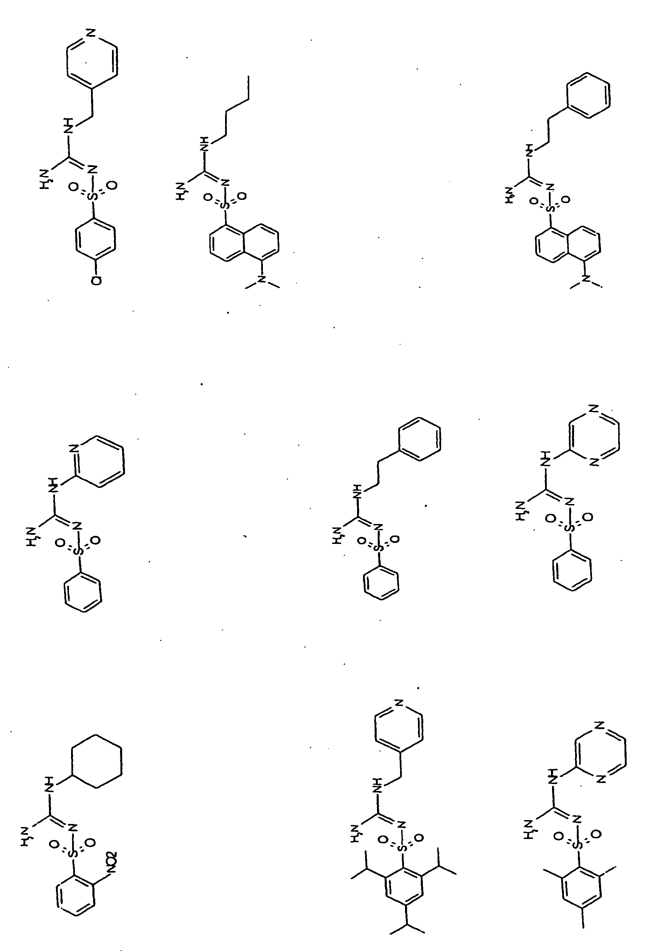

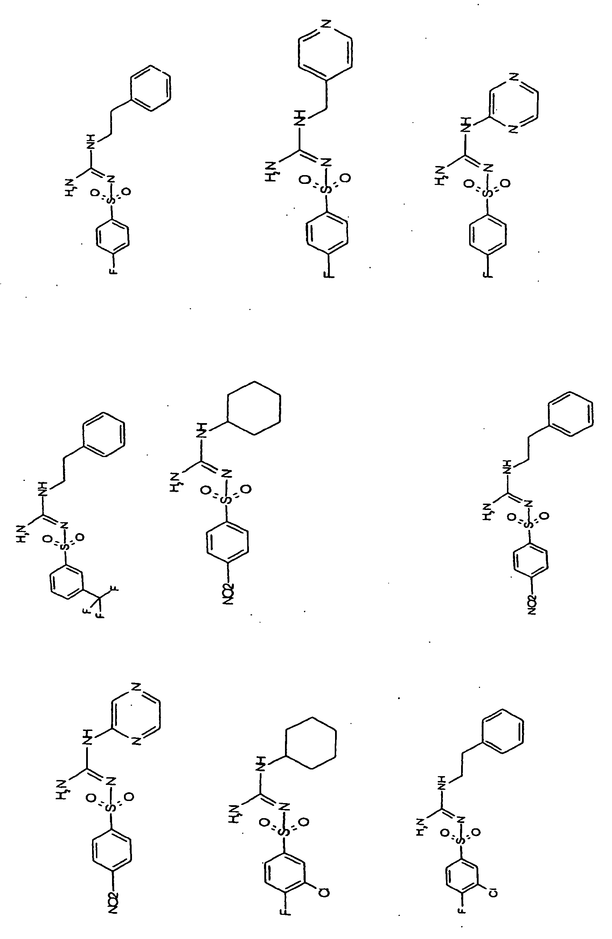

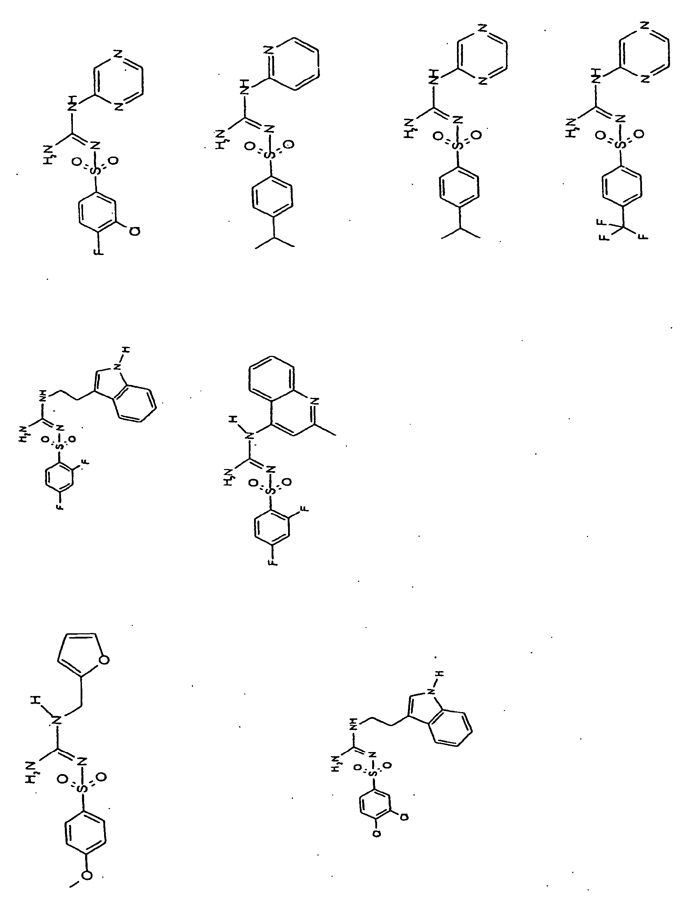

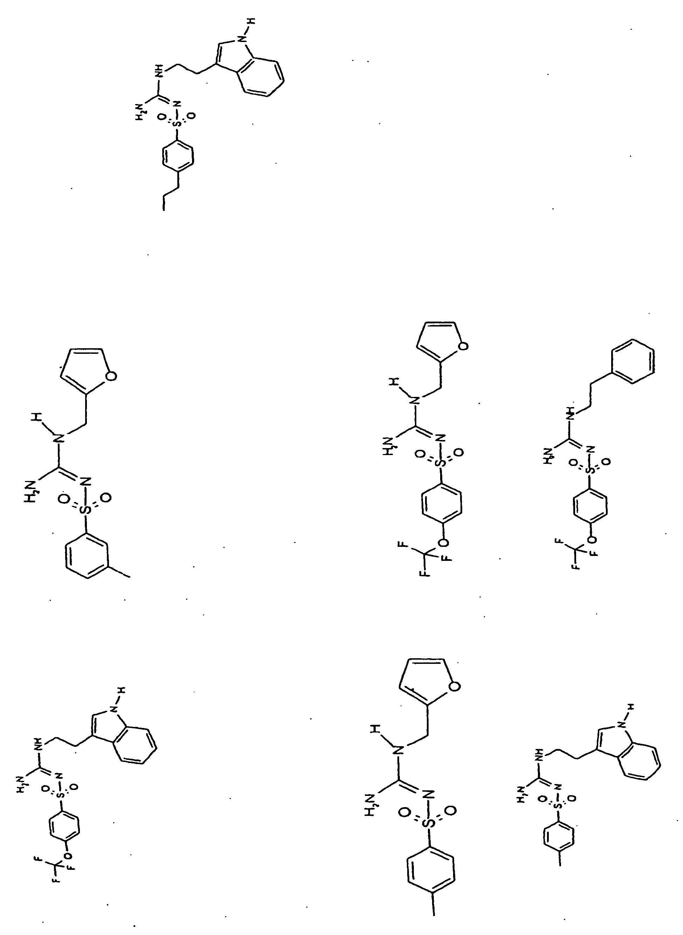

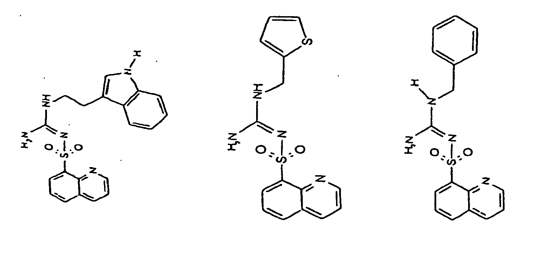

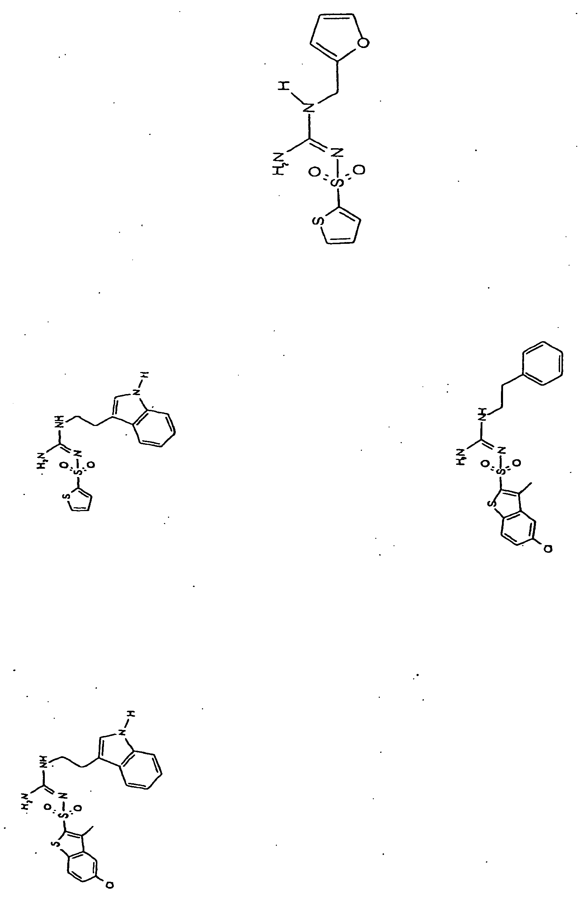

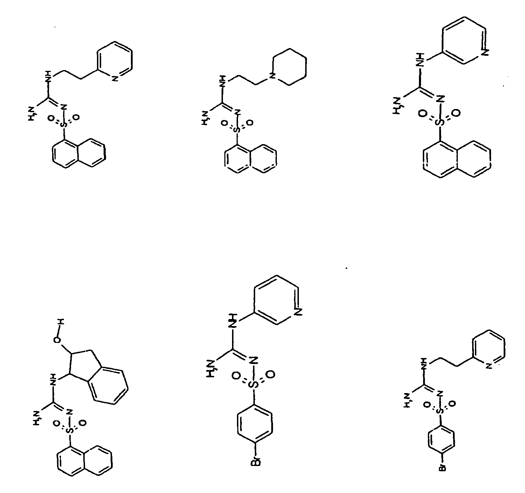

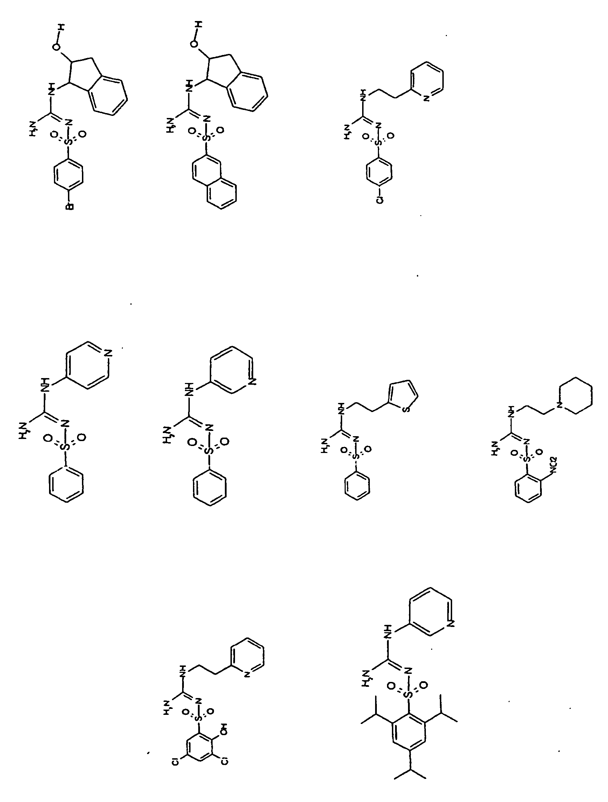

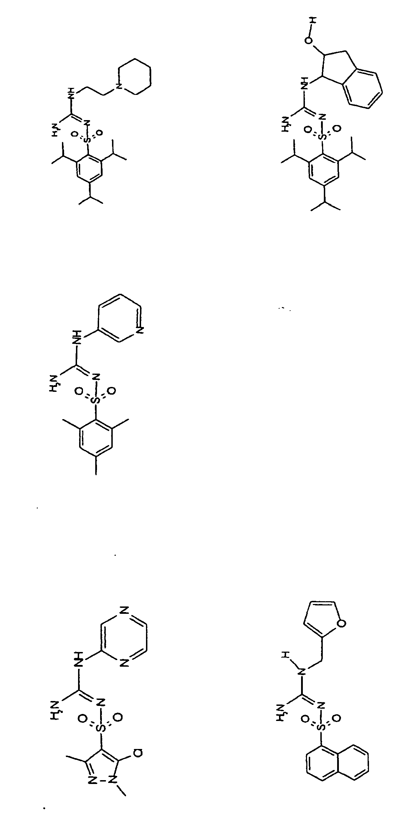

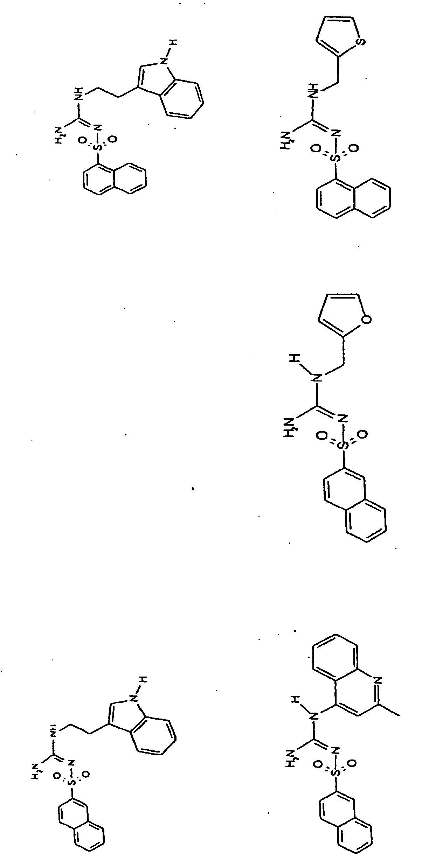

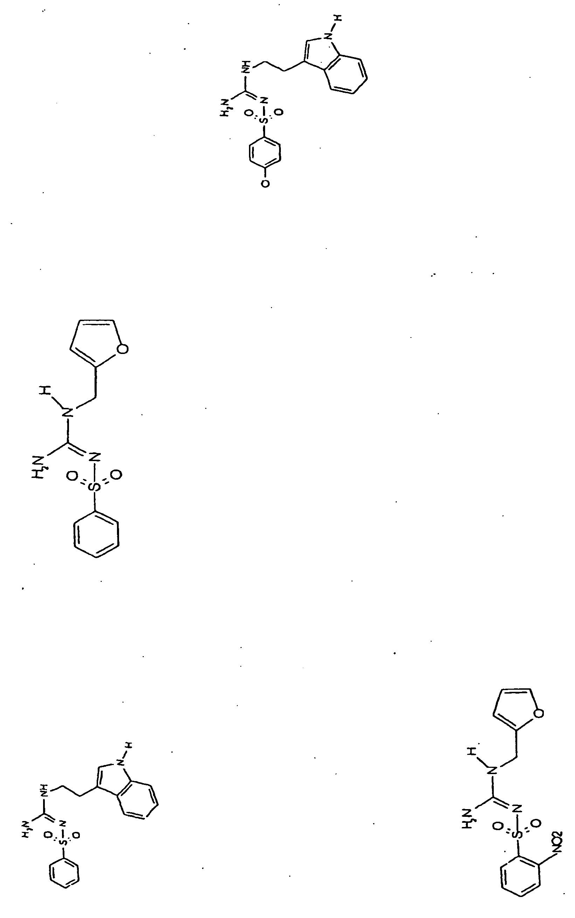

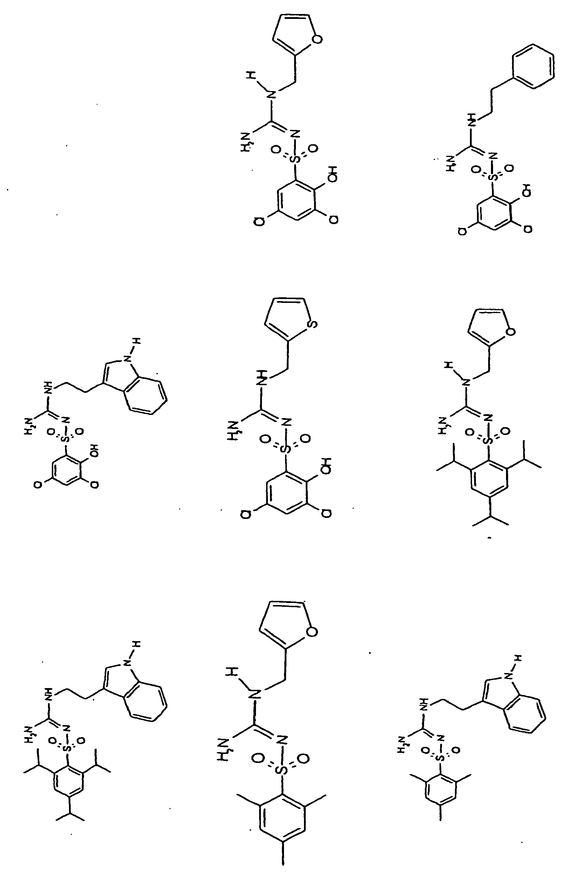

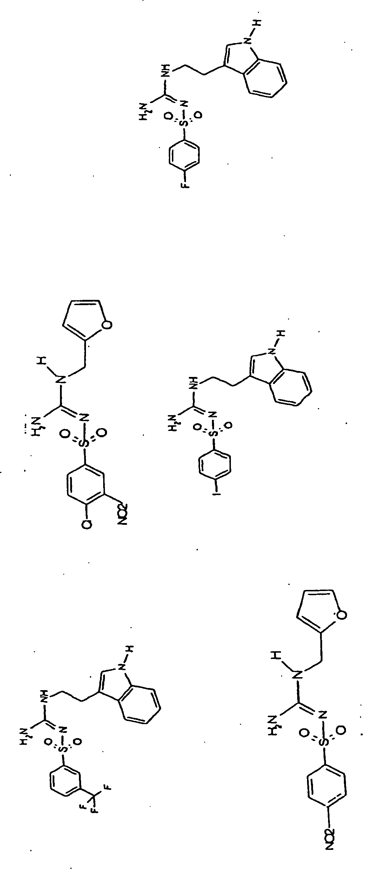

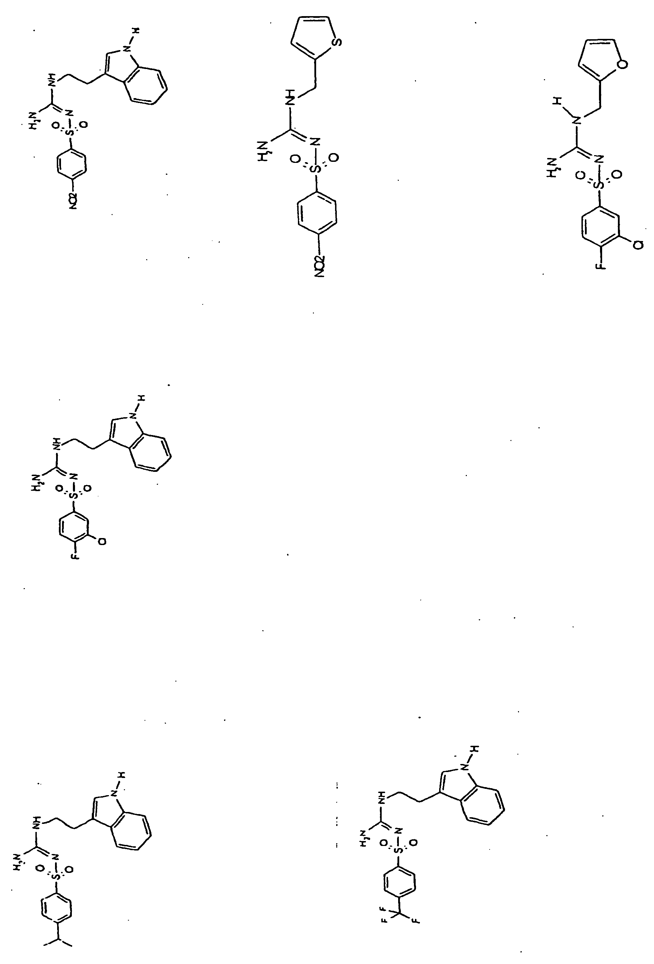

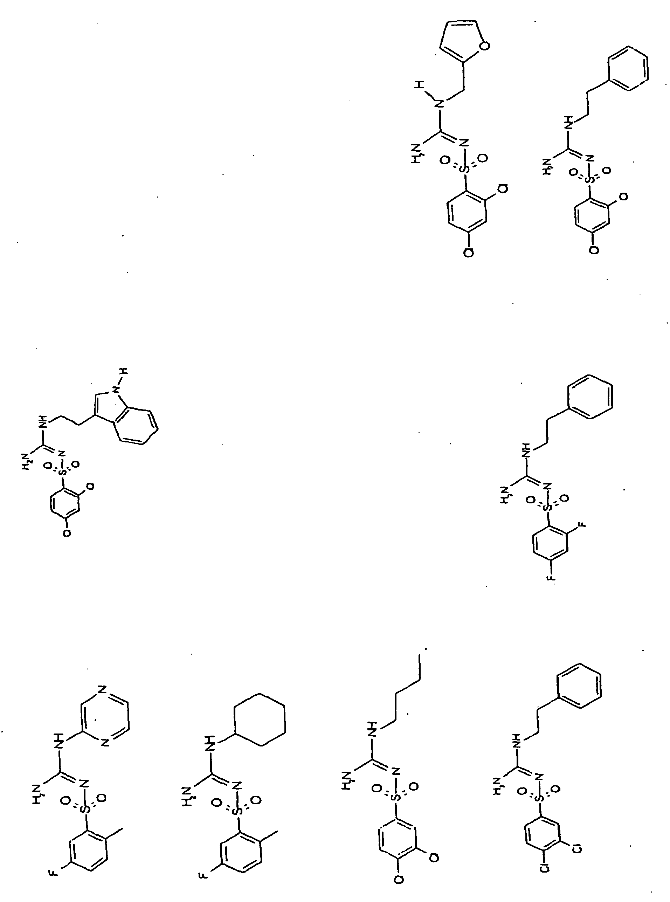

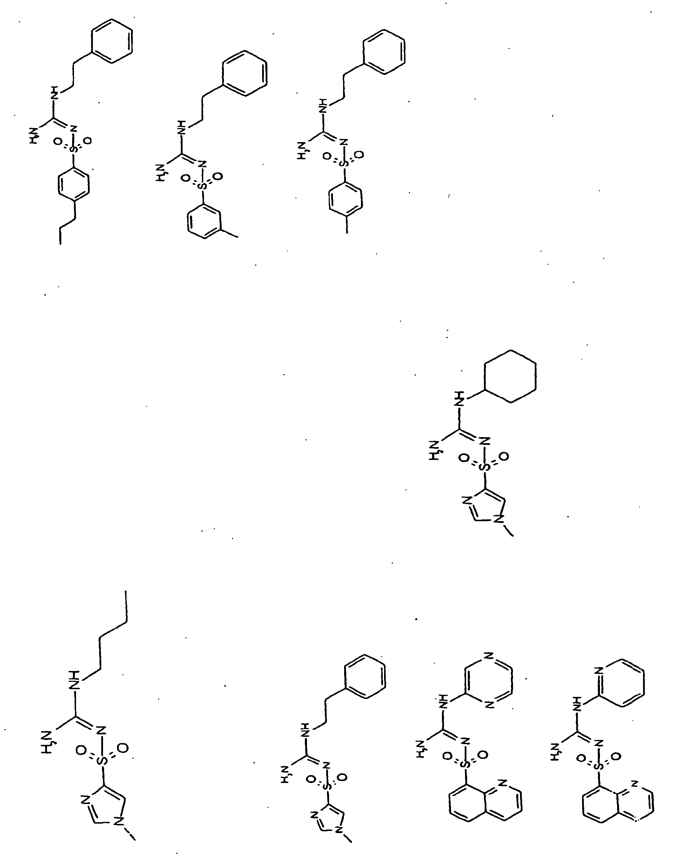

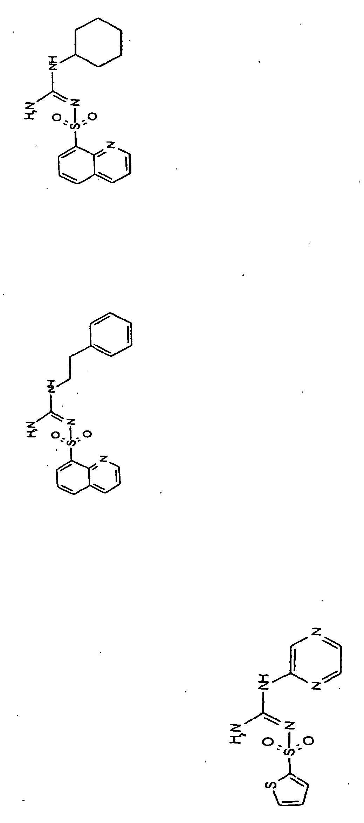

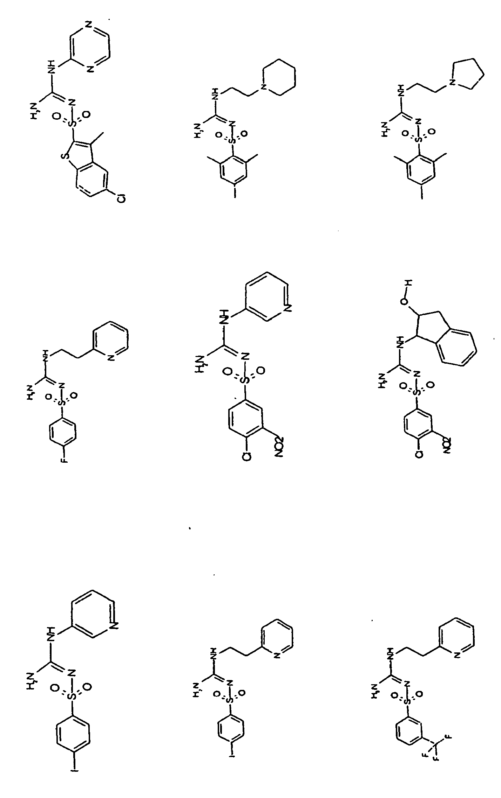

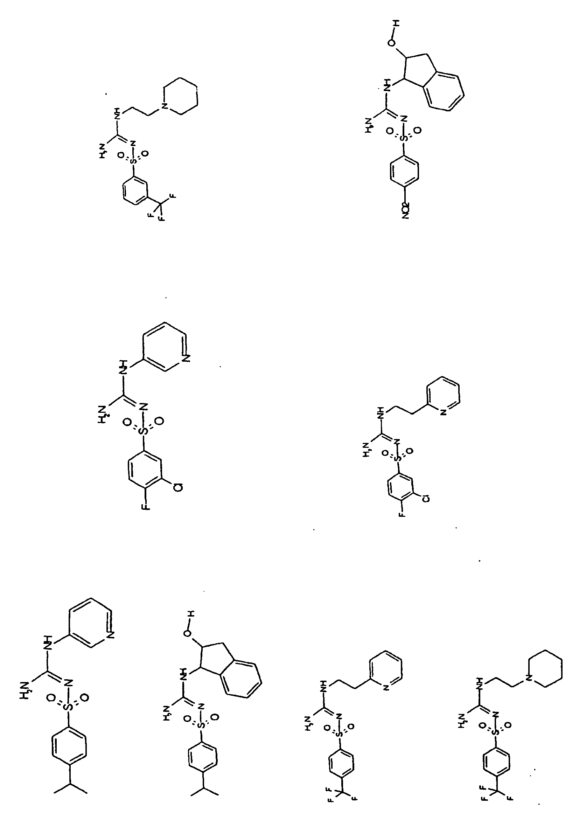

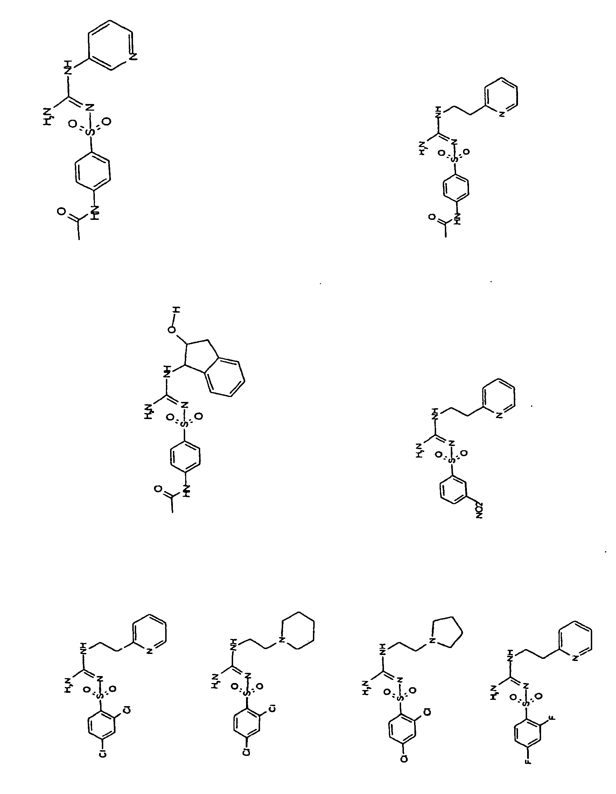

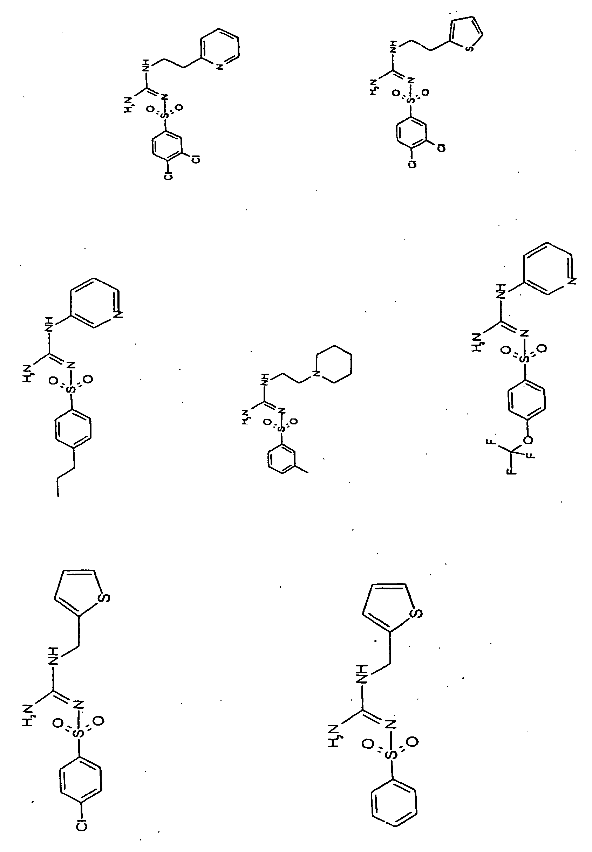

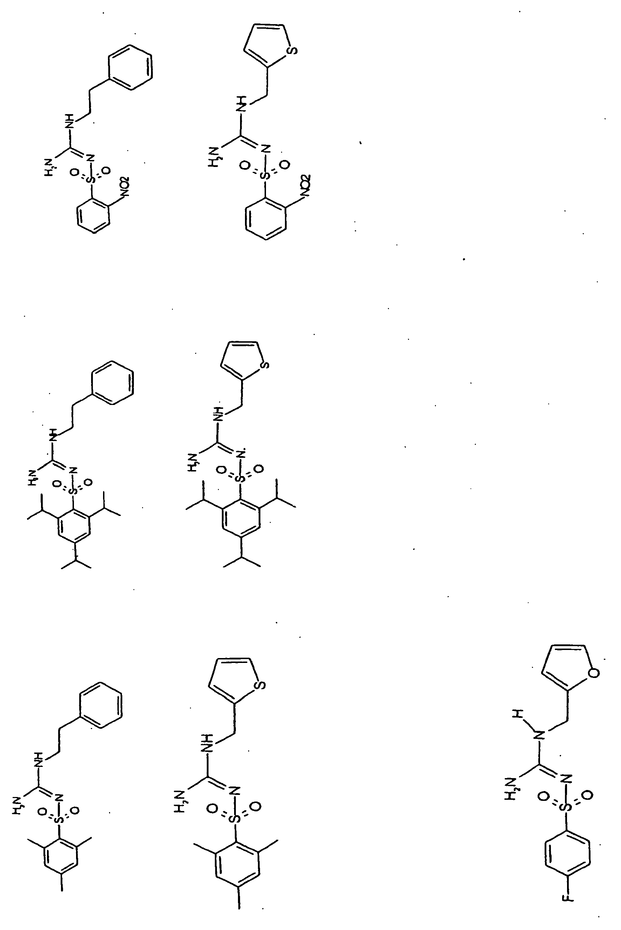

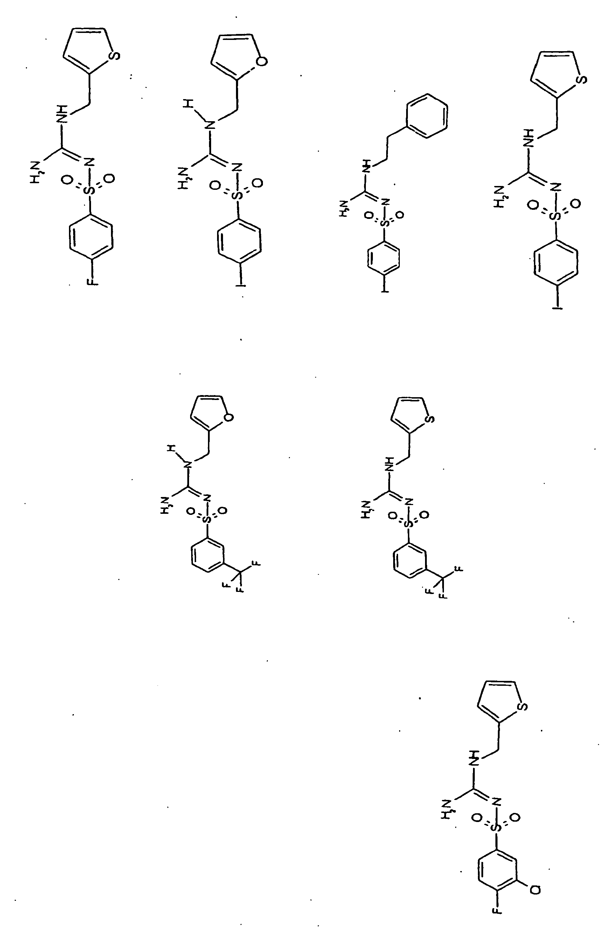

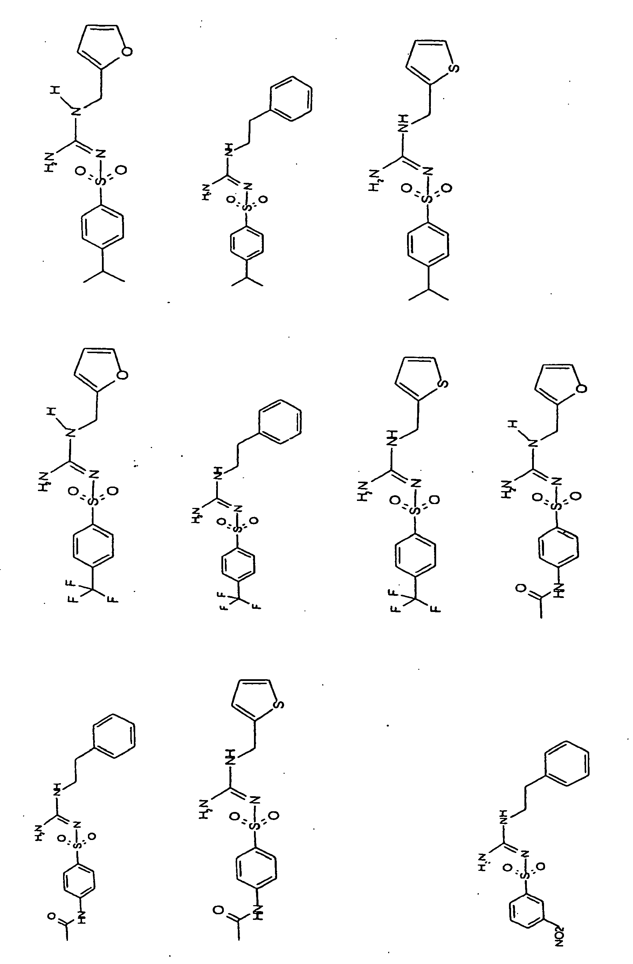

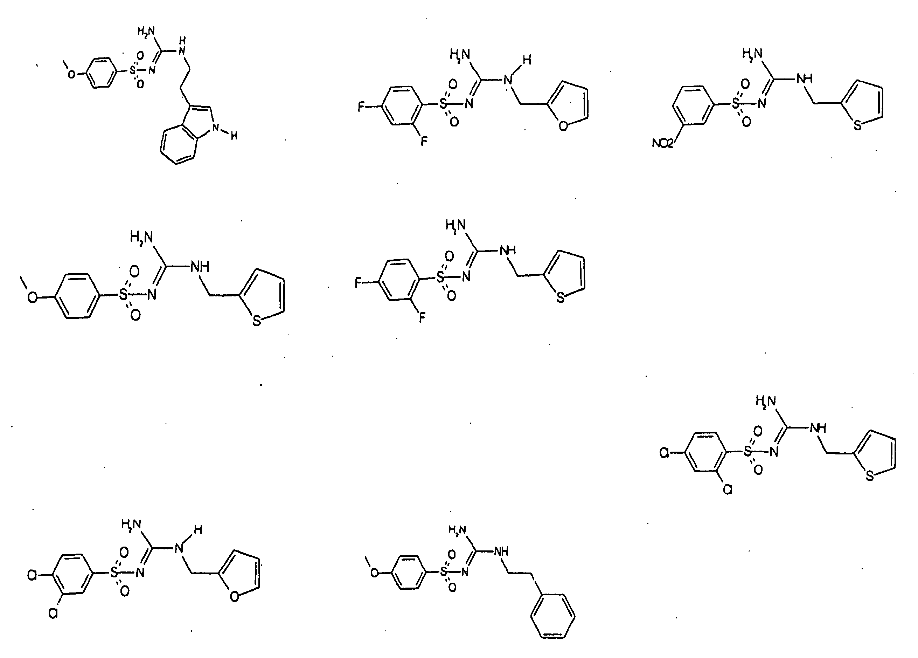

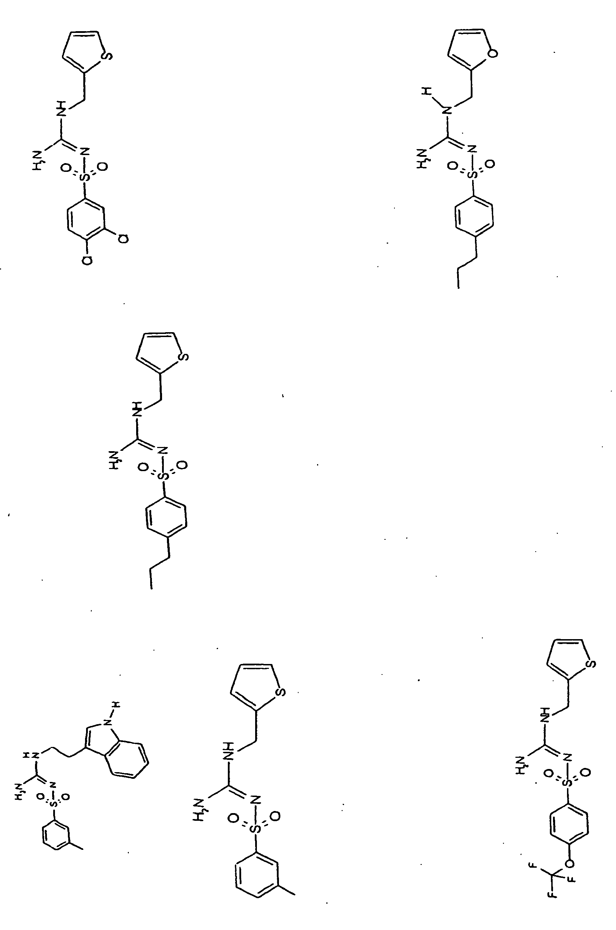

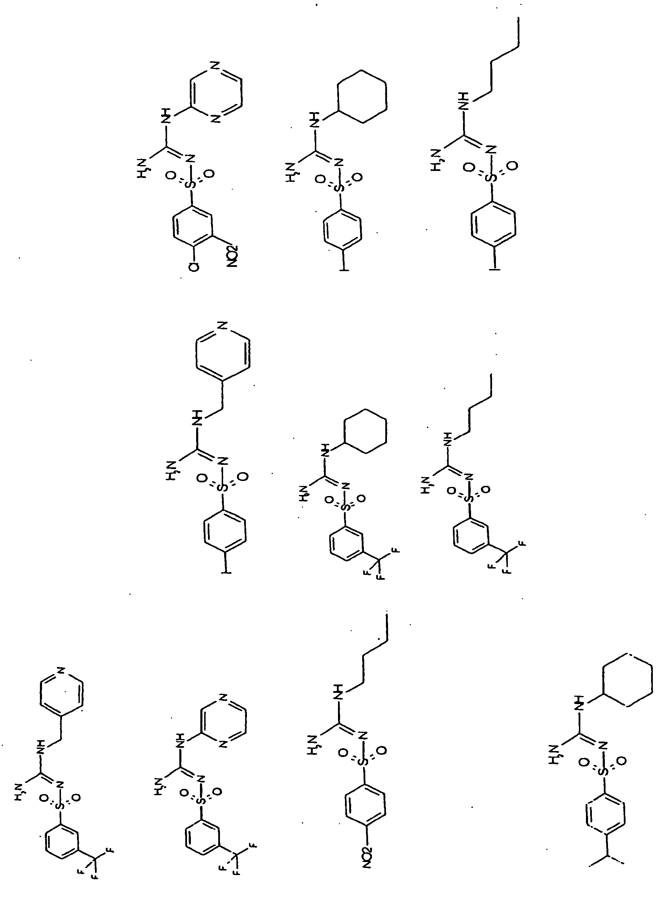

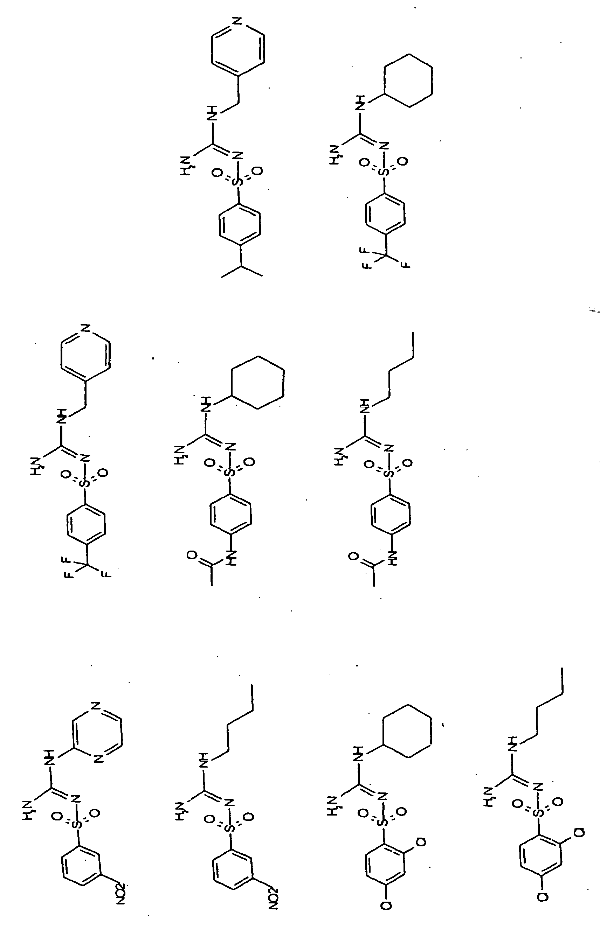

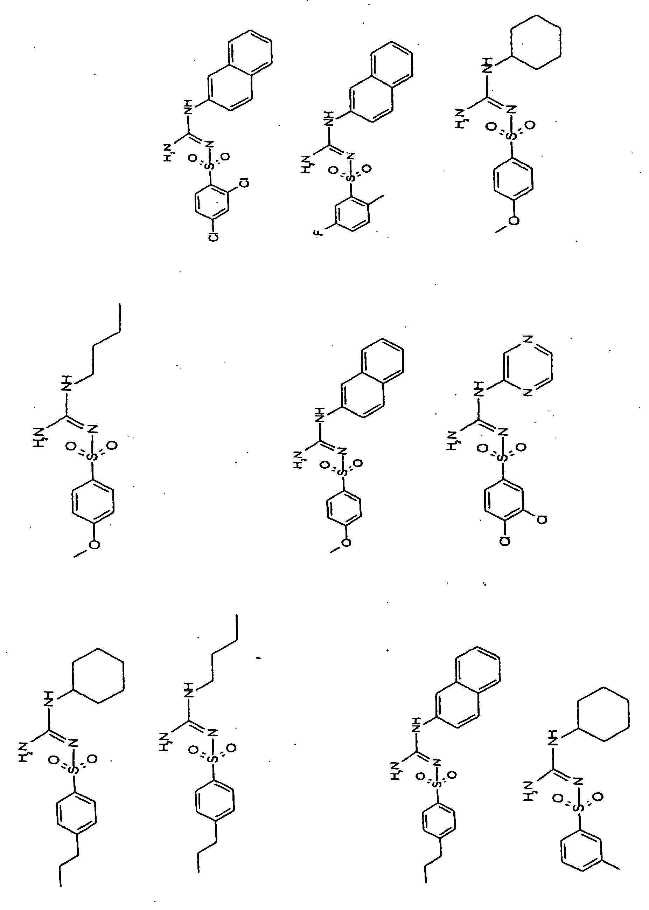

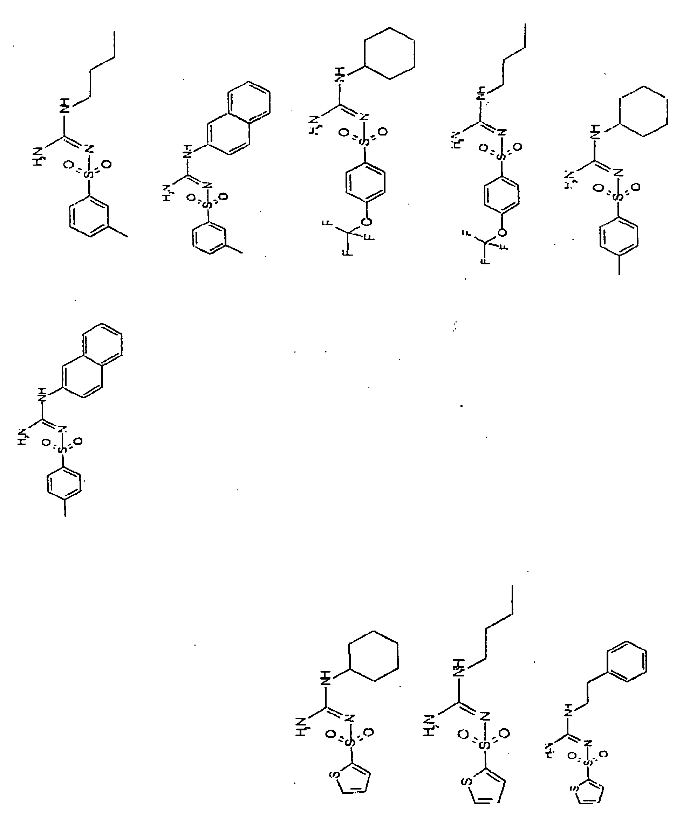

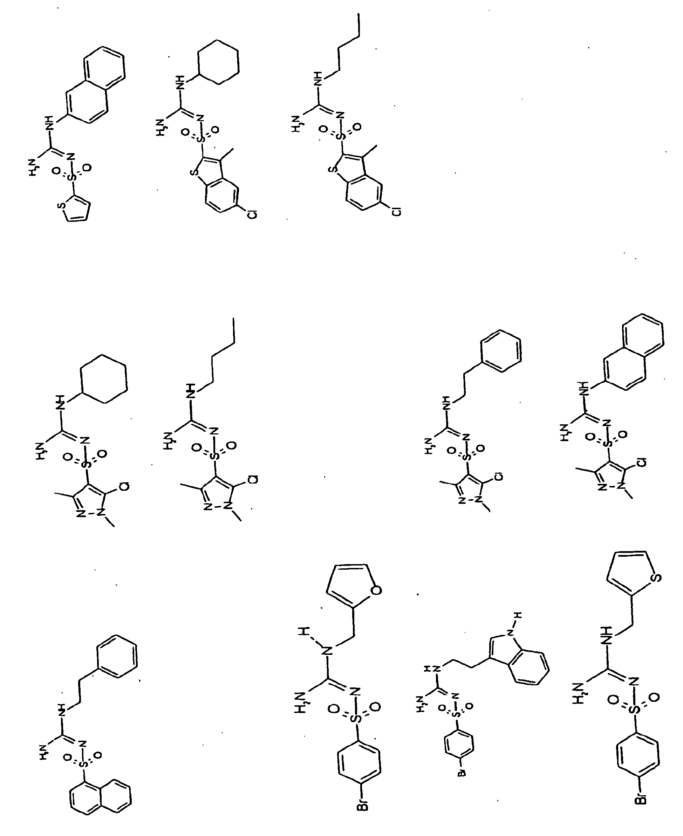

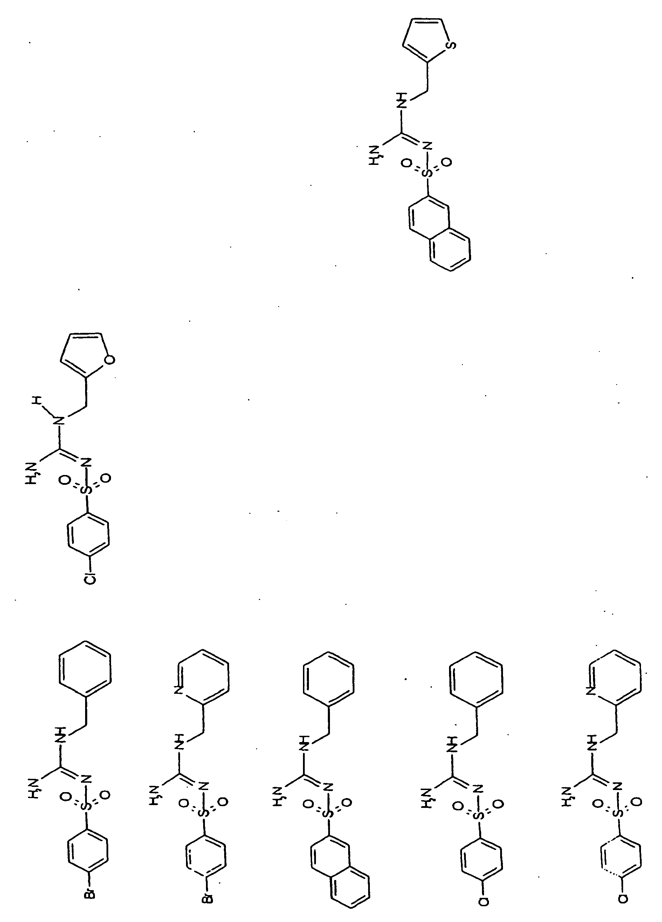

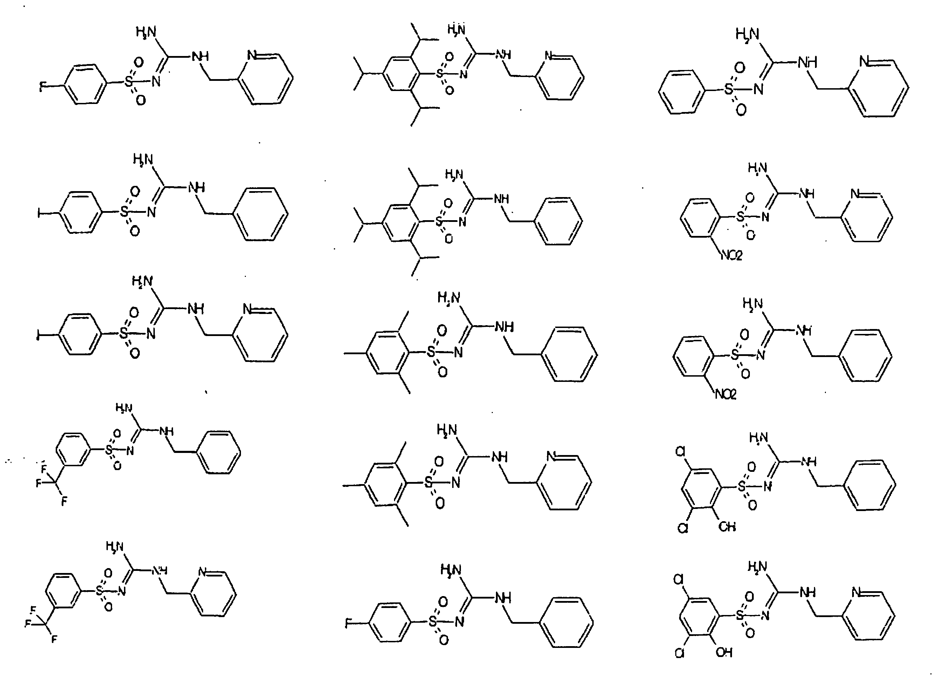

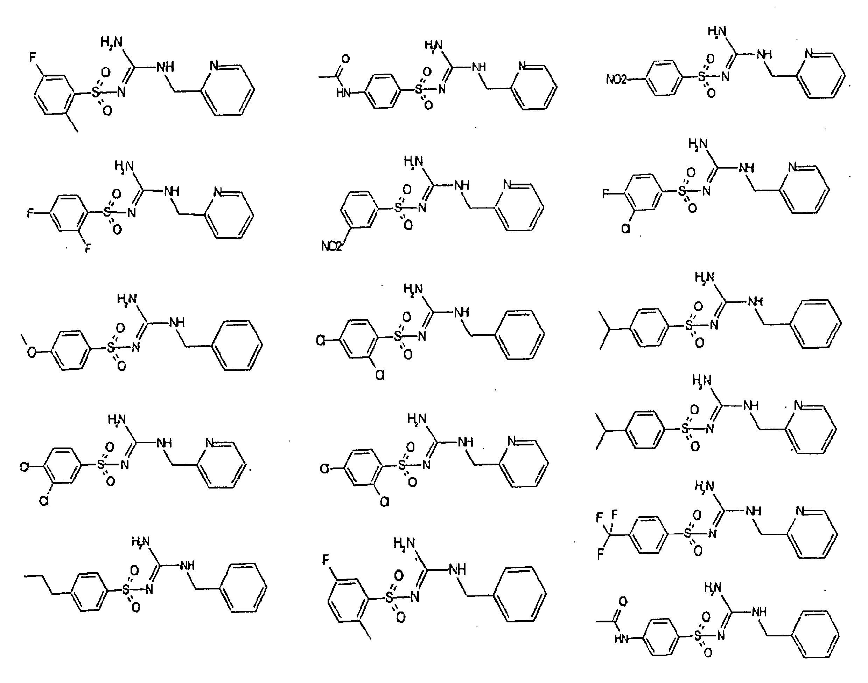

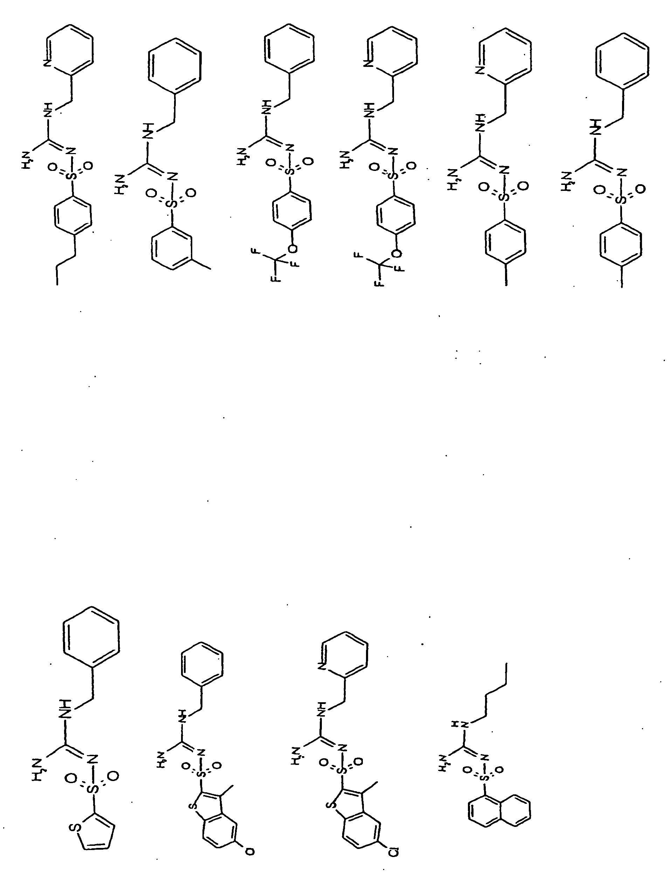

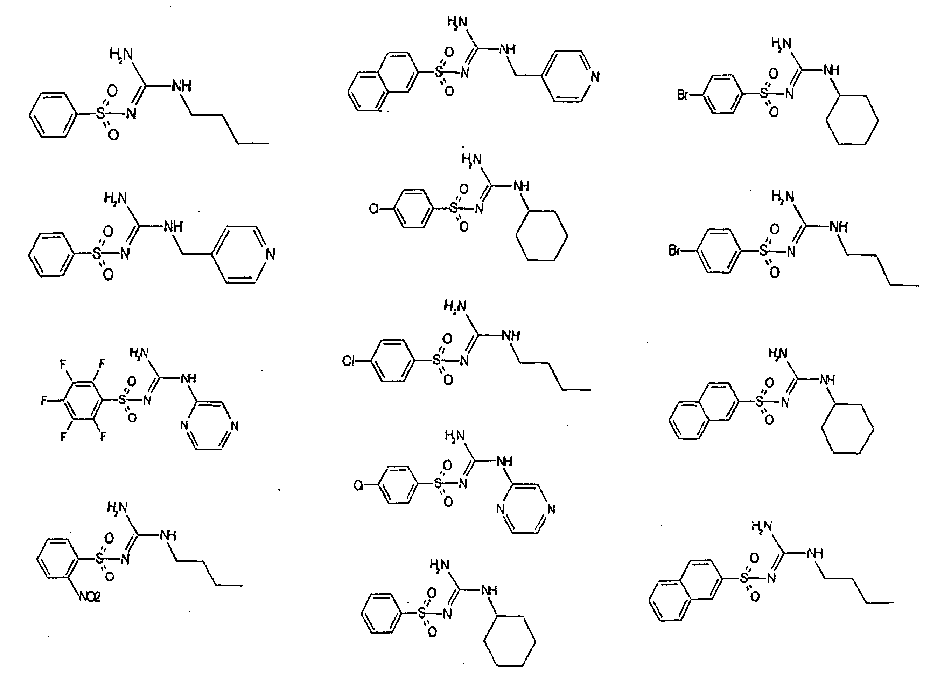

Weitere Verbindungen, aus denen bevorzugt erfindungsgemäß verwendete Verbindungen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen; oder in Form ihrer Salze, insbesondere der physiologisch verträglichen Salze, vorzugsweise des Hydrochlorid- oder Natriumsalzes; oder in Form ihrer Solvate, insbesondere der Hydrate; in beiden tautomeren Formen nach Formeln I und Ia, ausschließlich in einer der tautomeren Formen nach Formeln I oder Ia oder auch in Mischungen beider Formen nach Formeln I und Ia, wobei die bevorzugte tautomere Form von Verbindung zu Verbindung z.B. in Abhängigkeit vom Aggregatzustand oder vom gewählten Lösungsmittel variieren kann; ausgewählt werden, sind als Strukturformeln den Abbildungen 1 - 40 zu entnehmen.

Dabei ist es weiter bevorzugt, wenn das erfindungsgemäß verwendete Sulfonylguanidin ganz oder überwiegend in der tautomeren Form gemäß Formel I vorliegt.

Ein ebenfalls bevorzugter Gegenstand dieser Anmeldung umfaßt erfindungsgemäß verwendete Sulfonylguanidine, die ganz oder überwiegend in der tautomeren Form gemäß Formel Ia vorliegen.

Ein weiterer Gegenstand der Erfindung sind die Sulfonylguanidine ausgewählt aus der folgenden Gruppe:
- *N*-{Amino-[pyridin-2ylmethyl)-amino]-methylen}-4-methylbenzolsulfonamid **(1)**
- *N*-[Amino-(benzylamino-methylen)]-4-methylbenzolsulfonamid **(2)**
- *N*-[Amino-phenethylamino-methylen]-4-methylbenzolsulfonamid **(14)**
- N-{Amino-[pyridin-2ylmethyl)-amino]-methylen}-4-chlorbenzolsulfonamid **(21)**
- N-{Amino-[pyridin-4ylmethyl)-amino]-methylen}-4-methylbenzolsulfonamid **(22)**
- N-{Amino-[pyridin-3ylmethyl)-amino]-methylen}-4-methylbenzolsulfonamid **(23)**
- N-{Amino-[(pyridin-2-ylmethyl)-amino]-methylen}-4-brom-benzolsulfonamid **(32)**
- N-{Amino-[(pyridin-2-ylmethyl)-amino]-methylen}-3,4-dichlor-benzolsulfonamid **(33)**
- N-{Amino-[(pyridin-2-ylmethyl)-amino]-methylen}-4-isopropyl-benzolsulfonamid **(34)**
- N-{Amino-[(pyridin-2-ylmethyl)-amino]-methylen}-4-iod-benzolsulfonamid **(35)**
- N-{Amino-[(pyridin-2-ylmethyl)-amino]-methylene}-benzolsulfonamid **(36)**
- Naphthalin-2-sulfonsäure-amino-[(pyridin-2-ylmethyl)-amino]-methylenamid **(37)**
- 1-Methyl-1H-imidazol-4-sulfonsäure-amino-[(pyridin-2-ylmethyl)-amino]-methylenamid **(38)**
- N-[4-({Amino-[(pyridin-2-ylmethyl)-amino]-methylen}-sulfamoyl)-phenyl]-acetamid **(39)**
- N-{Amino-[(pyridin-2-ylmethyl)-amino]-methylen}-4-fluor-benzolsulfonamid **(40)**
- N-{Amino-[(pyridin-2-ylmethyl)-amino]-methylen}-2,4,6-trimethyl-benzolsulfonamid **(41)**
- N-{Amino-[(pyridin-2-ylmethyl)-amino]-methylen}-4-propyl-benzolsulfonamid **(42)**
- N-{Amino-[(pyridin-2-ylmethyl)-amino]-methylen}-4-methoxy-benzolsulfonamid **(43)**
- Naphthalin-1-sulfonsäure-amino-[(pyridin-2-ylmethyl)-amino]-methylenamid **(44)**
- N-{Amino-[(pyridin-2-ylmethyl)-amino]-methylen}-3-methyl-benzolsulfonamid **(45)**
- Thiophen-2-sulfonsäure-amino-[(pyridin-2-ylmethyl)-amino]-methylenamid **(46)**
- Quinolin-8-sulfonsäure-amino-[(pyridin-2-ylmethyl)-amino]-methylenamid **(47)**
- 5-Chlor-3-methyl-benzo[b]thiophen-2-sulfonsäure-amino-[(pyridin-2-ylmethyl)-amino]-methylenamid **(48)**
- N-{Amino-[(pyridin-2-ylmethyl)-amino]-methylen}-4-tert-butyl-benzolsulfonamid **(49)**
- N-{Amino-[(pyridin-2-ylmethyl)-amino]-methylen}-4-butyl-benzolsulfonamid **(50)**
- N-{Amino-[(pyrimidin-2-ylmethyl)-amino]-methylen}-4-tert-butyl-benzolsulfonamid **(51)**
- N-{Amino-[(furan-2-ylmethyl)-amino]-methylen}4-butyl-benzolsulfonamid **(52)**
- N-{Amino-[(furan-2-ylmethyl)-amino]-methylen}-4-methyl-benzolsulfonamid **(53)**
- N-{Amino-[(furan-2-ylmethyl)-amino]-methylen}-4-isopropyl-benzolsulfonamid **(54)**
- N-{Amino-[(furan-2-ylmethyl)-amino]-methylen}-4-propyl-benzolsulfonamid **(55)**
- N-{Amino-[(furan-2-ylmethyl)-amino]-methylen}-4-tert-butyl-benzolsulfonamid **(56)**
- N-{Amino-[(thiophen-2-ylmethyl)-amino]-methylen}-4-butyl-benzolsulfonamid **(57)**
- N-{Amino-[(thiophen-2-ylmethyl)-amino]-methylen}-4-propyl-benzolsulfonamid **(58)**
- N-{Amino-[(thiophen-2-ylmethyl)-amino]-methylene)-4-isopropyl-benzolsulfonamid **(59)**
- N-{Amino-[(thiophen-2-ylmethyl)-amino]-methylen}-4-tert-butyl-benzolsulfonamid **(60)**
- N-{Amino-[(thiophen-2-ylmethyl)-amino]-methylen}-4chlor-benzolsulfonamid **(61)**
- N-{Amino-[(thiophen-2-ylmethyl)-amino]-methylen}-4-methyl-benzolsulfonamid **(62)**
- N-{Amino-[(furan-2-ylmethyl)-amino]-methylen}-4-chlor-benzolsulfonamid **(63)**
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen; oder in Form ihrer Salze, insbesondere der physiologisch verträglichen Salze, vorzugsweise des Hydrochlorid oder Natriumsalzes; oder in Form ihrer Solvate, insbesondere der Hydrate; in beiden tautomeren Formen nach Formeln I und Ia, ausschließlich in einer der tautomeren Formen nach Formeln I oder Ia oder auch in Mischungen beider Formen nach Formeln I und Ia, wobei die bevorzugte tautomere Form von Verbindung zu Verbindung z.B. in Abhängigkeit vom Aggregatzustand oder vom gewählten Lösungsmittel variieren kann.

Weitere Verbindungen, aus denen bevorzugt erfindungsgemäße Verbindungen in Form ihrer Razernate; Enantiomere, Diastereomere, insbesondere Mischungen ihrer Enantiomere oder Diastereomere oder eines einzelnen Enantiomers oder Diastereomers; gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;in dargestellter Form oder in Form ihrer Säuren oder ihrer Basen; oder in Form ihrer Salze, insbesondere der physiologisch verträglichen Salze, vorzugsweise des Hydrochlorid- oder Natriumsalzes; oder in Form ihrer Solvate, insbesondere der Hydrate; in beiden tautomeren Formen nach Formeln I und Ia, ausschließlich in einer der tautomeren Formen nach Formeln I oder Ia oder auch in Mischungen beider Formen nach Formeln I und la, wobei die bevorzugte tautomere Form von Verbindung zu Verbindung z.B. in Abhängigkeit vom Aggregatzustand oder vom gewählten Lösungsmittel variieren kann, ausgewählt werden, sind als Strukturformeln den Abbildungen 1 - 40 zu entnehmen.

Dabei ist es weiter bevorzugt, wenn das erfindungsgemäße Sulfonylguanidin ganz oder überwiegend in der tautomeren Form gemäß Formel I vorliegt.

Ein ebenfalls bevorzugter Gegenstand dieser Anmeldung umfaßt erfindungsgemäße Sulfonylguanidine, die ganz oder überwiegend in der tautomeren Form gemäß Formel Ia vorliegen.

Die erfindungsgemäßen Sulfonylguanidine sind toxikologisch unbedenklich, so dass sie sich als pharmazeutischer Wirkstoff in Arzneimitteln eignen. Ein weiterer Gegenstand der Erfindung sind daher Arzneimittel enthaltend wenigstens ein erfindungsgemäßes Sulfonylguanidin, sowie gegebenenfalls geeignete Zusatz- und/oder Hilfsstoffe und/oder gegebenenfalls weitere Wirkstoffe.

Gleiches gilt für die erfindungsgemäß in den genannten Indikationen verwendeten Sulfonylguanidine, da auch die erfindungsgemäß verwendeten Sulfonylguanidinen natürlich toxikologisch unbedenklich, so dass sie sich als pharmazeutischer Wirkstoff in Arzneimitteln eignen. Ein weiterer Gegenstand der Erfindung sind daher Arzneimittel enthaltend wenigstens eine der erfindungsgemäß verwendeten Sulfonylguanidine, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen oder erfindungsgemäß verwendeten Sulfonylguanidin gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Sulfonylguanidine in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen erfindungsgemäß verwendeten Sulfonylguanidine verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen erfindungsgemäß verwendeten Sulfonylguanidins appliziert.

In einer bevorzugten Form des Arzneimittel liegt ein enthaltenes erfindungsgemäßes erfindungsgemäß verwendetes Sulfonylguanidin als reines Diastereomer und/oder Enantiomer, als Razemat oder als nicht-äquimolare oder äquimolare Mischung der Diastereomere und/oder Enantiomere und/oder ausschließlich in einer der tautomeren Formen nach Formeln I oder la oder auch in Mischungen beider Formen nach Formeln I und la vor.

Ein weiterer Gegenstand der Erfindung ist die Verwendung eines erfindungsgemäßen Sulfonylguanidins gemäß den allgemeinen tautomeren Formeln I und Ia zur Herstellung eines Arzneimittels zur Behandlung von Schmerz, insbesondere von neuropathischem, chronischem oder akutem Schmerz, von Epilepsie und/oder von Migräne
oder
zur Herstellung eines Arzneimittels zur Behandlung von Hyperalgesie und Allodynie, insbesondere thermischer Hyperalgesie, mechanischer Hyperalgesie und Allodynie und Kälte-Allodynie, oder von inflammatorischem oder postoperativem Schmerz
oder
zur Herstellung eines Arzneimittels zur Behandlung von Hitzewallungen, Beschwerden in der Postmenopause, Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Morbus Alzheimer, Morbus Huntington, Morbus Parkinson und Epilepsie; gastrointestinaler Schädigung; von erythromelalgischem oder postpoliomyelitischem Schmerz, trigeminaler oder postherpetischer Neuralgie; oder als Antikonvulsivum, Analgetikum oder Anxiolytikum.

Bei jeder der vorgenannten erfindungsgemäßen Verwendungen kann es bevorzugt sein, wenn ein verwendetes Sulfonylguanidin als reines Diastereomer und/oder Enantiomer, als Razemat oder als nicht-äquimolare oder äquimolare Mischung der Diastereomere und/oder Enantiomere und/oder ausschließlich in einer der tautomeren Formen nach Formeln I oder Ia oder auch in Mischungen beider Formen nach Formeln I und Ia vorliegt.

Die erfindungsgemäßen oder erfindungsgemäß verwendeten Sulfonylguanidine eignen sich für Verfahren zur Behandlung eines nichthumanen Säugetieres oder Menschen, das oder der eine Behandlung medizinisch relevanter Symptome benötigt, durch Verabreichung einer therapeutisch wiksamen Dosis eines erfindungsgemäßen oder erfindungsgemäß verwendeten Sulfonylguanidins oder eines erfindungsgemäßen Arzneimittels. Dies betrifft insbesondere entsprechende Verfahren zur Behandlung von Schmerz, insbesondere von neuropathischem, chronischem oder akutem Schmerz; Migräne, Hyperalgesie und Allodynie, insbesondere thermischer Hyperalgesie, mechanischer Hyperalgesie und Allodynie und Kälte-Allodynie, oder von inflammatorischem oder postoperativem Schmerz; Epilepsie, Hitzewallungen, Beschwerden in der Postmenopause, Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Alzheimer Disease, Huntington's Disease, Parkinson Disease und Epilepsie; von erythromelalgischem oder postpoliomyelitischem Schmerz, trigeminaler oder postherpetischer Neuralgie

Ein weiterer Gegenstand der Erfindung ist ein Verfahren zur Herstellung eines erfindungsgemäßen Sulfonylguanidins wie in der folgenden Beschreibung und Beispielen ausgeführt.

### Synthesevorschrift:

Die Synthese der erfindungsgemäßen Verbindungen erfolgt in der Weise, daß man Pyrrazolsulfonsäureamide mit der allgemeinen Formel II, die nach literaturbekannten Verfahren synthestisiert werden (Larock 2.Ausgabe), mit verschiedensten primären oder sekundären Aminen in Gegenwart von Methansulfonsäure umsetzt. R¹ bis R³ haben dabei die oben für Verbindungen der Formel I angegeben Bedeutung.

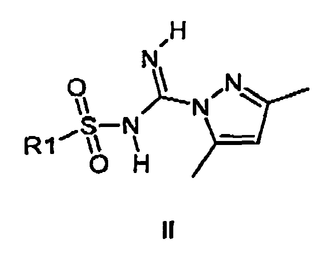

Vorzugsweise werden die Reaktionen in Acetonitril bei einer Bad-Temperatur von vorzugsweise 0°C bis 110°C, insbesondere bei einer Bad-Temperatur von 100°C bis 110°C durchgeführt.

Die Verbindungen der Formel I lassen sich mit physiologisch verträglichen Säuren, vorzugsweise Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure und/ oder Asparaginsäure und insbesondere Salzsäure, in der an sich bekannten Weise in ihre Salze überführen. Vorzugsweise wird die Salzbildung in einem Lösungsmittel, vorzugsweise Diethylether, Diisopropylether, Essigsäurealkylester, Aceton oder 2-Butanon oder einem Gemisch dieser Lösungsmittel durchgeführt. Zur Herstellung der Hydrochloride eignet sich alternativ auch Trimethylsilan in wässriger Lösung.

### Salzbildung

Die Verbindungen der Formel I lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure, in an sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon oder auch Wasser durchgeführt. Zur Herstellung der Hydrochloride eignet sich darüber hinaus Trimethylchlorsilan in wäßriger Lösung.

Im folgenden wird die Erfindung weiter durch Beispiele und Abbildungen erläutert, ohne sie darauf zu beschränken.

### Abbildungen :

**Fig. 1****-** **Fig 40****)** zeigen nach den folgenden Verfahren, insbesondere nach dem Grundverfahren 1 Beispiel 0 hergestellte weitere Verbindungen, die auf den Abbildungen als Strukturformeln abgebildet sind. Bei diesen Beispielen handelt es sich um erfindungsgemäße Verbindungen und/oder Verbindungen, die aufgrund ihrer Wirkungsstruktur erfindungsgemäß verwendet werden können.

### Beispiele

Die folgenden Beispiele zeigen erfindungsgemäße Verbindungen sowie deren Darstellung und mit diesen durchgeführte Wirksamkeitsuntersuchungen. Es wurde meist die Darstellung in der tautomeren Form nach Formel I gewählt. Darunter ist aber zu verstehen, daß die Verbindungen sowohl in der einen wie in der anderen Form vorliegen können und daher diese Darstellung keine Beschränkung auf die eine oder andere tautomere Form darstellen muß oder darstellt.

Dabei gelten generell folgende Angaben:
Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCI etc.) oder synthetisiert.

Die Analytik erfolgte über ESI-Massenspektrometrie oder HPLC.

Zur Charakterisierung wurde jeweils ein ESI-MS aufgenommen.

Die folgenden Beispiele zeigen Verbindungen, die in Substanzbibliotheken enthalten sein können sowie deren Darstellung und mit diesen durchgeführte Wirksamkeitsuntersuchungen.

Dabei gelten generell folgende Angaben:
Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCI etc) oder synthetisiert.

Die Analytik erfolgte über ESI-Massenspektrometrie und/oder ¹H-NMR (Bsp. 1-21). Für die erfindungsgemäßen Beispiele 22-30 liegen nur massenspektroskopische Daten vor.

### Beispiel 0:

### Grundverfahren 1

Ein Rundbodenröhrchen aus Glas (Durchmesser 16 mm, Länge 125 mm) mit Gewinde wurde manuell mit einem Rührer versehen und auf der Capper-Station mit einem Schraubdeckel mit Septum verschlossen. Das Röhrchen wurde von Roboter 1 in den auf 110°C temperierten Rührblock gestellt. Roboter 2 plpettierte nacheinander die folgenden Reagenzien hinzu:
1.) 0.5 ml einer Lösung, die Methansulfonsäure und die Pyrrazolsulfonsäureamide jeweils 0,2M enthält, in Acetonitril
2.) 0.5 ml einer 1.0 M Amin-Lösung in Acetonitril

Das Reaktionsgemisch wurde bei 110°C in einem der Rührblöcke 45 Stunden lang gerührt. Danach wurde die Reaktionslösung an der Filtrations-Station abfiltriert. Das Röhrchen wurde dabei zweimal mit je 1.5 ml Dichlormethan und 1.5 ml Wasser gespült.

Das Rack mit den Proben wurde manuell auf die Aufarbeitungsanlage gestellt. Das Reaktionsgemisch wurde 30 Minuten geschüttelt. Zur Ausbildung der Phasengrenze wurde in der Zentrifuge kurz zentrifugiert. Die Phasengrenze wurde optisch detektiert und die organische Phase abpipettiert. Im nächsten Schritt wurde die wäßrige Phase erneut mit 1.5 ml Dichlormethan versetzt, geschüttelt, zentrifugiert und die organische Phase abpipettiert. Die vereinigten organischen Phasen wurden über 2,4 g MgSO4 (granuliert) getrocknet. Das Lösungsmittel wurde in einer Vakuumzentrifuge entfernt. Jede Probe wurde mit ESI-MS und/oder NMR analysiert.

Durch die automatisierte Synthese ist eine Gleichbehandlung aller Proben sowie eine ausgesprochen konstante Reaktionsführung gewährleistet.

### Beispiel 1:

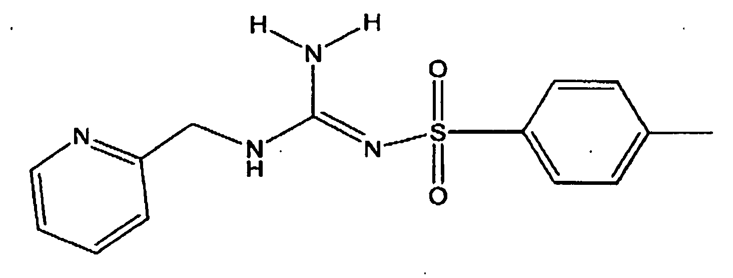

### N-{Amino-[pyridin-2ylmethyl)-amino]-methylen}-4-methylbenzölsulfonamid (1)

Beispiel **1** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml 2-Picolylamin-Lösung (1.0 M, Acetonitril) in einer Substanzbibliothek dargestellt.

Berechnete Masse 304.37; gefundene Masse (M+H) 305.2; 609.0 (Dimer)

### Beispiel 2:

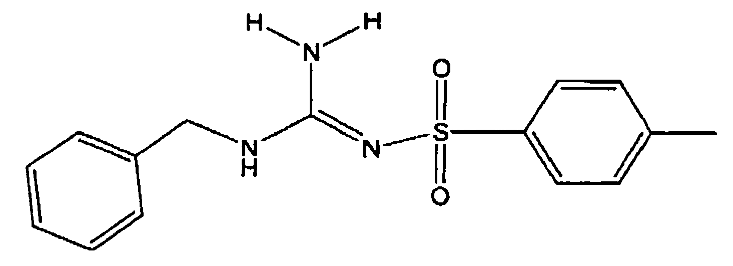

### N-[Amino-(benzylamino-methylen)]-4-methylbenzolsulfonamid (2)

Beispiel **2** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml Benzylamin-Lösung (1.0 M, Acetonitril) in einer Substanzbibliothek dargestellt.

Berechnete Masse 303.38; gefundene Masse (M+H) 304.4; 607.9 (Dimer)

### Beispiel 3:

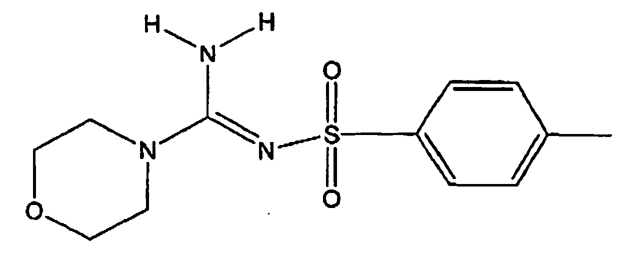

### N-(Amino-morpholin-4yl-methylen)-4-methylbenzolsulfonamid (3)

Beispiel 3 wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml Morpholin-Lösung (1.0 M, Acetonitril) in einer Substanzbibliothek dargestellt.

Berechnete Masse 283.35; gefundene Masse (M+H) 284.1

### Beispiel 4:

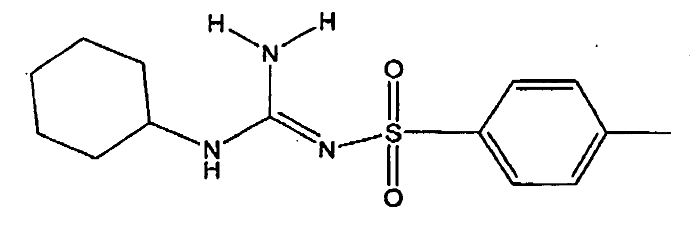

### N-(Amino-cyclohexylaminomethylen)-4-methylbenzolsulfonamid (4)

Beispiel **4** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M Acetonitril) mit 19 mg Methansulfonsäure und 0.5 ml Cyclohexylamin-Lösung (1.0 M, Acetonitril) in einer Substanzbibliothek dargestellt.

Berechnete Masse 295,40; gefundene Masse (M+H) 296.1; 591.5 (Dimer)

### Beispiel 5:

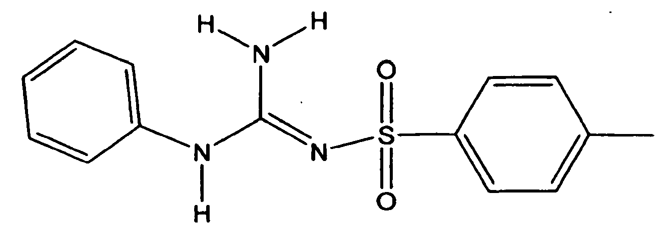

### N-(Amino-phenylaminomethylen)-4-methylbenzolsulfonamid (5)

Beispiel **5** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml Anilin-Lösung (1.0 M, Acetonitril) in einer Substanzbibliothek dargestellt.

Berechnete Masse 289,35; gefundene Masse (M+H) 290.2; 579.0 (Dimer)

### Beispiel 6 (gehört nicht zur Erfindung):

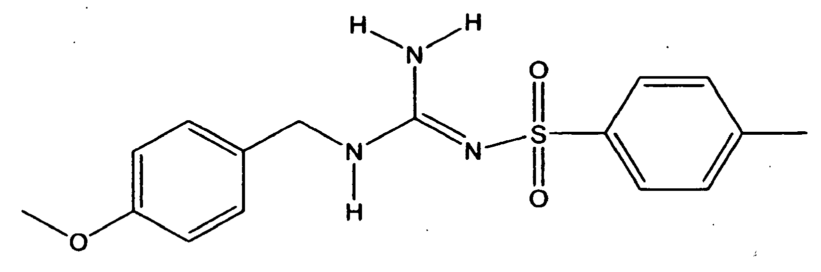

### N-[(Amino-4-methoxybenzylamino)methylen]-4-methylbenzolsulfonamid (6)

Beispiel **6** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml 4-Methoxybenzylamin-Lösung (1.0 M, Acetonitril) in einer Substanzbibliothek dargestellt.

Berechnete Masse 333,41; gefundene Masse (M+H) 334.2; 667.0 (Dimer)

### Beispiel 7:

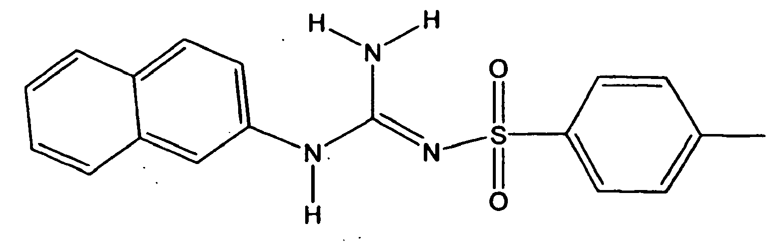

### N-[Amino-(naphthalin-2-ylamino)methylen]-4-methylbenzolsulfonamid (7)

Beispiel **7** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml 2-Naphthylamin-Lösung (1.0 M, Acetonitril) in einer Substanzbibliothek dargestellt.

Berechnete Masse 339,41; gefundene Masse (M+H) 340.3; 679.1 (Dimer)

### Beispiel 8 (gehört nicht zur Erfindung):

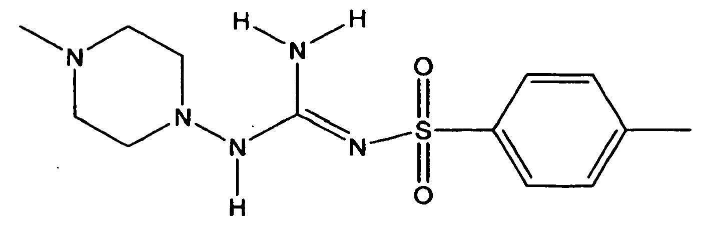

### N-[Amino-(4-methyl-piperazin-1-ylamino)-methylen]-4-methylbenzolsulfonamid (8)

Beispiel **8** wurde gemäß der beigefügten Synthesevorschrift aus 0.5 ml N-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml *N*-Amino-4-methylpiperazin-Lösung (1.0 M, Acetonitril) in einer Substanzbibliothek dargestellt.

Berechnete Masse 311,40; gefundene Masse (M+H) 312.2; 623.0 (Dimer)

### Beispiel 9:

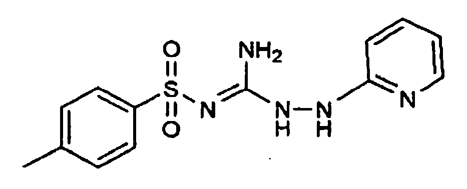

### N-[Amino-(N'-pyridin-2.yl.hydrazino)-methylen]-4-methylbenzolsulformamid (9)

Beispiel **9** wurde gemäß der beigefügten Synthesevorschrift aus 0.5 ml *N*-[(3,5-Dimethyl-pyrazyl-1-yl)-imino-methyl]-4-methylbenzolsulfynamid-Lösung (0.2 M, Acetonitril) mit 19 mg Methansulfonsäure und 0.5 ml 2-Hydrazinopyridin-Lösung (1.0 M, Acetonitril) in einer Substanzbibliothek dargestellt.

Berechnete Masse 305,36; gefundene Masse (M+H) 306.1: 621.6 (Dimer)

### Beispiel 10:

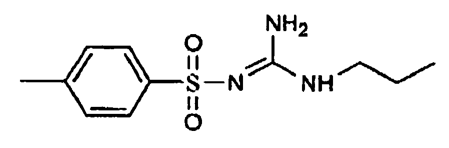

### N-[Amino-propylamino-methylen]-4-methylbenzolsulfonamid (10)

Beispiel **10** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml nPropylamin-Lösung (1.0 M, Acetonitril) in einer Substanzbibliothek dargestellt.

Berechnete Masse 255,34; gefundene Masse (M+H) 256.2; 511.0 (Dimer)

### Beispiel 11:

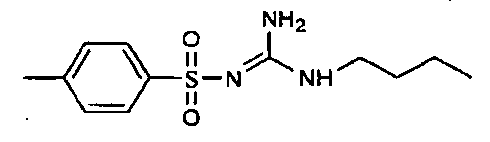

### N-(Amino-butylamino-methylen)-4-methylbenzolsulfonamid (11)

Beispiel **11** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml nButylamin-Lösung (1.0 M, Acetonitril) in einer Substanzbibliothek dargestellt.

Berechnete Masse 269,36; gefundene Masse (M+H) 270.3; 539.1 (Dimer)

### Beispiel 12:

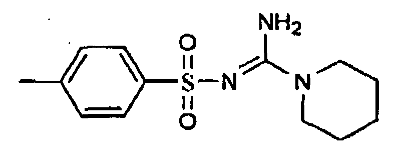

### N-[Amino-piperidin-methylen]-4-methylbenzolsulfonamid (12)

Beispiel **12** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml Piperidin-Lösung (1.0 M, Acetonitril) in einer Substanzbibliothek dargestellt.

Berechnete Masse 281,37; gefundene Masse (M+H) 282.2; 562.9 (Dimer)

### Beispiel 13:

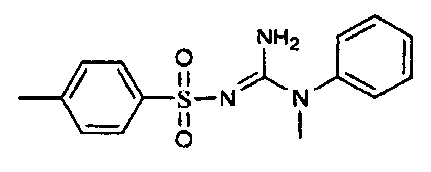

### N-[Amino-(N-methyl-N-phenylamino)-methylen]-4-methylbenzolsulfonamid (13)

Beispiel **13** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml *N*-Methylanilin-Lösung (1.0 M, Acetonitril) in einer Substanzbibliothek dargestellt.

Berechnete Masse 303,38; gefundene Masse (M+H) 304.2

### Beispiel 14:

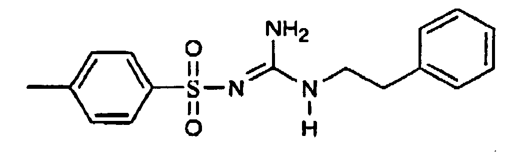

### N-[Amino-phenethylamino-methylen]-4-methylbenzolsulfonamid (14)

Beispiel **14** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml Phenethylamin-Lösung (1.0 M, Acetonitril) in einer Substanzbibliothek dargestellt.

Berechnete Masse 317,41; gefundene Masse (M+H) 318.3; 635.1 (Dimer)

### Beispiel 15:

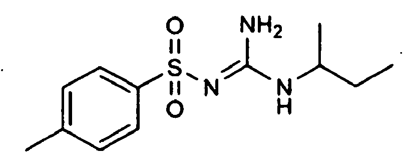

### N-[Amino-sec-butylamino-methylen]-4-methylbenzolsulfonamid (15)

Beispiel **15** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml sButylamin-Lösung (1.0 M, Acetonitril) in einer Substanzbibliothek dargestellt.

Berechnete Masse 269,36; gefundene Masse (M+H) 270.2; 539.1 (Dimer)

### Beispiel 16 (gehört nicht zur Erfindung):

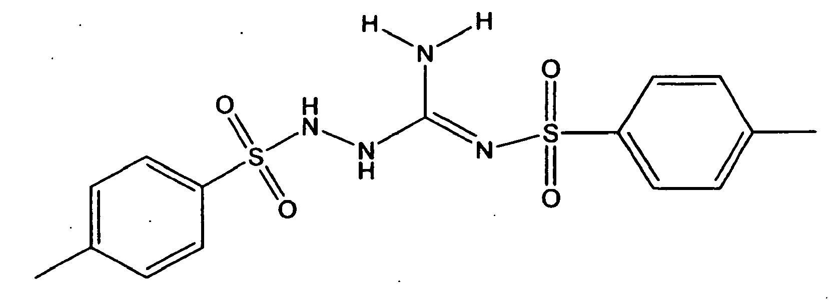

### N-[Amino-(N'-tosyl-2-yl-hydrazino)-methylen]-4-methylbenzolsulfonamid (16)

Beispiel **16** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml Tosylhydrazid-Lösung (1.0 M, Acetonitril) in einer Substanzbibliothek dargestellt.

Berechnete Masse 382,46; gefundene Masse (M+H) 383.2; 764.9 (Dimer)

### Beispiel 17:

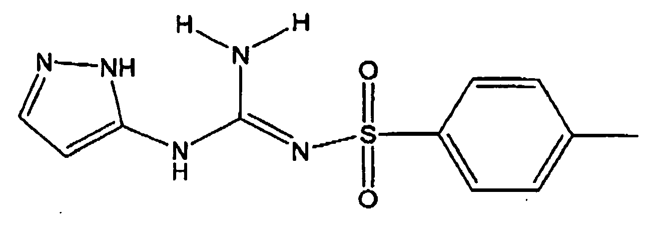

### N-[Amino-(2H-pyrazol-3-ylamino)-methylen]-4-methylbenzolsulfonamid (17)

Beispiel **17** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml 2-Aminopyrazol-Lösung (1.0 M, Acetonitril) in einer Substanzbibliothek dargestellt.

Berechnete Masse 279,32; gefundene Masse (M+H) 280.3; 559.0 (Dimer)

### Beispiel 18:

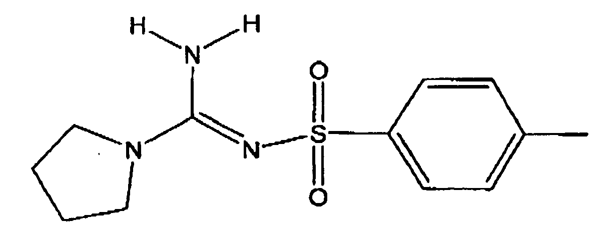

### N-(Amino-pyrrolidin-1-yl-methylen)-4-methylbenzolsulfonamid (18)

Beispiel **18** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml Pyrrolidin-Lösung (1.0 M, Acetonitril) in einer Substanzbibliothek dargestellt.

Berechnete Masse 267,35; gefundene Masse (M+H) 268.2; 534.9 (Dimer)

### Beispiel 19:

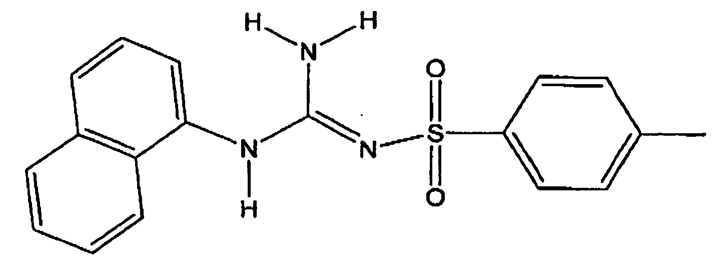

### N-[Amino-(naphthalin-1-ylamino)-methylen]-4-methylbenzolsulfonamid (19)

Beispiel **19** wurde gemäß der beigefügten Synthesevorschrift aus 0.5 ml *N*-[(3,5-Dimethyl-pyrazot-1-yl)-imino-methyl]-4-methylbenzolsulfonamid-Lösung (0.2 M, Acetonitril) mit 19 mg Methansulfonsäure und 0.5 ml 1-Naphthylamin-Lösung (1.0 M, Acetonitril) in einer Substanzbibliothek dargestellt.

Berechnete Masse 339,41; gefundene Masse (M+H) 340.3: 679.1 (Dimer)

### Beispiel 20:

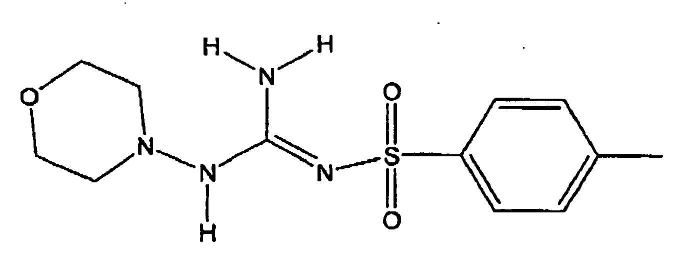

### N-[Amino-(morpholin-4-ylamino)-methylen]-4-methylbenzolsulfonamid (20)

Beispiel **20** wurde gemäß der beigefügten Synthesevorschrift aus 0.5 ml *N*-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml *N*-Aminomorpholin-Lösung (1.0 M, Acetonitril) in einer Substanzbibliothek dargestellt.

Berechnete Masse 298,11; gefundene Masse (M+H) 299.3; 597.1 (Dimer)

### Beispiel 21:

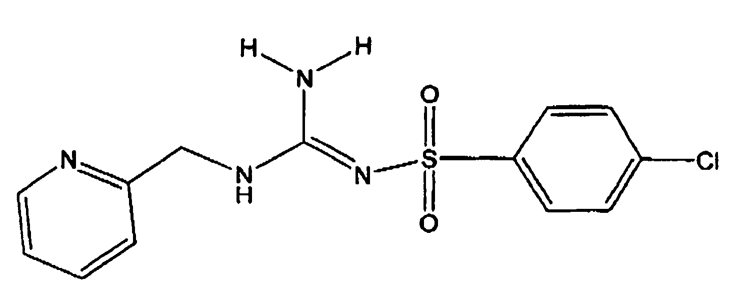

### N-{Amino-[pyridin-2ylmethyl)-amino]-methyen}-4-chlorbenzolsulfonamid (21)

Beispiel **21** wurde gemäß der beigefügten Synthesevorschrift aus 0.5 ml N-[(3,5-Dimethyl-pyrazol-1-yl)-imino-methyl]-4-chlorbenzolsulfonamid-Lösung (0.2 M, Acetonitril) mit 19 mg Methansulfonsäure und 0.5 ml 2-Picolylamin-Lösung (1.0 M, Acetonitril) in einer Substanzbibliothek dargestellt.

Berechnete Masse 327.79; gefundene Masse (M+H) 328.3; 655.6 (Dimer)

### Beispiel 22:

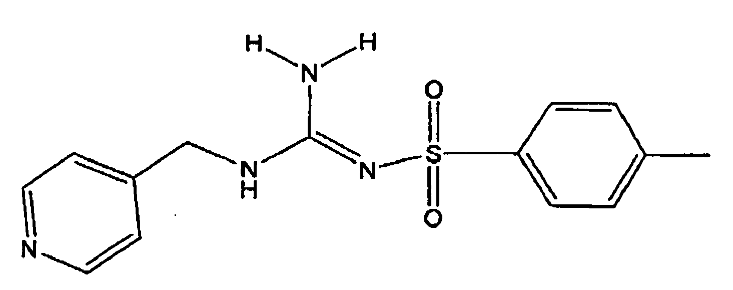

### N-{Amino-[pyridin-4ylmethyl)-amino]-methylen}4-methylbenzolsulfonamid (22)

Beispiel **22** wurde gemäß der beigefügten Synthesevorschrift aus 0.5 ml N-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml 4-Picolylamin-Lösung (1.0 M, Acetonitril) in einer Substanzbibliothek dargestellt

Berechnete Masse 304.37; gefundene Masse (M+H) 305.2; 609.1 (Dimer)

### Beispiel 23:

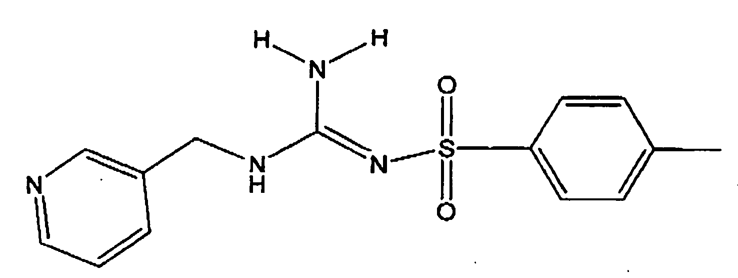

### N-{Amino-[pyridin-3ylmethyl)-amino]-methylen}-4-methylbenzolsulfonamid (23)

Beispiel **23** wurde gemäß der beigefügten Synthesevorschrift aus 0.5 ml N-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml 3-Picolylamin-Lösung (1.0 M, Acetonitril) in einer Substanzbibliothek dargestellt.

Berechnete Masse 304.37; gefundene Masse (M+H) 305.3; 609.2 (Dimer)

### Beispiel 24:

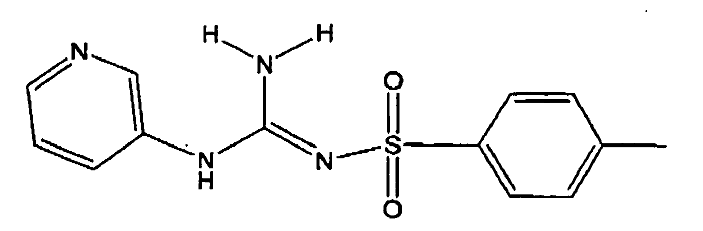

### N-[Amino-(pyridin-3ylamino)-methylen]-4-methylbenzolsulfonamid (24)

Beispiel **24** wurde gemäß der beigefügten Synthesevorschrift aus 0.5 ml N-[(3,5-Dimethyl-pyrazol-1-yl)-imino-methyl]-4-methylbenzolsulfonamid-Lösung (0.2 M, Acetonitril) mit 19 mg Methansulfonsäure und 0.5 ml 3-Aminopyridin-Lösung (1.0 M, Acetonitril) in einer Substanzbibliothek dargestellt.

Berechnete Masse 290,34; gefundene Masse (M+H) 291.1; 580.9 (Dimer)

### Beispiel 25:

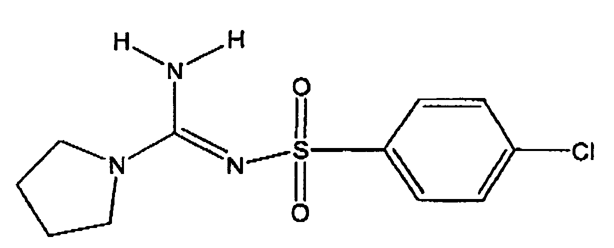

### N-(Amino-pyrrolidin-1-yl-methylen)-4-chlorbenzolsulfonamid (25)

Beispiel **25** wurde gemäß der beigefügten Synthesevorschrift aus 0.5 ml *N*-[(3,5-Dimethyl-pyrazol-1-yl)-imino-methyl]-4-chlorbenzolsulfonamid-Lösung (0.2 M, Acetonitril) mit 19 mg Methansulfonsäure und 0.5 ml Pyrrolidin-Lösung (1.0 M. Acetonitril) in einer Substanzbibliothek dargestellt.

Berechnete Masse 287.77: gefundene Masse (M+H) 288.3; 574.8 (Dimer)

### Beispiel 26:

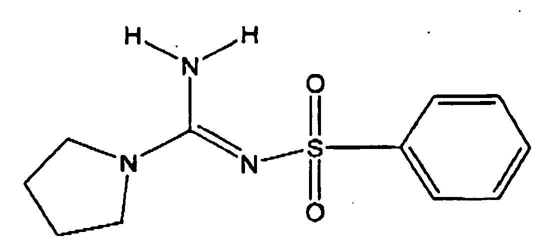

### N-(Amino-pyrrolidin-1-yl-methylen)-benzolsulfonamid (26)

Beispiel **26** wurde gemäß der beigefügten Synthesevorschrift aus 0.5 ml *N*-[(3,5-Dimethyl-pyrazol-1-yl)-imino-methyl]-benzolsulfonamid-Lösung (0.2 M, Acetonitril) mit 19 mg Methansulfonsäure und 0.5 ml Pyrrolidin-Lösung (1.0 M, Acetonitril) in einer Substanzbibliothek dargestellt.

Berechnete Masse 253,32; gefundene Masse (M+H) 254.3; 506.9 (Dimer)

### Beispiel 27:

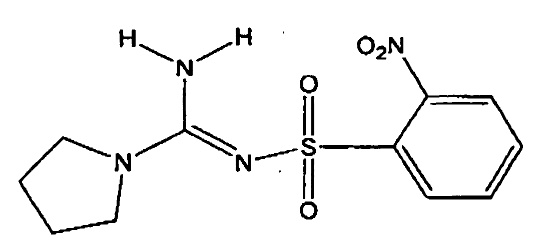

### N-(Amino-pyrrolidin-1-yl-methylen)-2-nitrobenzolsulfonamid (27)

Beispiel **27** wurde gemäß der beigefügten Synthesevorschrift aus 0.5 ml N-[(3,5-Dimethyl-pyrazol-1-yl)-imino-methyl]-2-nitrobenzolsulfonamid-Lösung (0.2 M, Acetonitril) mit 19 mg Methansulfonsäure und 0.5 ml Pyrrolidin-Lösung (1.0 M, Acetonitril) in einer Substanzbibliothek dargestellt.

Berechnete Masse 298,32; gefundene Masse (M+H) 299.3: 596.8 (Dimer)

### Beispiel 28:

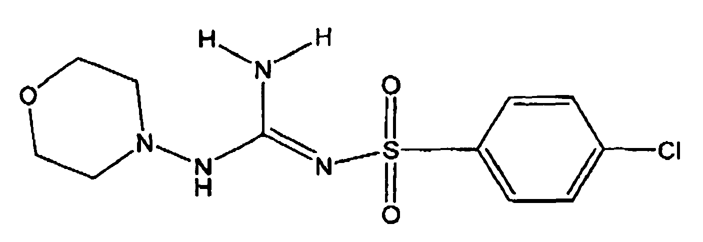

### N-[Amino-(morpholin-4-ylamino)-methylen]-4-chlorbenzolsulfonamid (28)

Beispiel **28** wurde gemäß der beigefügten Synthesevorschrift aus 0.5 ml *N*-[(3,5-Dimethyl-pyrazol-1-yl)-imino-methyl]-4-chlorbenzolsulfonamid-Lösung (0.2 M, Acetonitril) mit 19 mg Methansulfonsäure und 0.5 ml *N*-Aminomorpholin-Lösung (1.0 M, Acetonitril) in einer Substanzbibliothek dargestellt.

Berechnete Masse 318.78; gefundene Masse (M+H) 319.3; 637.0 (Dimer)

### Beispiel 29:

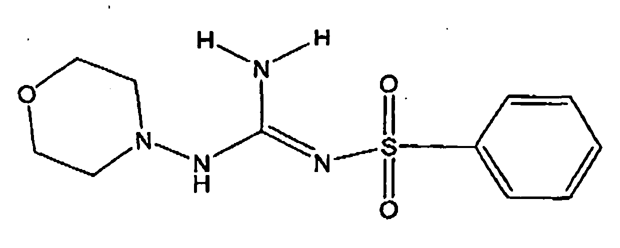

### N-[Amino-(morpholin-4-ylamino)-methylen]-benzolsulfonamid (29)

Beispiel **29** wurde gemäß der beigefügten Synthesevorschrift aus 0.5 ml *N*-[(3,5-Dimethyl-pyrazol-1-yl)-imino-methyl]-benzolsulfonamid-Lösung (0.2 M, Acetonitril) mit 19 mg Methansulfonsäure und 0.5 ml *N*-Aminomorpholin-Lösung (1.0 M, Acetonitril) in einer Substanzbibliothek dargestellt.

Berechnete Masse 284.34; gefundene Masse (M+H) 285.3; 569.1 (Dimer)

### Beispiel 30:

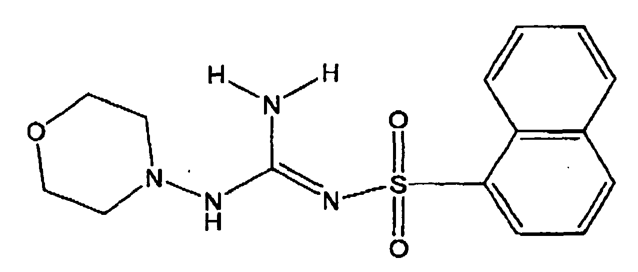

### Naphthalin-1-sulfonsäureamino-(morpholin-4-ylamino)-methylenamid (30)

Beispiel **30** wurde gemäß der beigefügten Synthesevorschrift aus 0.5 ml *N*-[(3,5-Dimethyl-pyrazol-1-yl)-imino-methyl]-naphthol-1-sulfonamid-Lösung (0.2 M, Acetonitril) mit 19 mg Methansulfonsäure und 0.5 ml *N*-Aminomorpholin-Lösung (1.0 M, Acetonitril) in einer Substanzbibliothek dargestellt.

Berechnete Masse 334,39; gefundene Masse (M+H) 335.3; 669.1 (Dimer)

### Beispiele 32 bis 63:

Die Beispiele 32 bis 63 wurden nach Varianten der bereits im allgemeinen Verfahren bzw. den vorangehenden Beispielen beschriebenen Syntheseverfahren hergestellt:

### Beispiel 32:

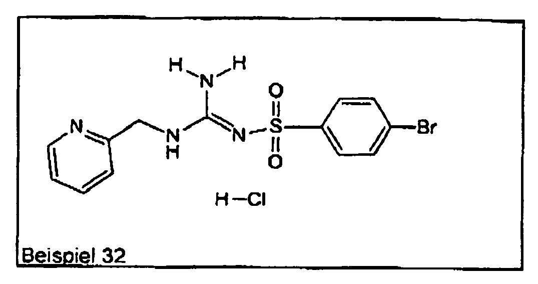

### N-{Amino-[(pyridin-2-ylmethyl)-amino]-methylen}-4-brom-benzolsulfonamid: Hydrochlorid (32)

### Beispiel 33:

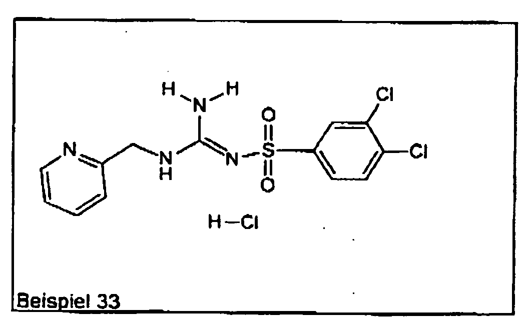

### N-{Amino-[(pyridin-2-ylmethyl)-amino]-methylen}-3,4-dichlor-benzolsulfonamid; Hydrochlorid (33)

### Beispiel 34:

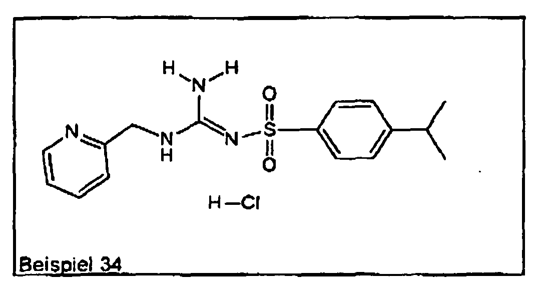

### N-{Amino[(pyridin-2-ylmethyl)-amino]-methylen}-4-isopropyl-benzolsulfonamid; Hydrochlorid (34)

### Beispiel 35:

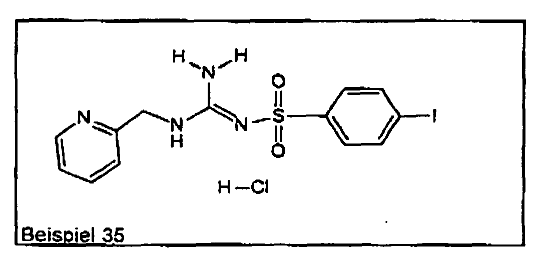

### N-{Amino-[(pyridin-2-ylmethyl)-amino]-methylen}-4-iod-benzolsulfonamid; Hydrochlorid (35)

### Beispiel 36:

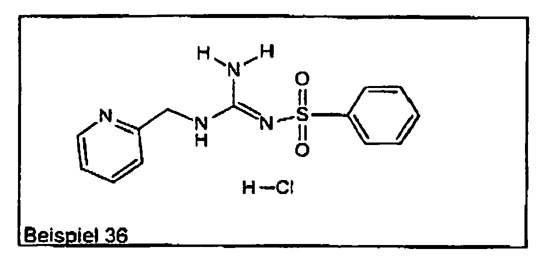

### N-{Amino-[(pyridin-2-ylmethyl)-amino]-methylene}benzolsulfonamid; Hydrochlorid (36)

### Beispiel 37:

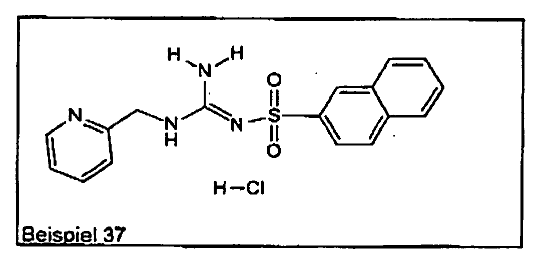

### Naphthalin-2-sulfonsäure-amino-[(pyridin-2-ylmethyl)-amino]-methylenamid; Hydrochlorid (37)

### Beispiel 38:

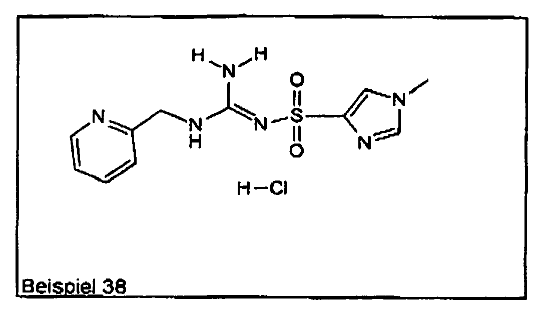

### 1-Methyl-1H-imidazol-4-sulfonsäure-amino-[(pyridin-2-ylmethyl)-amino]-methylenamid; Hydrochlorid (38)

### Beispiel 39:

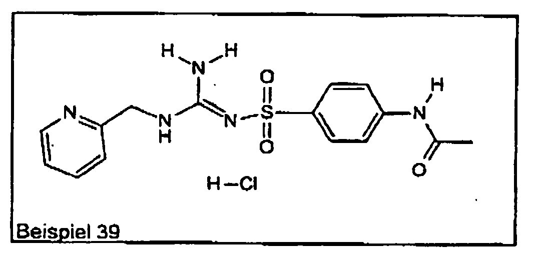

### N-[4-({Amino-[(pyridin-2-ylmethyl)-amino]-Methylen}-sulfamoyl)-phenyl]-acetamid; Hydrochlorid (39)

### Beispiel 40:

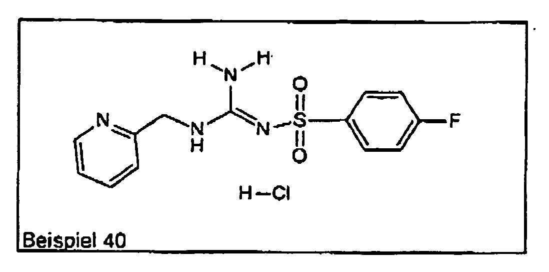

### N-{Amino-[(pyridin-2-ylmethyl)-amino]-methylen}-4-fluor-benzolsulfonamid; Hydrochlorid (40)

### Beispiel 41:

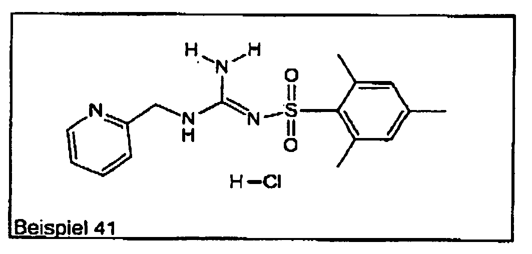

### N-(Amino-[(pyridin-2-ylmethyl)-amino]-methylen)-2,4,6-trimethyl-benzotsulfonamid; Hydrochlorid (41)

### Beispiel 42:

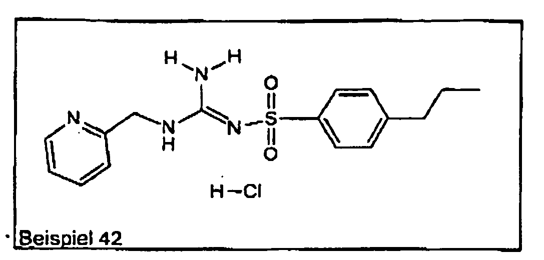

### N-{Amino-[(pyridin-2-ylmethyl)-amino]-methylen}-4-propyl-benzolsulfonamid; Hydrochlorid (42)

### Beispiel 43:

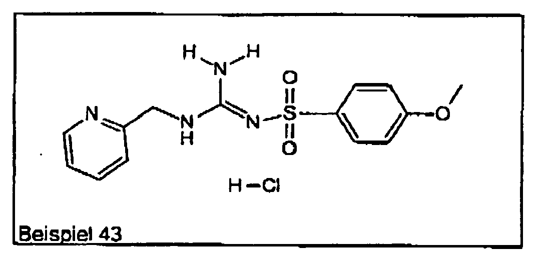

### N-{Amino-[(pyridin-2-ylmethyl)-amino]-methylen}-4-methoxy-benzolsulfonamid; Hydrochlorid (43)

### Beispiel 44:

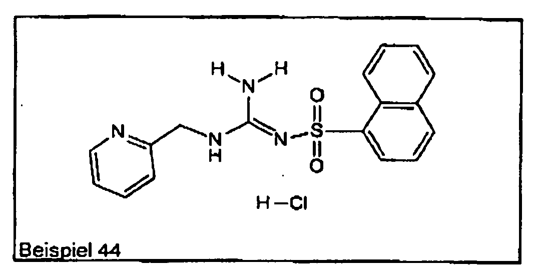

### Naphthalin-1-sulfonsäure-amino-[(pyridin-2-ylmethyl)-amino]-methylenamid; Hydrochlorid (44)

### Beispiel 45:

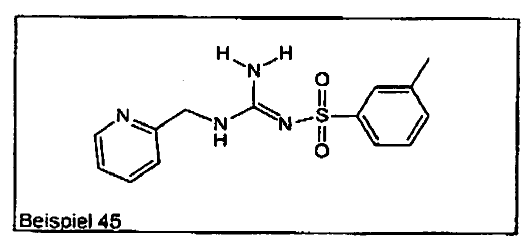

### N-{Amino-[(pyridln-2-ylmethyl)-amino]-methylen}-3-methyl-benzolsulfonamid (45)

### Beispiel 46:

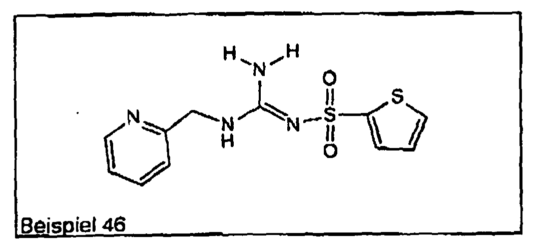

### Thiophen-2-sulfonsäure-amino-[(pyridin-2-ylmethyl)-amino]-methylenamide (46)

### Beispiel 47:

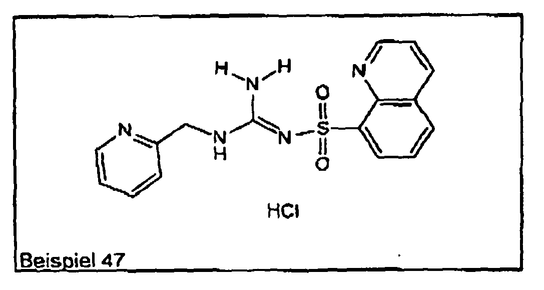

### Quinolin-8-sulfonsäure-amino-[(pyridin-2-ytmethyl)-amino]-methylenamid (47)

### Beispiel 48:

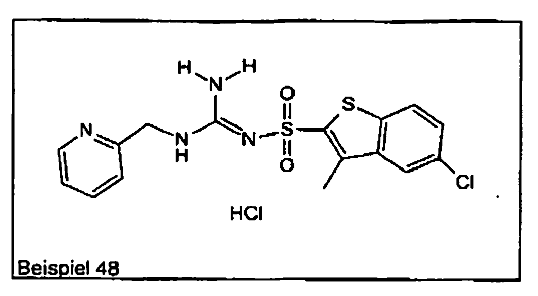

### 5-Chlor-3-methyl-benzo[b]thiophen-2-sulfonsäure-amino-[(pyridin-2-ylmethyl)-amino]-methylenamid (48)

### Beispiel 49:

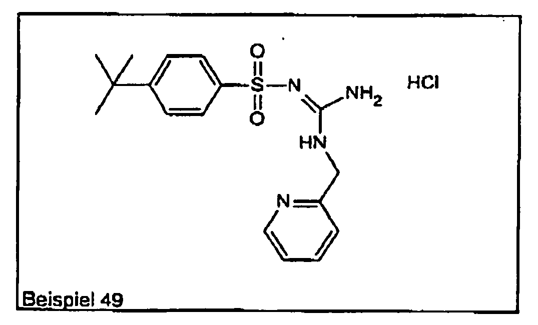

### N-{Amino-[(pyridin-2-ylmethyl)-amino]-methylen}-4-tert-butyl-benzolsulfonamid; Hydrochlorid (49)

### Beispiel 50:

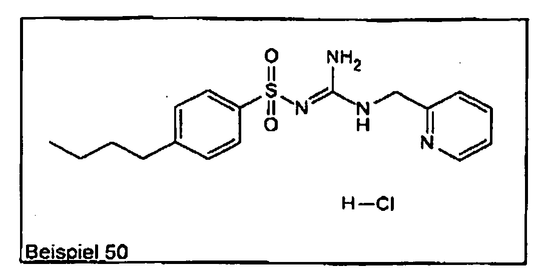

### N-{Amino-[(pyridin-2-ylmethyl)-amino]-methylen}-4-butyl-benzolsulfonamid; Hydrochlorid (50)

### Beispiel 51:

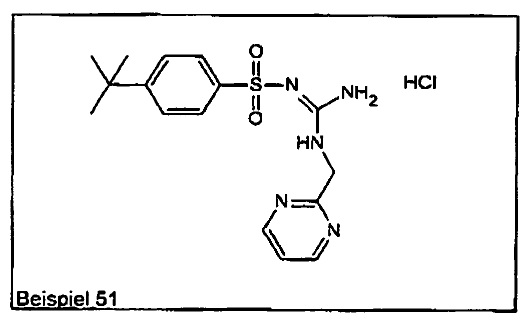

### N-{Amino-[(pyrimidin-2-ylmethyl)-amino]-methylen}-4-tert-butyl-benzolsulfonamid; Hydrochlorid (51)

### Beispiel 52:

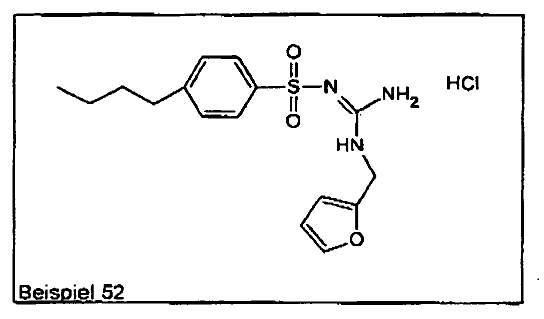

### N-{Amino-[(furan-2-ylmethyl)-amino]-methyle}-4-butyl-benzolsulfonamid; Hydrochlorid (52)

### Beispiel 53:

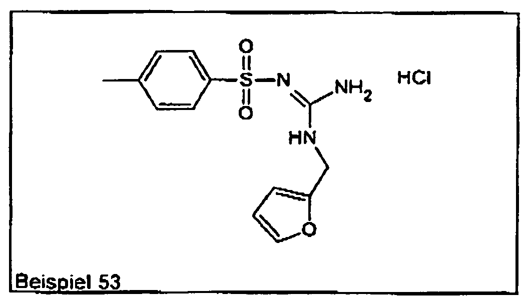

### N-{Amino-[(furan-2-ylmethyl)-amino]-methylen}-4-methyl-benzolsulfonamid; Hydrochlorid (53)

### Beispiel 54:

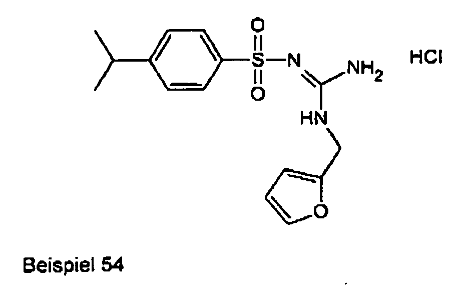

### N-{Amino-[(furan-2-ylmethyl)-amino]-methylen}-4-isopropyl-benzolsulfonamid; Hydrochlorid (54)

### Beispiel 55:

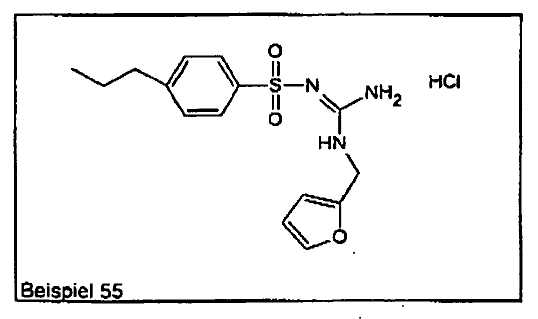

### N-{Amino-[(furan-2-ylmethyl)-amino]-methylen}-4-propyl-benzolsulfonamid; Hydrochlorid (55)

### Beispiel 56:

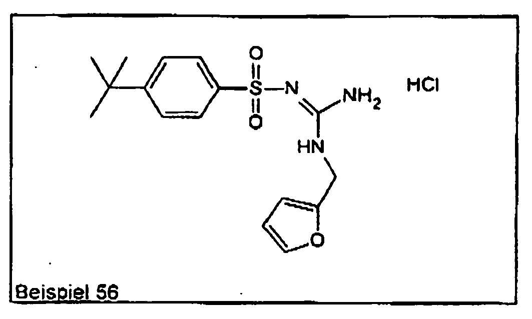

### N-{Amino-[(furan-2-ylmethyl)-amino]-methylen}-4-tert-butyl-benzolsulfonamid; Hydrochlorid (56)

### Beispiel 57:

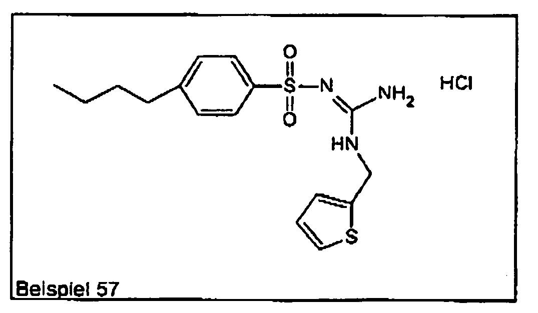

### N-{Amino-[(thiophen-2-ylmethyl)-amino]-methylen}-4-butyl-benzolsulfonamid; Hydrochlorid (57)

### Beispiel 58:

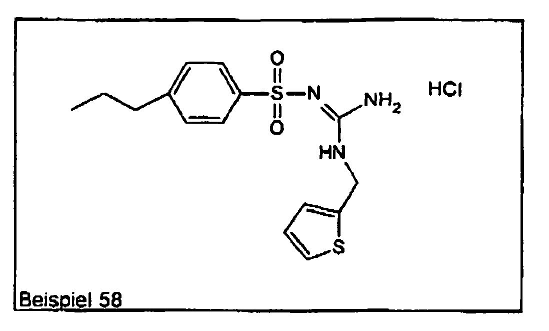

### N-{Amino-[(thiophen-2-ylmethyl)-amino]-methylen}-4-propyl-benzolsulfonamid; Hydrochlorid (58)

### Beispiel 59:

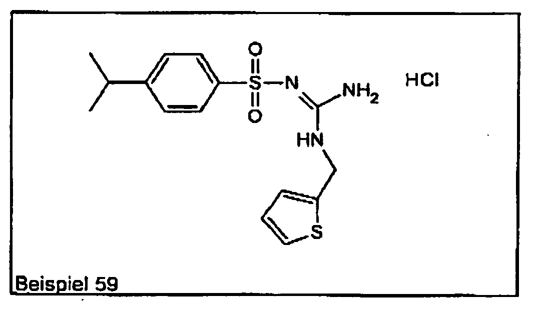

### N-{Amino-[(thiophen-2-ylmethyl)-amino]-methylene}-4-isopropyl-benzolsulfonamid; Hydrochlorid (59)

### Beispiel 60:

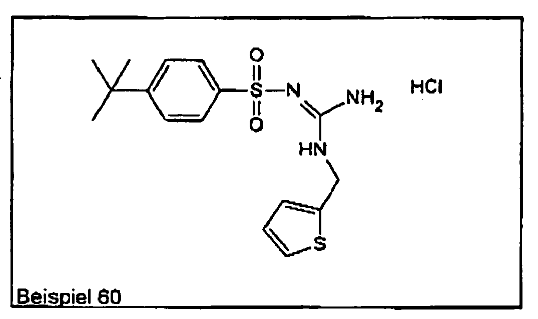

### N-{Amino-[(thiophen-2-ylmethyl)-amino]-methylen}-4-tert-butyl-benzolsulfonamid; Hydrochlorid (60)

### Beispiel 61:

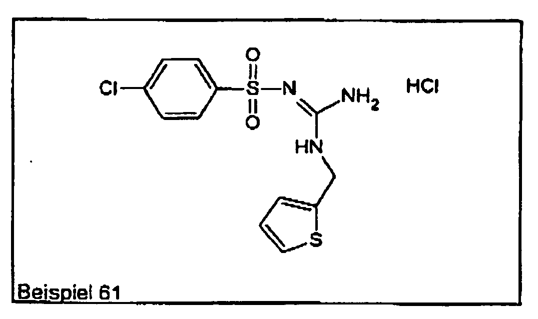

### N-{Amino-[(thiophen-2-ylmethyl)-amino]-methylen}-4-chlor-benzolsulfonamid; Hydrochlorid (61)

### Beispiel 62:

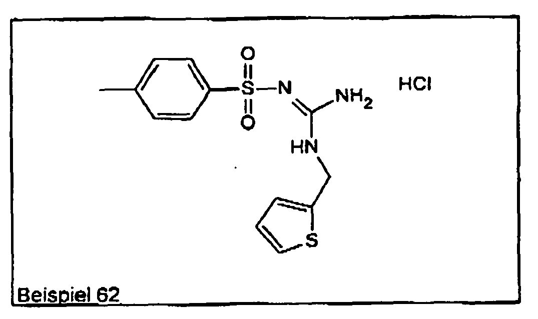

### N-{Amino-[(thiophen-2-ylmethyl)-aminoJ-methyten}-4-methyl-benzolsulfonamid; Hydrochlorid (62)

### Beispiel 63:

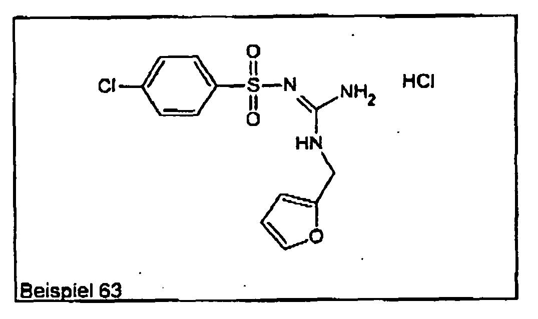

### N-{Amino-[(furan-2-ylmethyl)-amino]-methylen}-4-chlor-benzolsulfonamid; Hydrochlorid (63)

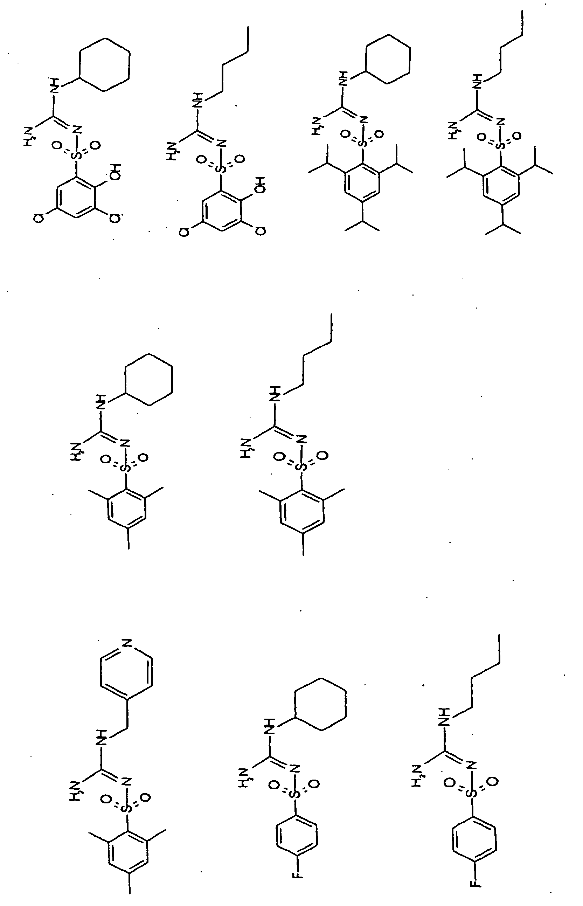

Im übrigen wurden nach den vorgenannten Verfahren, insbesondere nach dem Grundverfahren 1 eine erhebliche Anzahl weiterer Verbindungen synthetisiert, die auf den beiliegenden Abbildungen Fig1) - Fig 40) als Strukturformeln abgebildet sind. Bei diesen Beispielen handelt es sich um erfindungsgemäße Verbindungen und/oder Verbindungen, die aufgrund ihrer Wirkungsstruktur erfindungsgemäß verwendet werden können.

### Phartnakologische Untersuchungen

### Beispiel 64:

### Bindungsassay

Beim Bindungsassay wird Gabapentin benutzt, um die Bindung und Affinitäten der ausgewählten Verbindungen zu überprüfen. Die Affinität der erfindungsgemäßen Verbindungen wird über die Verdrängung von Gabapentin von seiner Bindungsstelle gemessen. Wenn die ausgewählten Verbindungen Gabapentin von seiner Bindungsstelle verdrängen können, so kann man erwarten, daß sie dem Gabapentin vergleichbare pharmakologische Eigenschaften entfalten z.B. als Agenz gegen Schmerz oder Epilepsie. Die erfindungsgemäßen Verbindungen zeigen eine gute HemmungNerdrängung von Gabapentin in diesem Assay. Die untersuchten Verbindungen weisen daher in diesem biochemischen Assay eine Affinität zur bislang unbekannten Gabapentin-Bindungsstelle auf.

**Tabelle 1;**

| **Beispiel** | **Inhibition [10⁻⁵ µM]** | **IC₅₀ [nM]** |
|---|---|---|
| 1 | 82% | 421 |
| 2 | 69.7% | |
| 3 | | |
| 4 | | |
| 5 | | |
| 6 | | |
| 7 | | |
| 8 | | |
| 9 | | |
| 10 | | |
| 11 | | |
| 12 | | |
| 13 | | |
| 14 | | |
| 15 | | |
| 16 | | |
| 17 | | |
| 18 | | |
| 19 | | |
| 20 | | |
| 21 | 75% | 779 |
| 22 | | |
| 23 | | |
| 24 | | |
| 25 | | |
| 26 | | |
| 27 | | |
| 28 | | |
| 29 | | |
| 30 | | |
| 31 | n.a. | |
| 32 | | 218 |
| 33 | | 1100 |
| 34 | | 33 |
| 35 | | 50 |
| 36 | 17% | |
| 37 | 122 | |
| 38 | n.a. | |
| 39 | 27% | |
| 40 | 21% | |
| 41 | | 438 |
| 42 | | 27 |
| 43 | | 634 |
| 44 | | 5300 |
| 45 | 35% | |
| 46 | 36% | |
| 47 | | 2800 |
| 48 | | 136 |
| 49 | | 90 |
| 50 | | 170 |
| 51 | 5% | |
| 52 | 47% | |
| 53 | 26% | |
| 54 | | 3510 |
| 55 | | 3200 |
| 56 | 49% | |
| 57 | 41% | |
| 58 | | 1820 |
| 59 | | 970 |
| 60 | | 5690 |
| 61 | 24% | |
| 62 | 29% | |
| 63 | 23,5% | |

| | | |
|---|---|---|
| n.a. bedeutet kein ausreichendes Meßsignal. | | |

### Beispiel 65:

### In vivo Experimente nach Chung

In mänlichen Sprague-Dawley-Ratten wurden an den linken L5/L6 Spinal-Nerven Spinal-Nerven-Ligaturen gemäß Kim und Chung (1992) angelegt. Zur selben Zeit wurden Spinal-Katheter gemäß Pogatzki et al.⁶ (2000) implantiert. 4 bis 6 Tage nach der Operation wurde die taktile Schwellen-Basislinie (Rückzugs-Schwellentwithdrawal threshholds) an der ipsi- und contralateralen Hinterpfote durch ein elektronisches vonFrey-Anethesiometer (IITC Life Science, USA) gemessen. Der korrekte Sitz der Spinal-Katheter wurde durch Lidocain-Gabe (10µl, 2%) bestätigt, die in einer kurzfristigen Lähmung des bilateralen Hinterglieds resultierte. Nach dem Test und Messung der Basislinie wurden Gabapentin und erfindungsgemäßes Substanzen gegeben. Die taktilen Rückzugs-Schwellen (withdrawal threshholds) wurden 30 Minuten nach der Gabe gemessen. Die Ergebnisse sind als ED50 bzw. % maximal possible effect (%MPE; % des maximal möglichen Effekts) auf der ipsilateralen Seite angegeben, in dem man die Basisline als 0% und die Rückzugs-Schwelle einer Kontroll-Gruppe als 100%MPE annimmt.

Die Substanz nach Beispiel 1 zeigte einen 50 % Effekt bei 25 mg/kg, was in etwa dem ED50 entspricht und Gabapentin zeigte einen ED50 von 92,6 mg/kg. Beide Substanzen wirkten mindestens 30 h. Damit ist die Substanz nach Beispiel 1 Gabapentin in diesem Modell um den Faktor 4 überlegen.

Auch in einer weiteren Untersuchung zeigen die Substanzen nach Beispiel 1 und nach Beispiel 21 einen Effekt im Chung. Beide Substanzen wirken mindestens 30 h und sind in ihrer Wirkung Gabapentin (ED₅₀ 92.6 mg/kg) überlegen.

**Tabelle 2 : Analgesieprüfung im Chung Ratte:**

| Beispiel Nr. | Chung Ratte ED₅₀ |
|---|---|
| 21 | 13.3 mg/kg |
| 1 | 10 - 20 mg/kg |

### Literatur:

Kim, S. H.; Chung, J. M. (1992) An experimental model for peripheral mononeuropathy produced by segmental spinal nerve ligation in the rat. Pain 50, 355-363.

Pogatzki, E. M.; Zahn, P. K.; Brennan, T. J. (2000) Lumbar catheterization of the subarachnoid space with a 32-gauge polyurethane catheter in the rat. Eur. J. Pain 4, 111-113.

### Beispiel 66:

### Formalin Test Maus

Die Untersuchungen zur Bestimmung der antinociceptiven Wirkung der erfindungsgemäßen Verbindungen wurden im Formalin-Test an männlichen Albino-Mäusen (NMRI, 25 - 35 g, lffa Credo, Belgien) durchgeführt.

Im Formalin-Test werden die erste (frühe) Phase (0-15 min nach Formalin-Injektion) und die zweite (späte) Phase (15 - 60 min nach Formalin-Injektion) unterschieden (D. Dubuisson er al, Pain, Vol. 4, pp. 161 - 174 (1977)). Die frühe Phase stellt als direkte Reaktion auf die Formalin-Injektion ein Modell für Akutschmerz dar, während die späte Phase als Modell für persistierenden (chronischen) Schmerz angesehen wird (T.J. Coderre et al, Pain, Vol. 52, pp. 259 - 285 (1993)).

Die erfindungsgemäßen Verbindungen wurden in der zweiten Phase des Formalin-Tests untersucht, um Aussagen über Substanzwirkungen im chronischlentzündlichen Schmerz zu erhalten.

Durch eine einmalige subkutane Formalin-Injektion (20 µl, 1 %ige wäßrige Lösung) in die dorsale Seite der rechten Hinterpfote wurde bei freibeweglichen Versuchstieren eine nociceptive Reaktion induziert, die sich in deutlichem Lecken und Beißen der betroffenen Pfote äußert.

Für den Untersuchungszeitraum in der zweiten (späten) Phase des Formalin-Tests wurde das nozizeptive Verhalten durch Beobachtung der Tiere kontinuierlich erfaßt. Die Quantifizierung des Schmerzverhaltens erfolgte durch Summation der Sekunden, in denen die Tiere im Untersuchungszeitraum Lecken und Beißen der betroffenen Pfote zeigten. Nach Injektion von Substanzen, die im Formalin-Test antinozizeptiv wirksam sind, sind die beschriebenen Verhaltensweisen der Tiere reduziert, evtl. sogar aufgehoben. Entsprechend den Substanzversuchen, bei denen die Tiere Testsubstanz vor Formalin injiziert bekommen hatten, wurde den Kontrolltieren Vehikel, d.h. Lösungmittel (z.B. 0,9%ige NaCl-Lösung), vor der Formalin-applikation gespritzt. Das Verhalten der Tiere nach Substanzgabe (n=10 pro Substanzdosierung) wurde mit einer Kontrollgruppe (n=10) verglichen.

Basierend auf der Quantifizierung des Schmerzverhaltens wurde die Substanzwirkung im Formalin-Test als Änderung der Kontrolle in Prozent ermittelt. Die ED₅₀-Berechnungen erfolgten mittels Regressionsanalyse. Abhängig von der Applikationsart der erfindungsgemäßen Verbindungen wurde der Applikationszeitpunkt vor der Formalin-Injektion gewählt (intraperitoneal: 15 min, intravenös: 5 min).

Die erfindungsgemäße Verbindung **Beispiel 21** zeigte eine Hemmung der durch Formalin induzierten Nociception. Die entsprechenden Ergebnisse im Formalin-Test an der Maus sind in der nachfolgenden Tabelle 3 dargestellt. Gabapentin zeigt einen ED50 Wert von 79 mg/kg (i.v.).

**Tabelle 3: Analgesieprüfung im Formalin Test Maus**

| Beispiel Nr. | Formalin Test Maus ED₅₀ |
|---|---|
| 21 | 97 mg/kg |

## Patentansprüche

1. Verwendung eines Sulfonylguanidins gemäß den allgemeinen tautomeren Formeln I und la, 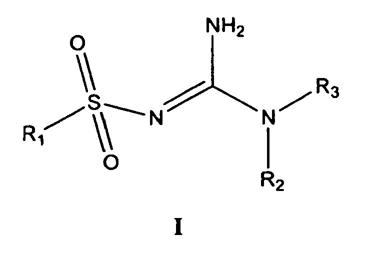 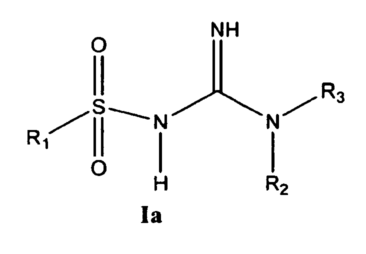 , worin
R¹ ausgewählt ist aus Aryl oder Heteroaryl, jeweils unsubstituiert oder einfach oder mehrfach substituiert
und
R² ausgewählt ist aus C₁₋₁₀-Alkyl, verzweigt oder unverzweigt, gesättigt oder ungesättigt, unsubstituiert; C₃-C₈-Cycloalkyl, gesättigt oder ungesättigt, unsubstituiert; Aryl oder Heteroaryl, jeweils unsubstituiert oder einfach oder mehrfach substituiert; oder über gesättigtes oder ungesättigtes C₁₋₃-Alkyl, S(O)₂ oder NH gebundenem Aryl, C₃₋₉-Cycloalkyl oder Heteroaryl, jeweils unsubstituiert; oder
NR⁴R⁵,
mit R⁴, R⁵ unabhängig voneinander ausgewählt aus H; C₁₋₁₈-Alkyl, verzweigt oder unverzweigt, gesättigt oder ungesättigt, unsubstituiert; C₃-C₈-Cycloalkyl, gesättigt oder ungesättigt, unsubstituiert; Aryl oder Heteroaryl, jeweils unsubstituiert; oder über gesättigtes oder ungesättigtes C₁₋₃-Alkyl gebundenem Aryl, C₃₋₉-Cycloalkyl oder Heteroaryl, jeweils unsubstituiert; oder
R⁴ und R⁵ zusammen: -CH₂CH₂OCH₂CH₂-; -CH₂CH₂N(R⁷)CH₂CH₂-bilden
mit R⁷ ausgewählt aus C₁₋₁₈-Alkyl, verzweigt oder unverzweigt, gesättigt oder ungesättigt, unsubstituiert; C₃-C₈-Cycloalkyl, gesättigt oder ungesättigt, oder unsubstituiert; Aryl oder Heteroaryl, jeweils unsubstituiert; oder über gesättigtes oder ungesättigtes C₁₋₃-Alkyl gebundenem Aryl, C₃₋₉-Cycloalkyl oder Heteroaryl, jeweils unsubstituiert;
oder
SO₂R⁶
mit R⁶ ausgewählt aus C₁₋₁₈-Alkyl, verzweigt oder unverzweigt, gesättigt oder ungesättigt, unsubstituiert; C₃-C₈-Cycloalkyl, gesättigt oder ungesättigt, unsubstituiert; Aryl oder Heteroaryl, jeweils unsubstituiert; oder über gesättigtes oder ungesättigtes C₁₋₃-Alkyl gebundenem Aryl, C₃₋₉-Cycloalkyl oder Heteroaryl, jeweils unsubstituiert;
und
R³ ausgewählt ist aus H oder CH₃;
oder
R² und R³ zusammen
-CH₂(CH₂)ₙCH₂- n=2, 3
-CH₂CH₂OCH₂CH₂- oder
-CH₂CH₂N(R⁸)CH₂CH₂- bilden
mit R⁸ ausgewählt aus C₁₋₁₈-Alkyl, verzweigt oder unverzweigt, gesättigt oder ungesättigt, unsubstituiert;
gegebenenfalls in Form ihrer Racemate, ihrer reinen Stereoisomeren, oder in Form von Mischungen der Stereoisomeren in einem beliebigen Mischungsverhältnis; in dargestellter Form oder in Form ihrer Säuren oder ihrer Basen oder in Form ihrer physiologisch verträglichen Salze, oder in Form ihrer Solvate; in beiden tautomeren Formen nach Formeln I und Ia, ausschließlich in einer der tautomeren Formen nach Formeln I oder Ia oder auch in Mischungen beider Formen nach Formeln I und Ia, wobei die bevorzugte tautomere Form von Verbindung zu Verbindung z.B. in Abhängigkeit vom Aggregatzustand oder vom gewählten Lösungsmittel variieren kann;
zur Herstellung eines Arzneimittels zur Behandlung von Schmerz, von Epilepsie und/oder von Migräne
oder
zur Herstellung eines Arzneimittels zur Behandlung von Hyperalgesie und Allodynie oder von inflammatorischem oder postoperativem Schmerz
oder
zur Herstellung eines Arzneimittels zur Behandlung von Hitzewallungen, Beschwerden in der Postmenopause, Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Morbus Alzheimer, Morbus Huntington, Morbus Parkinson und Epilepsie; gastrointestinaler Schädigung; von erythromelalgischem oder postpoliomyelitischem Schmerz, trigeminaler oder postherpetischer Neuralgie; oder als Antikonvulsivum, Analgetikum oder Anxiolytikum.

2. Verwendung gemäß Anspruch 1, **dadurch gekennzeichnet, dass** sie zur Herstellung eines Arzneimittels zur Behandlung von neuropathischem, chronischem oder akuten Schmerz
oder
thermischer Hyperalgesie, mechanischer Hyperalgesie und Allodynie und Kälteallodynie erfolgt

3. Verwendung gemäß Anspruch 1 oder 2, **dadurch gekennzeichnet, dass**:
R² ausgewählt ist aus C₁₋₁₀-Alkyl, verzweigt oder unverzweigt, gesättigt oder ungesättigt, unsubstituiert; C₃-C₈-Cycloalkyl, gesättigt oder ungesättigt, unsubstituiert; Aryl oder Heteroaryl, jeweils unsubstituiert; oder über gesättigtes oder ungesättigtes C₁₋₃-Alkyl gebundenem Aryl, C₃₋₉-Cycloalkyl oder Heteroaryl, jeweils unsubstituiert.

4. Verwendung gemäß einem der Ansprüche 1 bis 3, **dadurch gekennzeichnet, dass**
R² ausgewählt ist aus über gesättigtes oder ungesättigtes C₁₋₃-Alkyl gebundenem Aryl oder Heteroaryl, jeweils unsubstituiert.

5. Verwendung gemäß Anspruch 4, **dadurch gekennzeichnet, dass** das Aryl oder Heteroaryl über eine -CH₂-Gruppe gebunden ist.

6. Verwendung gemäß einem der Ansprüche 4 oder 5, **dadurch gekennzeichnet, dass** das in R² über C₁₋₃-Alkyl gebundene Heteroaryl ausgewählt ist aus Pyridinyl, Thiophenyl, Furanyl oder Pyrimidinyl, jeweils unsubstituiert.

7. Verwendung gemäß Anspruch 6, **dadurch gekennzeichnet, dass** das Heteroaryl Pyridinyl bedeutet.

8. Verwendung eines Sulfonylguanidins gemäß einem der Ansprüche 1 bis 7, **dadurch gekennzeichnet, daß** sie ausgewählt sind aus der folgenden Gruppe:
• *N*-{Amino-[pyridin-2ylmethyl)-amino]-methylen}-4-methylbenzolsulfonamid **(1)**
• *N*-[Amino-(benzylamino-methylen)]-4-methylbenzolsulfonamid **(2)**
• *N*-(Amino-morpholin-4yl-methylen)-4-methylbenzolsulfonamid **(3)**
• *N*-(Amino-cyclohexylaminomethylen)-4-methylbenzolsulfonamid **(4)**
• *N*-(Amino-phenylaminomethylen)-4-methylbenzolsulfonamid **(5)**
• *N*-[Amino-(naphthalin-2-ylamino)methylen]-4-methylbenzolsulfonamid **(7)**
• *N*-[Amino-(*N*'pyridin-2-yl-hydrazino)-methylen]-4-methylbenzolsulfonamid **(9)**
• *N*-[Amino-propylamino-methylen]-4-methylbenzolsulfonamid **(10)**
• *N*-(Amino-butylamino-methylen)-4-methylbenzolsulfonamid **(11)**
• *N*-[Amino-piperidin-methylen]-4-methylbenzolsulfonamid **(12)**
• *N*-[Amino-(*N*-methyl-N-phenyl-amino)-methylen]-4-methylbenzolsulfonamid **(13)**
• *N*-[Amino-phenethylamino-methylen]-4-methylbenzolsulfonamid **(14)**
• *N*-[Amino-sec-butylamino-methylen]-4-methylbenzolsulfonamid **(15)**
• *N*-[Amino-(2H-pyrazol-3-ylamino)-methylen]-4-methylbenzolsulfonamid **(17)**
• *N*-(Amino-pyrrolidin-1-yl-methylen)-4-methylbenzolsulfonamid **(18)**
• *N*-[Amino-(naphthalin-1-ylamino)-methylen]-4-methylbenzolsulfonamid **(19)**
• *N*-[Amino-(morpholin-4-ylamino)-methylen]-4-methylbenzolsulfonamid **(20)**
• N-{Amino-[pyridin-2ylmethyl)-amino]-methylen}-4-chlorbenzolsulfonamid **(21)**
• N-{Amino-[pyridin-4ylmethyl)-amino]-methylen}-4-methylbenzolsulfonamid **(22)**
• N-{Amino-(pyridin-3ylmethyl)-amino]-methylen}-4-methylbenzolsulfonamid **(23)**
• N-[Amino-(pyridin-3ylamino)-methylen]-4-methylbenzolsulfonamid **(24)**
• *N*-(Amino-pyrrolidin-1-yl-methylen)-4-chlorbenzolsulfonamid **(25)**
• *N*-(Amino-pyrrolidin-1-yl-methylen)-benzolsulfonamid **(26)**
• *N*-(Amino-pyrrolidin-1-yl-methylen)-2-nitrobenzolsulfonamid **(27)**
• *N*-[Amino-(morpholin-4-ylamino)-methylen]-4-chlorbenzolsulfonamid **(28)**
• *N*-[Amino-(morpholin-4-ylamino)-methylen]-benzolsulfonamid **(29)**
• Naphthalin-1-sulfonsäureamino-(morpholin-4-ylamino)-methylenamid **(30)**
• N-{Amino-[(pyridin-2-ylmethyl)-amino]-methylen}-4-brom-benzolsulfonamid **(32)**
• N-{Amino-[(pyridin-2-ylmethyl)-amino]-methylen}-3,4-dichlor-benzolsulfonamid **(33)**
• N-{Amino-[(pyridin-2-ylmethyl)-amino]-methylen}-4-isopropyl-benzolsulfonamid **(34)**
• N-{Amino-[(pyridin-2-ylmethyl)-amino]-methylen}-4-iod-benzolsulfonamid **(35)**
• N-{Amino-[(pyridin-2-ylmethyl)-amino]-methylene}-benzolsulfonamid **(36)**
• Naphthalin-2-sulfonsäure-amino-[(pyridin-2-ylmethyl)-amino}-methylenamid **(37)**
• 1-Methyl-1H-imidazol-4-sulfonsäure-amino-[(pyridin-2-ylmethyl)-amino]-methylenamid **(38)**
• N-[4-({Amino-[(pyridin-2-ylmethyl)-amino]-methylen}-sulfamoyl)-phenyl]-acetamid **(39)**
• N-{Amino-[(pyridin-2-ylmethyl)-amino]-methylen}-4-fluor-benzolsutfonamid **(40)**
• N-{Amino-[(pyridin-2-ylmethyl)-amino]-methylen}-2,4,6-trimethyl-benzolsulfonamid **(41)**
• N-{Amino-[(pyridin-2-ylmethyl)-amino]-methylen}-4-propyl-benzolsulfonamid **(42)**
• N-{Amino-[(pyridin-2-ylmethyl)-amino]-methylen}-4-methoxy-benzolsulfonamid **(43)**
• Naphthalin-1-sulfonsäure-amino-[(pyridin-2-ylmethyl)-amino]-methylenamid **(44)**
• N-{Amino-[(pyridin-2-ylmethyl)-amino]-methylen}-3-methyl-benzolsulfonamid **(45)**
• Thiophen-2-sulfonsäure-amino-[(pyridin-2-ylmethyl)-amino]-methylenamid **(46)**
• Quinolin-8-sulfonsäure-amino-[(pyridin-2-ylmethyl)-amino]-methylenamid **(47)**
• 5-Chlor-3-methyl-benzo[b]thiophen-2-sulfonsäure-amino-[(pyridin-2-ylmethyl)-amino]-methylenamid **(48)**
• N-{Amino-[(pyridin-2-ylmethyl)-amino]-methylen}-4-tert-butyl-benzolsulfonamid **(49)**
• N-{Amino-[(pyridin-2-ylmethyl)-amino]-methylen}-4-butyl-benzolsulfonamid **(50)**
• N-{Amino-[(pyrimidin-2-ylmethyl)-amino]-methylen}-4-tert-butyl-benzolsulfonamid **(51)**
• N-{Amino-[(furan-2-ylmethyl)-amino]-methylen}-4-butyl-benzolsulfonamid **(52)**
• N-{Amino-[(furan-2-ylmethyl)-amino]-methylen}-4-methyl-benzolsulfonamid **(53)**
• N-{Amino-[(furan-2-ylmethyl)-amino]-methylen}-4-isopropyl-benzotsulfonamid **(54)**
• N-{Amino-[(furan-2-ylmethyl)-amino]-methylen}-4-propyl-benzolsulfonamid **(55)**
• N-{Amino-[(furan-2-ylmethyl)-amino]-methylen}-4-tert-butyl-benzolsulfonamid **(56)**
• N-{Amino-[(thiophen-2-ylmethyl)-amino]-methylen}-4-butyl-benzolsulfonamid **(57)**
• N-{Amino-[(thiophen-2-ylmethyl)-amino]-methylen}-4-propyl-benzolsulfonamid **(58)**
• N-Amino-[(thiophen-2-ylmethyl)-amino]-methylene)-4-isopropyl-benzolsulfonamid **(59)**
• N-{Amino-[(thiophen-2-ylmethyl)-amino]-methylen}-4-tert-butyl-benzylsulfonamid **(60)**
• N-{Amino-[(thiophen-2-ylmethyl)-amino]-methylen}-4-chlor-benzolsulfonamid **(61)**
• N-{Amino-[(thiophen-2-ylmethyl)-amino]-methylen}-4-methyl-benzolsulfonamid **(62)**
• N-Amino-[(furan-2-ylmethyl)-amino]-methylen}-4-chlor-benzolsulfonamid **(63)**
gegebenenfalls in Form ihrer Racemate, ihrer reinen Stereoisomeren oder in Form von Mischungen der Stereoisomeren in einem beliebigen Mischungsverhältnis, in dargestellter Form oder in Form ihrer Säuren oder ihrer Basen, oder in Form ihrer Salze oder in Form ihrer Solvate in beiden tautomeren Formen nach Formeln I und Ia, ausschließlich in einer der tautomeren Formen nach Formeln I oder Ia oder auch in Mischungen beider Formen nach Formeln I und Ia, wobei die bevorzugte tautomere Form von Verbindung zu Verbindung z.B. in Abhängigkeit vom Aggregatzustand oder vom gewählten Lösungsmittel variieren kann.

9. Verwendung gemäß einem der Ansprüche 1 bis 8, **dadurch gekennzeichnet, dass** die verwendete Verbindung ganz oder überwiegend in der tautomeren Form gemäß Formel I vorliegt.

10. Verwendung gemäß einem der Ansprüche 1 bis 8, **dadurch gekennzeichnet, dass** die verwendete Verbindung ganz oder überwiegend in der tautomeren Form gemäß Formel Ia vorliegt.

11. Verwendung gemäß einem der Ansprüche 1 bis 10, **dadurch gekennzeichnet, dass** ein verwendetes Sulfonylguanidin als reines Diastereomer und/oder Enantiomer, als Razemat oder als nicht-äquimolare oder äquimolare Mischung der Diastereomere und/oder Enantiomere und/oder ausschließlich in einer der tautomeren Formen nach Formeln oder Ia oder auch in Mischungen beider Formen nach Formeln I und Ia vorliegt.

12. Sulfonylguanidine, **dadurch gekennzeichnet, dass** die Verbindungen ausgewählt sind aus:
• *N*-{Amino-[pyridin-2ylmethyl)-amino]-methylen}-4-methylbenzolsulfonamid **(1)**
• *N*-[Amino-(benzylamino-methylen)]-4-methylbenzolsulfonamid **(2)**
• *N*-[Amino-phenethylamino-methylen]-4-methylbenzolsulfonamid **(14)**
• N-{Amino-[pyridin-2ylmethyl)-amino]-methylen}-4-chlorbenzolsulfonamid **(21)**
• N-{Amino-[pyridin-4ylmethyl)-amino]-methylen}-4-methylbenzolsulfonamid **(22)**
• N-{Amino-[pyridin-3ylmethyl)-amino]-methylen}-4-methylbenzolsulfonamid **(23)**
• N-{Amino-[(pyridin-2-ylmethyl)-amino]-methylen}-4-brom-benzolsulfonamid **(32)**
• N-{Amino-[(pyridin-2-ylmethyl)-amino]-methylen}-3,4-dichlor-benzolsulfonamid **(33)**
• N-{Amino-[(pyridin-2-ylmethyl)-amino]-methylen}-4-isopropyl-benzolsulfonamid **(34)**
• N-{Amino-[(pyridin-2-ylmethyl)-amino]-methylen}-4-iod-benzolsulfonamid **(35)**
• N-{Amino-[(pyridin-2-ylmethyl)-amino]-methylene}-benzolsulfonamid **(36)**
• N-{Amino-[(thiophen-2-ylmethyl)-amino]-methylen}-4-tert-butyl-benzolsulfonamid **(60)**
• N-{Amino-[(thiophen-2-ylmethyl)-amino]-methylen}-4-chlor-benzolsulfonamid **(61)**
• N-{Amino-[(thiophen-2-ylmethyl)-amino]-methylen}-4-methyl-benzolsulfonamid **(62)**
• N-{Amino-[(furan-2-ylmethyl)-amino]-methylen}-4-chlor-benzolsulfonamid **(63)**
gegebenenfalls in Form ihrer Racemate, ihrer reinen Stereoisomeren oder in Form von Mischungen der Stereoisomeren in einem beliebigen Mischungsverhältnis, in dargestellter Form oder in Form ihrer Säuren oder ihrer Basen, oder in Form ihrer Salze oder in Form ihrer Solvate; in beiden tautomeren Formen nach Formeln I und la, ausschließlich in einer der tautomeren Formen nach Formeln I oder Ia oder auch in Mischungen beider Formen nach Formeln I und Ia, wobei die bevorzugte tautomere Form von Verbindung zu Verbindung z.B. in Abhängigkeit vom Aggregatzustand oder vom gewählten Lösungsmittel variieren kann.

13. Sulfonylguanidine gemäß Anspruch 12, **dadurch gekennzeichnet, dass** ein Sulfonylguanidin ganz oder überwiegend in der tautomeren Form gemäß Formel I vorliegt.

14. Sulfonylguanidine gemäß Anspruch 12, **dadurch gekennzeichnet, dass** ein Sulfonylguanidin ganz oder überwiegend in der tautomeren Form gemäß Formel Ia vorliegt.

15. Arzneimittel enthaltend wenigstens ein Sulfonylguanidin gemäß einem der Ansprüche 12 bis 14, sowie gegebenenfalls geeignete Zusatz- und/oder Hilfsstoffe und/oder gegebenenfalls weitere Wirkstoffe.

16. Arzneimittel gemäß Anspruch 15, **dadurch gekennzeichnet, dass** ein enthaltenes Sulfonylguanidin gemäß einem der Ansprüche 12 bis 14, als reines Diastereomer und/oder Enantiomer, als Razemat oder als nicht-äquimolare oder äquimolare Mischung der Diastereomere und/oder Enantiomere vorliegt.

## Claims

1. Use of a sulfonylguanidine according to the general tautomeric formulae I and Ia 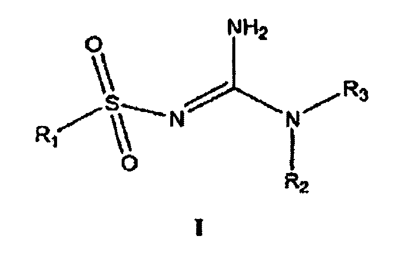 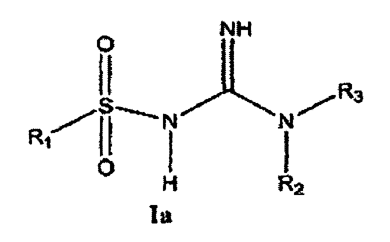 wherein
R¹ is chosen from aryl or heteroaryl, in each case unsubstituted or mono- or polysubstituted
and
R² is chosen from C₁₋₁₀-alkyl, branched or unbranched, saturated or unsaturated, unsubstituted; C₃-C₈-cycloalkyl, saturated or unsaturated, unsubstituted; aryl or heteroaryl, in each case unsubstituted or mono- or polysubstituted; or aryl, C₃₋₉-cycloalkyl or heteroaryl, bonded via saturated or unsaturated C₁₋₃-alkyl, S(O)₂ or NH and in each case unsubstituted; or
NR⁴R⁵,
where R⁴, R⁵ independently of each other are chosen from H; C₁₋₁₈-alkyl, branched or unbranched, saturated or unsaturated, unsubstituted; C₃-C₈-cycloalkyl, saturated or unsaturated, unsubstituted; aryl or heteroaryl, in each case unsubstituted; or aryl, C₃₋₉-cycloalkyl or heteroaryl, bonded via saturated or unsaturated C₁₋₃-alkyl and in each case unsubstituted; or
R⁴ and R⁵ together: form -CH₂CH₂OCH₂CH₂-; -CH₂CH₂N(R⁷)CH₂CH₂- where R⁷ is chosen from C₁₋₁₈-alkyl, branched or unbranched, saturated or unsaturated, unsubstituted; C₃-C₈-cycloalkyl, saturated or unsaturated, or unsubstituted; aryl or heteroaryl, in each case unsubstituted; or aryl, C₃₋₉-cycloalkyl or heteroaryl, bonded via saturated or unsaturated C₁₋₃-alkyl and in each case unsubstituted;
or
SO₂R⁶
where R⁶ is chosen from C₁₋₁₈-alkyl, branched or unbranched, saturated or unsaturated, unsubstituted; C₃-C₈-cycloalkyl, saturated or unsaturated, unsubstituted; aryl or
heteroaryl, in each case unsubstituted; or aryl, C₃₋₉-cycloalkyl or heteroaryl, bonded via saturated or unsaturated C₁₋₃-alkyl and in each case unsubstituted;
and
R³ is chosen from H or CH₃;
or
R² and R³ together form
-CH₂(CH₂)ₙCH₂- n = 2, 3
-CH₂CH₂OCH₂CH₂- or
-CH₂CH₂N(R⁸)CH₂CH₂-
where R⁸ is chosen from C₁₋₁₈-alkyl, branched or unbranched, saturated or unsaturated, unsubstituted;
optionally in the form of their racemates, their pure stereoisomers, or in the form of mixtures of the stereoisomers in any desired mixture ratio; in the form shown or in the form of their acids or their bases or in the form of their physiologically acceptable salts, or in the form of their solvates; in the two tautomeric forms according to formulae I and Ia, exclusively in one of the tautomeric forms according to formulae I or Ia or also in mixtures of the two forms according to formulae I and Ia, wherein the preferred tautomeric form can vary from compound to compound, e.g. depending on the state of aggregation or on the solvent chosen;
for the preparation of a medicament for treatment of pain, of epilepsy and/or of migraine
or
for the preparation of a medicament for treatment of hyperalgesia and allodynia or of inflammatory or postoperative pain
or
for the preparation of a medicament for treatment of hot flushes, symptoms in the postmenopause, amyotrophic lateral sclerosis (ALS), reflex sympathetic dystrophy (RSD), spastic paralysis, restless leg syndrome, acquired nystagmus; psychiatric or neuropathological disorders, such as bipolar disorders, anxiety, panic attacks, mood swings, manic behaviour, depression, manic-depressive behaviour; painful diabetic neuropathy, symptoms and pain due to multiple sclerosis or Parkinson's disease, neurodegenerative diseases, such as Alzheimer's disease, Huntington's disease, Parkinson's disease and epilepsy; gastrointestinal damage; of erythromelalgic or postpoliomyelitic pain, trigeminal or postherpetic neuralgia; or as an anticonvulsive, analgesic or anxiolytic.

2. Use according to claim 1, **characterized in that** it takes place for the preparation of a medicament for treatment of neuropathic, chronic or acute pain or thermal hyperalgesia, mechanical hyperalgesia and allodynia and cold allodynia.

3. Use according to claim 1 or 2, **characterized in that**:
R² is chosen from C₁₋₁₀-alkyl, branched or unbranched, saturated or unsaturated, unsubstituted; C₃-C₈-cycloalkyl, saturated or unsaturated, unsubstituted; aryl or heteroaryl, in each case unsubstituted; or aryl, C₃₋₉-cycloalkyl or heteroaryl, bonded via saturated or unsaturated C₁₋₃-alkyl and in each case unsubstituted.

4. Use according to one of claims 1 to 3, **characterized in that**
R² is chosen from aryl or heteroaryl, bonded via saturated or unsaturated C₁₋₃-alkyl and in each case unsubstituted.

5. Use according to claim 4, **characterized in that** the aryl or heteroaryl is bonded via a -CH₂- group.

6. Use according to one of claims 4 or 5, **characterized in that** the heteroaryl bonded via C₁₋₃-alkyl in R² is chosen from pyridinyl, thiophenyl, furanyl or pyrimidinyl, in each case unsubstituted.

7. Use according to claim 6, **characterized in that** the heteroaryl denotes pyridinyl.

8. Use of a sulfonylguanidine according to one of claims 1 to 7, **characterized in that** it is chosen from the following group:
• *N*-{amino-[(pyridin-2ylmethyl)-amino]-methylene}-4-methylbenzenesulfonamide **(1)**
• *N*-[amino-(benzylamino-methylene)]-4-methylbenzenesulfonamide **(2)**
• *N*-(amino-morpholin-4-yl-methylene)-4-methylbenzenesulfonamide **(3)**
• *N*-(amino-cyclohexylaminomethylene)-4-methylbenzenesulfonamide **(4)**
• *N*-(amino-phenylaminomethylene)-4-methylbenzenesulfonamide **(5)**
• *N*-[amino-(naphthalen-2-ylamino)methylene]-4-methylbenzenesulfonamide **(7)**
• *N*-[amino-(*N*'-pyridin-2-yl-hydrazino)-methylene]-4-methylbenzenesulfonamide **(9)**
• *N*-[amino-propylamino-methylene]-4-methylbenzenesulfonamide **(10)**
• *N*-(amino-butylamino-methylene)-4-methylbenzenesulfonamide **(11)**
• *N*-[amino-piperidine-methylene]-4-methylbenzenesulfonamide **(12)**
• *N*-[amino-(N-methyl-N-phenyl-amino)-methylene]-4-methylbenzenesulfonamide **(13)**
• *N*-[amino-phenethylamino-methylene]-4-methylbenzenesulfonamide **(14)**
• *N*-[amino-sec-butylamino-methylene]-4-methylbenzenesulfonamide **(15)**
• *N*-[amino-(2H-pyrazol-3-ylamino)-methylene]-4-methylbenzenesulfonamide **(17)**
• *N*-(amino-pyrrolidin-1-yl-methylene)-4-methylbenzenesulfonamide **(18)**
• *N*-[amino-(naphthalen-1-ylamino)-methylene]-4-methylbenzenesulfonamide **(19)**
• *N*-[amino-(morpholin-4-ylamino)-methylene]-4-methylbenzenesulfonamide **(20)**
• N-{amino-[(pyridin-2-ylmethyl)-amino]-methylene}-4-chlorobenzenesulfonamide **(21)**
• N-{amino-[(pyridin-4-ylmethyl)-amino]-methylene}-4-methylbenzenesulfonamide **(22)**
• N-{amino-[(pyridin-3-ylmethyl)-amino]-methylene}-4-methylbenzenesulfonamide **(23)**
• N-[amino-(pyridin-3-ylamino)-methylene]-4-methylbenzenesulfonamide **(24)**
• *N*-(amino-pyrrolidin-1-yl-methylene)-4-chlorobenzenesulfonamide **(25)**
• *N*-(amino-pyrrolidin-1-yl-methylene)-benzenesulfonamide **(26)**
• *N*-(amino-pyrrolidin-1-yl-methylene)-2-nitrobenzenesulfonamide **(27)**
• *N*-[amino-(morpholin-4-ylamino)-methylene]-4-chlorobenzenesulfonamide **(28)**
• *N*-[amino-(morpholin-4-ylamino)-methylene]-benzenesulfonamide **(29)**
• Naphthalene-1-sulfonic acid amino-(morpholin-4-ylamino)-methylenamide **(30)**
• N-{amino-[(pyridin-2-ylmethyl)-amino]-methylene}-4-bromo-benzenesulfonamide **(32)**
• N-{amino-[(pyridin-2-ylmethyl)-amino]-methylene}-3,4-dichloro-benzenesulfonamide **(33)**
• N-{amino-[(pyridin-2-ylmethyl)-amino]-methylene}-4-isopropyl-benzenesulfonamide **(34)**
• N-{amino-[(pyridin-2-ylmethyl)-amino]-methylene}-4-iodo-benzenesulfonamide **(35)**
• N-{amino-[(pyridin-2-ylmethyl)-amino]-methylene}-benzenesulfonamide **(36)**
• naphthalene-2-sulfonic acid amino-[(pyridin-2-ylmethyl)-amino]-methylenamide **(37)**
• 1-methyl-1H-imidazole-4-sulfonic acid amino-[(pyridin-2-ylmethyl)-amino]-methylenamide **(38)**
• N-[4-(amino-[(pyridin-2-ylmethyl)-amino]-methylene}-sulfamoyl)-phenyl]-acetamide **(39)**
• N-{amino-[(pyridin-2-ylmethyl)-amino]-methylene}-4-fluoro-benzenesulfonamide **(40)**
• N-{amino-[(pyridin-2-ylmethyl)-amino]-methylene}-2,4,6-trimethyl-benzenesulfonamide **(41)**
• N-{amino-[(pyridin-2-ylmethyl)-amino]-methylene}-4-propyl-benzenesulfonamide **(42)**
• N-{amino-[(pyridin-2-ylmethyl)-amino]-methylene}-4-methoxy-benzenesulfonamide **(43)**
• naphthalene-1-sulfonic acid amino-[(pyridin-2-ylmethyl)-amino]-methylenamide **(44)**
• N-{amino-[(pyridin-2-ylmethyl)-amino]-methylene}-3-methyl-benzenesulfonamide **(45)**
• thiophene-2-sulfonic acid amino-[(pyridin-2-ylmethyl)-amino]-methylenamide **(46)**
• quinoline-8-sulfonic acid amino-[(pyridin-2-ylmethyl)-amino]-methylenamide **(47)**
• 5-chloro-3-methyl-benzo[b]thiophene-2-sulfonic acid amino-[(pyridin-2-ylmethyl)-amino]-methylenamide **(48)**
• N-{amino-[(pyridin-2-ylmethyl)-amino]-methylene}-4-tert-butyl-benzenesulfonamide **(49)**
• N-{amino-[(pyridin-2-ylmethyl)-amino]-methylene}-4-butyl-benzenesulfonamide **(50)**
• N-{amino-[(pyrimidin-2-ylmethyl)-amino]-methylene}-4-tert-butyl-benzenesulfonamide **(51)**
• N-{amino-[(furan-2-ylmethyl)-amino]-methylene}-4-butyl-benzenesulfonamide **(52)**
• N-{amino-[(furan-2-ylmethyl)-amino]-methylene}-4-methyl-benzenesulfonamide **(53)**
• N-{amino-[(furan-2-ylmethyl)-amino]-methylene}-4-isopropyl-benzenesulfonamide **(54)**
• N-{amino-[(furan-2-ylmethyl)-amino]-methylene}-4-propyl-benzenesulfonamide **(55)**
• N-{amino-[(furan-2-ylmethyl)-amino]-methylene}-4-tert-butyl-benzenesulfonamide **(56)**
• N-{amino-[(thiophen-2-ylmethyl)-amino]-methylene}-4-butyl-benzenesulfonamide **(57)**
• N-{amino-[(thiophen-2-ylmethyl)-amino]-methylene}-4-propyl-benzenesulfonamide **(58)**
• N-{amino-[(thiophen-2-ylmethyl)-amino]-methylene)-4-isopropyl-benzenesulfonamide **(59)**
• N-{amino-[(thiophen-2-ylmethyl)-amino]-methylene}-4-tert-butyl-benzenesulfonamide **(60)**
• N-{amino-[(thiophen-2-ylmethyl)-amino]-methylene}-4-chloro-benzenesulfonamide **(61)**
• N-{amino-[(thiophen-2-ylmethyl)-amino]-methylene}-4-methyl-benzenesulfonamide **(62)**
• N-{amino-[(furan-2-ylmethyl)-amino]-methylene}-4-chloro-benzenesulfonamide **(63)**
optionally in the form of their racemates, their pure stereoisomers or in the form of mixtures of the stereoisomers in any desired mixture ratio, in the form shown or in the form of their acids or their bases, or in the form of their salts or in the form of their solvates in the two tautomeric forms according to formulae I and Ia, exclusively in one of the tautomeric forms according to formulae I or Ia or also in mixtures of the two forms according to formulae I and Ia, wherein the preferred tautomeric form can vary from compound to compound, e.g. depending on the state of aggregation or on the solvent chosen.

9. Use according to one of claims 1 to 8, **characterized in that** the compound used is present completely or predominantly in the tautomeric form according to formula I.

10. Use according to one of claims 1 to 8, **characterized in that** the compound used is present completely or predominantly in the tautomeric form according to formula Ia.

11. Use according to one of claims 1 to 10, **characterized in that** a sulfonylguanidine used is present as a pure diastereomer and/or enantiomer, as a racemate or as a non-equimolar or equimolar mixture of the diastereomers and/or enantiomers and/or exclusively in one of the tautomeric forms according to formulae I or Ia or also in mixtures of the two forms according to formulae I and Ia.

12. Sulfonylguanidines, **characterized in that** the compounds are chosen from:
• *N*-{amino-[(pyridin-2ylmethyl)-amino]-methylene}-4-methylbenzenesulfonamide **(1)**
• *N*-[amino-(benzylamino-methylene)]-4-methylbenzenesulfonamide **(2)**
• *N*-[amino-phenethylamino-methylene]-4-methylbenzenesulfonamide **(14)**
• *N*-{amino-[(pyridin-2-ylmethyl)-amino]-methylene}-4-chlorobenzenesulfonamide **(21)**
• *N*-{amino-[(pyridin-4-ylmethyl)-amino]-methylene}-4-methylbenzenesulfonamide **(22)**
• *N*-{amino-[(pyridin-3-ylmethyl)-amino]-methylene}-4-methylbenzenesulfonamide **(23)**
• N-{amino-[(pyridin-2-ylmethyl)-amino]-methylene}-4-bromo-benzenesulfonamide **(32)**
• N-{amino-[(pyridin-2-ylmethyl)-amino]-methylene}-3,4-dichloro-benzenesulfonamide **(33)**
• N-{amino-[(pyridin-2-ylmethyl)-amino]-methylene}-4-isopropyl-benzenesulfonamide **(34)**
• N-{amino-[(pyridin-2-ylmethyl)-amino]-methylene}-4-iodo-benzenesulfonamide **(35)**
• N-{amino-[(pyridin-2-ylmethyl)-amino]-methylene}-benzenesulfonamide **(36)**
• naphthalene-2-sulfonic acid amino-[(pyridin-2-ylmethyl)-amino]-methylenamide **(37)**
• 1-methyl-1H-imidazole-4-sulfonic acid amino-[(pyridin-2-ylmethyl)-amino]-methylenamide **(38)**
• N-[4-(amino-[(pyridin-2-ylmethyl)-amino]-methylene}-sulfamoyl)-phenyl]-acetamide **(39)**
• N-{amino-[(pyridin-2-ylmethyl)-amino]-methylene}-4-fluoro-benzenesulfonamide **(40)**
• N-{amino-[(pyridin-2-ylmethyl)-amino]-methylene}-2,4,6-trimethyl-benzenesulfonamide **(41)**
• N-{amino-[(pyridin-2-ylmethyl)-amino]-methylene}-4-propyl-benzenesulfonamide **(42)**
• N-{amino-[(pyridin-2-ylmethyl)-amino]-methylene}-4-methoxy-benzenesulfonamide **(43)**
• naphthalene-l-sulfonic acid amino-[(pyridin-2-ylmethyl)-amino]-methylenamide **(44)**
• N-{amino-[(pyridin-2-ylmethyl)-amino]-methylene}-3-methyl-benzenesulfonamide **(45)**
• thiophene-2-sulfonic acid amino-[(pyridin-2-ylmethyl)-amino]-methylenamide **(46)**
• quinoline-8-sulfonic acid amino-[(pyridin-2-ylmethyl)-amino]-methylenamide **(47)**
• 5-chloro-3-methyl-benzo[b]thiophene-2-sulfonic acid amino-[(pyridin-2-ylmethyl)-amino]-methylenamide **(48)**
• N-{amino-[(pyridin-2-ylmethyl)-amino]-methylene}-4-tert-butyl-benzenesulfonamide **(49)**
• N-famino-[(pyridin-2-ylmethyl)-amino]-methylenel-4-butyl-benzenesulfonamide **(50)**
• N-{amino-[(pyrimidin-2-ylmethyl)-amino]-methylene}-4-tert-butyl-benzenesulfonamide **(51)**
• N-{amino-[(furan-2-ylmethyl)-amino]-methylene}-4-butyl-benzenesulfonamide **(52)**
• N-{amino-[(furan-2-ylmethyl)-amino]-methylene}-4-methyl-benzenesulfonamide **(53)**
• N-{amino-[(furan-2-ylmethyl)-amino]-methylene}-4-isopropyl-benzenesulfonamide **(54)**
• N-{amino-[(furan-2-ylmethyl)-amino]-methylene}-4-propyl-benzenesulfonamide **(55)**
• N-{amino-[(furan-2-ylmethyl)-amino]-methylene}-4-tert-butyl-benzenesulfonamide **(56)**
• N-{amino-[(thiophen-2-ylmethyl)-amino]-methylene}-4-butyl-benzenesulfonamide **(57)**
• N-{amino-[(thiophen-2-ylmethyl)-amino]-methylene}-4-propyl-benzenesulfonamide **(58)**
• N-{amino-[(thiophen-2-ylmethyl)-amino]-methylene)-4-isopropyl-benzenesulfonamide **(59)**
• N-{amino-[(thiophen-2-ylmethyl)-amino]-methylene}-4-tert-butyl-benzenesulfonamide **(60)**
• N-{amino-[(thiophen-2-ylmethyl)-amino]-methylene}-4-chloro-benzenesulfonamide **(61)**
• N-famino-[(thiophen-2-ylmethyl)-amino]-methylenel-4-methyl-benzenesulfonamide **(62)**
• N-{amino-[(furan-2-ylmethyl)-amino]-methylene}-4-chloro-benzenesulfonamide **(63)**
optionally in the form of their racemates, their pure stereoisomers or in the form of mixtures of the stereoisomers in any desired mixture ratio, in the form shown or in the form of their acids or their bases, or in the form of their salts or in the form of their solvates, in the two tautomeric forms according to formulae I and Ia, exclusively in one of the tautomeric forms according to formulae I or Ia or also in mixtures of the two forms according to formulae I and Ia, wherein the preferred tautomeric form can vary from compound to compound, e.g. depending on the state of aggregation or on the solvent chosen.

13. Sulfonylguanidines according to claim 12, **characterized in that** a sulfonylguanidine is present completely or predominantly in the tautomeric form according to formula I.

14. Sulfonylguanidines according to claim 12, **characterized in that** a sulfonylguanidine is present completely or predominantly in the tautomeric form according to formula Ia.

15. Medicaments containing at least one sulfonylguanidine according to one of claims 12 to 14 and optionally suitable additives and/or auxiliary substances and/or optionally further active compounds.

16. Medicaments according to claim 15, **characterized in that** a sulfonylguanidine according to one of claims 12 to 14 contained therein is present as a pure diastereomer and/or enantiomer, as a racemate or as a non-equimolar or equimolar mixture of the diastereomers and/or enantiomers.

## Revendications

1. Utilisation d'une sulfonylguanidine suivant les
formules générales tautomères I et Ia, 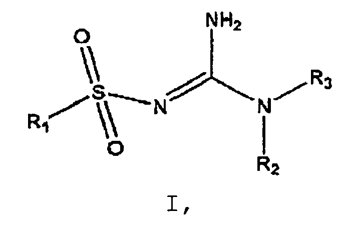 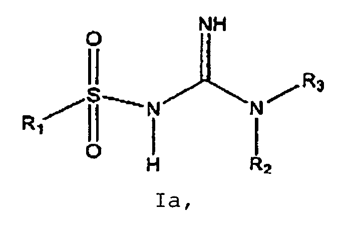 dans laquelle
R¹ est choisi parmi un aryle ou un hétéroaryle, chacun étant non substitué ou bien mono- ou polysubstitué,
et
R² est choisi parmi un alkyle en C₁ à C₁₀ ramifié ou non ramifié, saturé ou insaturé, non substitué ; un cycloalkyle en C₃ à C₈ saturé ou insaturé, non substitué ; un aryle ou un hétéroaryle, chacun non substitué ou bien mono- ou polysubstitué ; ou un aryle, un cycloalkyle en C₃ à C₉ ou un hétéroaryle, chacun non substitué, rattaché par le biais d'un groupe alkyle en C₁ à C₃ saturé ou insaturé, S(O)₂ ou NH ;
ou
NR⁴R⁵,
où R⁴ et R⁵ sont choisis indépendamment l'un de l'autre parmi H ; un alkyle en C₁ à C₁₈ ramifié ou non ramifié, saturé ou insaturé, non substitué ; un cycloalkyle en C₃ à C₈ saturé ou insaturé, non substitué ; un aryle ou un hétéroaryle, chacun non substitué ; ou un aryle, un cycloalkyle en C₃ à C₉ ou un hétéroaryle, chacun non substitué, rattaché par le biais d'un alkyle en C₁ à C₃ ;
ou
R⁴ et R⁵ pris ensemble forment un groupe -CH₂CH₂OCH₂CH₂- ou -CH₂CH₂N(R⁷)CH₂CH₂-, où R⁷ est choisi parmi un alkyle en C₁ à C₁₈ ramifié ou non ramifié, saturé ou insaturé, non substitué ; un cycloalkyle en C₃ à C₈ saturé ou insaturé ou non substitué ; un aryle ou un hétéroaryle, chacun non substitué ; ou un aryle, un cycloalkyle en C₃ à C₉ ou un hétéroaryle, chacun non substitué, rattaché par le biais d'un alkyle en C₁ à C₃ saturé ou insaturé ;
ou
SO₂R⁸,
où R⁸ est choisi parmi un alkyle en C₁ à C₁₈ ramifié ou non ramifié, saturé ou insaturé, non substitué ; un cycloalkyle en C₃ à C₈ saturé ou insaturé, non substitué ; un aryle ou un hétéroaryle, chacun non substitué ; ou un aryle, un cycloalkyle en C₃ à C₉ ou un hétéroaryle, chacun non substitué, rattaché par le biais d'un alkyle en C₁ à C₃ saturé ou insaturé ;
et
R³ est choisi parmi H ou CH₃ ;
ou
R² et R³ pris ensemble forment un groupe -CH₂(CH₂)ₙCH₂-, où n = 2 ou 3, -CH₂CH₂OCN₂CH₂- ou -CH₂CH₂N(R⁸)CH₂CH₂-,
où R⁸ est choisi parmi un alkyle en C₁ à C₁₈ ramifié ou non ramifié, saturé ou insaturé, non substitué ;
le cas échéant sous la forme de leurs racémates, de leurs stéréoisomères purs, ou sous la forme de mélanges des stéréoisomères selon un ratio de mélange quelconque ; sous une forme galénique ou sous la forme de leurs acides ou de leur bases ou sous la forme de leurs sels physiologiquement compatibles, ou sous la forme de leurs solvates ; sous leurs deux formes tautomères suivant les formules I et Ia, exclusivement sous une des formes tautomères suivant les formules I ou Ia ou également dans des mélanges des deux formes suivant les formules I et Ia, la forme tautomère préférée pouvant varier d'un composé à l'autre, par exemple en fonction de l'état des agrégats ou en fonction du solvant choisi ;
pour fabriquer un médicament destiné au traitement de la douleur, de l'épilepsie et/ou de la migraine,
ou
pour fabriquer un médicament destiné au traitement de l'hyperalgésie et de l'allodynie, ou de la douleur inflammatoire ou post-opératoire,
ou
pour fabriquer un médicament destiné au traitement des bouffées de chaleur, des troubles de la post-ménopause, de la sclérose latérale amyotrophique (SLA), de la dystrophie sympathique réflexe (DSR), de la paralysie spastique, du syndrome des jambes sans repos, du nystagmus acquis ; de troubles psychiatriques ou neuropathologiques tels que les troubles bipolaires, l'anxiété, les crises de panique, les fluctuations de l'humeur, les comportements maniaques, les dépressions, les comportements maniaco-dépressifs ; de la neuropathie diabétique douloureuse, des symptômes et des douleurs dus à la sclérose en plaques ou à la maladie de Parkinson, à des affections neurodégénératives telles que la maladie d'Alzheimer, la maladie de Huntington, la maladie de Parkinson et l'épilepsie ; des dommages gastro-intestinaux ; de la douleur érythromélalgique ou post-poliomyélitique, de la névralgie trigéminale ou post-herpétique ; ou destiné à être utilisé comme anti-convulsivant, analgésique ou anxiolytique.

2. Utilisation selon la revendication 1, **caractérisée en ce qu'**elle est faite pour préparer un médicament destiné au traitement de la douleur neuropathique, chronique ou aiguë
ou
de l'hyperalgésie thermique, de l'hyperalgésie mécanique et de l'allodynie et de l'allodynie au froid.

3. Utilisation selon la revendication 1 ou la revendication 2, **caractérisée en ce que**:
R² est choisi parmi un alkyle en C₁ à C₁₀ ramifié ou non ramifié, saturé ou insaturé, non substitué ; un cycloalkyle en C₃ à C₈ saturé ou insaturé, non substitué ; un aryle ou hétéroaryle, chacun non substitué ; ou un aryle, un cycloalkyle en C₃ à C₉ ou un hétéroaryle, chacun non substitué, rattaché par le biais d'un alkyle en C₁ à C₃ saturé ou insaturé.

4. Utilisation selon l'une des revendications 1 à 3, **caractérisée en ce que** R² est choisi parmi un aryle ou un hétéroaryle, chacun non substitué, rattaché par le biais d'un alkyle en C₁ à C₃ saturé ou insaturé.

5. Utilisation selon la revendication 4, **caractérisée en ce que** l'aryle ou l'hétéroaryle est rattaché par le biais d'un groupe -CH₂.

6. Utilisation selon la revendication 4 ou la revendication 5, **caractérisée en ce que**, dans R² l'hétéroaryle rattaché par le biais d'un alkyle en C₁ à C₃ est choisi parmi le pyridinyle, le thiophényle, le furannyle ou le pyrimidinyle, chacun non substitué.

7. Utilisation selon la revendication 6, **caractérisée en ce que** l'hétéroaryle est le pyridinyle.

8. Utilisation d'une sulfonylguanidine selon l'une des revendications 1 à 7, **caractérisée en ce qu'**elle est choisie dans le groupe suivant :
• *N*-{amino[pyridine-2-ylméthyl)amino]méthylène}-4-méthylbenzosulfonamide (1)
• *N*-[amino(benzylaminométhylène)]-4-méthylbenzosulfonamide (2)
• *N*-(aminomorpholine-4-ylméthylène)-4-méthylbenzosulfonamide (3)
• *N*-(aminocyclohexylaminométhylène)-4-méthylbenzosulfonamide (4)
• *N*-(aminophénylaminométhylène)-4-méthylbenzosulfonamide (5)
• *N*-[amino(naphtaline-2-ylamino)méthylène]-4-méthylbenzosulfonamide (7)
• *N*-[amino(*N*'-pyridine-2-ylhydrazino)méthylène]-4-méthylbenzosulfonamide (9)
• *N*-[aminopropylaminométhylène]-4-méthylbenzosulfonamide (10)
• *N*-(aminobutylaminométhylène)-4-méthylbenzosulfonamide (11)
• *N*-[aminopipéridinométhylène]-4-méthylbenzosulfonamide (12)
• *N*-[amino(*N*-méthyl-*N*-phénylamino)méthylène]-4-méthylbenzosulfonamide (13)
• *N*-(aminophénéthylaminométhylène)-4-méthylbenzosulfonamide (14)
• *N*-[amino-sec.-butylaminométhylène]-4-méthylbenzosulfonamide (15)
• *N*-[amino(2H-pyrazole-3-yl-amino)méthylène]-4-méthylbenzosulfonamide (17)
• *N*-(aminopyrrolidine-1-yl-méthylène)-4-méthylbenzosulfonamide (18)
• *N*-[amino(naphtaline-1-yl-amino)méthylène]-4-méthylbenzosulfonamide (19)
• *N*-[amino(morpholine-4-yl-amino)méthylène]-4-méthylbenzosulfonamide (20)
• *N*-{amino[pyridine-2yl-méthyl)amino]méthylène}-4-chlorobenzosulfonamide (21)
• *N*-{amino(pyridine-4-yl-méthyl)amino]méthylène}-4-méthylbenzosulfonamide (22)
• *N*-{amino[pyridine-3-yl-méthyl)amino]méthylène}-4-méthylbenzosulfonamide (23)
• *N*-[amino(pyridine-3-yl-amino)méthylène]-4-méthylbenzosulfonamide (24)
• *N*-(aminopyrrolidine-1-yl-méthylène)-4-chlorobenzosulfonamide (25)
• *N*-(aminopyrrolidine-1-yl-méthylène)benzosulfonamide (26)
• *N*-(aminopyrrolidine-1-yl-méthylène)-2-nitrobenzosulfonamide (27)
• *N*-[amino(morpholine-4-yl-amino)méthylène]-4-chlorobenzosulfonamide (28)
• *N*-[amino(morpholine-4-yl-amino)méthylène]benzosulfonamide (29)
• amino(morpholine-4-yl-amino)méthylène-amide d'acide naphtalène-1-sulfonique (30)
• *N*-{amino[(pyridine-2-yl-méthyl)amino]méthylène}-4-bromobenzosulfonamide (32)
• *N*-{amino[(pyridine-2-yl-méthyl)amino]méthylène}-3,4-dichlorobenzosulfonamide (33)
• *N*-{amino[(pyridine-2-yl-méthyl)amino]méthylène}-4-isopropylbenzosulfonamide (34)
• *N*-{amino[(pyridine-2-yl-méthyl)amino]méthylène}-4-iodo-benzosulfonamide (35)
• *N*-(amino[(pyridine-2-yl-méthyl)amino]méthylène}benzosulfonamide (36)
• amino[(pyridine-2-yl-méthyl)amino]méthylène-amide d'acide naphtalène-2-sulfonique (37)
• amino[(pyridine-2-yl-méthyl)amino]méthylène-amide d'acide 1-méthyl-1H-imidazole-4-sulfonique (38)
• *N*-[4-({amino[(pyridine-2-yl-méthyl)amino]méthylène}sulfamoyl)-phényl]acétamide (39)
• *N*-{amino[(pyridine-2-yl-méthyl)amino]méthylène}-4-fluorobenzosulfonamide (40)
• *N*-{amino[(pyridine-2-yl-méthyl)amino]méthylène}-2,4,6-triméthylbenzosulfonamide (41)
• *N*-{amino[(pyridine-2-yl-méthyl)amino]méthylène}-4-propylbenzosulfonamide (42)
• *N*-{amino[(pyridine-2-yl-méthyl)amino]méthylène}-4-méthoxybenzosulfonamide (43)
• amino[(pyridine-2-yl-méthyl)amino]méthylène-amide d'acide naphtalène-1-sulfonique (44)
• *N*-{amino[(pyridine-2-yl-méthyl)amino]méthylène}-3-méthylbenzosulfonamide (45)
• amino[(pyridine-2-yl-méthyl)amino]méthylène-amide d'acide thiophène-2-sulfonique (46)
• amino[(pyridine-2-yl-méthyl)amino]méthylène-amide d'acide quinoléine-8-sulfonique (47)
• amino[(pyridine-2-yl-méthyl)amino]méthylène-amide d'acide 5-chloro-3-méthylbenzo[b]thiophène-2-sulfonique (48)
• *N*-{amino[(pyridine-2-yl-méthyl)amino]méthylène}-4-tertio-butylbenzosulfonamide (49)
• *N*-{amino[(pyridine-2-yl-méthyl)amino]méthylène}-4-butylbenzosulfonamide (50)
• *N*-{amino[(pyrimidine-2-yl-méthyl)amino]méthylènel-4-tertio-butylbenzosulfonamide (51)
• *N*-{amino[(furanne-2-yl-méthyl)amino]méthylène}-4-butylbenzosulfonamide (52)
• *N*-{amino[(furanne-2-yl-méthyl)amino]méthylène}-4-méthylbenzosulfonamide (53)
• *N*-{amino[(furanne-2-yl-méthyl)amino]méthylène}-4-isopropyl-benzosulfonamide (54)
• *N*-{amino[(furanne-2-yl-méthyl)amino]méthylène}-4-propyl-benzosulfonamide (55)
• *N*-{amino[(furanne-2-yl-méthyl)amino]méthylène}-4-tertio-butylbenzosulfonamide (56)
• *N*-{amino[(thiophène-2-yl-méthyl)amino]méthylène}-4-butylbenzosulfonamide (57)
• *N*-{amino[(thiophène-2-yl-méthyl)amino]méthylène}-4-propyl-benzosulfonamide (58)
• *N*-{amino[(thiophène-2-yl-méthyl)amino]méthylène)-4-isopropyl-benzosulfonamide (59)
• *N*-{amino[(thiophène-2-yl-méthyl)amino]méthylène}-4-tertio-butylbenzosulfonamide (60)
• *N*-{amino[(thiophène-2-yl-méthyl)amino]méthylène}-4-chloro-benzosulfonamide (61)
• *N*-{amino[(thiophène-2-yl-méthyl)amino)méthylène}-4-méthyl-benzosulfonamide (62)
• *N*-{amino[(furanne-2-yl-méthyl)amino]méthylène}-4-chloro-benzosulfonamide (63)
le cas échéant sous la forme de leurs racémates, de leurs stéréoisomères purs, ou sous la forme de mélanges des stéréoisomères selon un ratio de mélange quelconque ; sous une forme galénique ou sous la forme de leurs acides ou de leur bases ou sous la forme de leurs sels physiologiquement compatibles, ou sous la forme de leurs solvates ; sous leurs deux formes tautomères suivant les formules I et Ia, exclusivement sous une des formes tautomères suivant les formules I ou Ia ou également dans des mélanges des deux formes suivant les formules I et Ia, la forme tautomère préférée pouvant varier d'un composé à l'autre, par exemple en fonction de l'état des agrégats ou en fonction du solvant choisi.

9. Utilisation selon l'une des revendications 1 à 8, **caractérisée en ce que** le composé utilisé est présent en totalité ou majoritairement sous la forme tautomère de formule I.

10. Utilisation selon l'une des revendications 1 à 8, **caractérisée en ce que** le composé utilisé est présent en totalité ou majoritairement sous la forme tautomère de formule Ia.

11. Utilisation selon l'une des revendications 1 à 10, **caractérisée en ce qu'**une sulfonylguanidine utilisée est présente sous la forme d'un diastéréoisomère et/ou d'un énantiomère pur, sous forme de racémate ou sous la forme d'un mélange non équimolaire ou équimolaire des diastéréoisomères et/ou énantiomères et/ou exclusivement sous l'une des formes tautomères des formules I ou Ia, ou également sous la forme de mélanges des deux formes des formules I et Ia.

12. Sulfonylguanidines, **caractérisées en ce que** les composés sont choisis parmi les suivants :
• *N*-{amino[pyridine-2-yl-méthyl)amino]méthylène}-4-méthylbenzosulfonamide (1)
• *N*-[amino(benzylaminométhylène)]-4-méthylbenzosulfonamide (2)
• *N*-[aminophénéthylaminométhylène]-4-méthylbenzosulfonamide (14)
• *N*-{amino[pyridine-2yl-méthyl)amino]méthylène}-4-chloro-benzosulfonamide (21)
• *N*-{amino(pyridine-4-yl-méthyl)amino]méthylène}-4-méthyl-benzosulfonamide (22)
• *N*-{amino[pyridine-3-yl-méthyl)amino]méthylène}-4-méthyl-benzosulfonamide (23)
• *N*-{amino[(pyridine-2-yl-méthyl)amino]méthylène}-4-bromo-benzosulfonamide (32)
• *N*-{amino[(pyridine-2-yl-méthyl)amino]méthylène}-3,4-dichloro-benzosulfonamide (33)
• *N*-{amino[(pyridine-2-yl-méthyl)amino]méthylène}-4-isopropyl-benzosulfonamide (34)
• *N*-{amino[(pyridine-2-yl-méthyl)amino]méthylène}-4-iodo-benzosulfonamide (35)
• *N*-(amino[(pyridine-2-yl-méthyl)amino]méthylène}benzosulfonamide (36)
• amino[(pyridine-2-yl-méthyl)amino]méthylène-amide d'acide naphtalène-2-sulfonique (37)
• amino[(pyridine-2-yl-méthyl)amino]méthylène-amide d'acide 1-méthyl-1H-imidazole-4-sulfonique (38)
• *N*-[4-({amino[(pyridine-2-yl-méthyl)amino]méthylène}sulfamoyl)-phényl]acétamide (39)
• *N*-{amino[(pyridine-2-yl-méthyl)amino]méthylène}-4-fluoro-benzosulfonamide (40)
• *N*-{amino[(pyridine-2-yl-méthyl)amino]méthylène}-2,4,6-triméthylbenzosulfonamide (41)
• *N*-{amino[(pyridine-2-yl-méthyl)amino]méthylène}-4-propyl-benzosulfonamide (42)
• *N*-{amino[(pyridine-2-yl-méthyl)amino]méthylène}-4-méthoxy-benzosulfonamide (43)
• amino[(pyridine-2-yl-méthyl)amino]méthylène-amide d'acide naphtalène-1-sulfonique (44)
• *N*-{amino[(pyridine-2-yl-méthyl)amino]méthylène}-3-méthyl-benzosulfonamide (45)
• amino[(pyridine-2-yl-méthyl)amino]méthylène-amide d'acide thiophène-2-sulfonique (46)
• amino[(pyridine-2-yl-méthyl)amino]méthylène-amide d'acide quinoléine-8-sulfonique (47)
• amino[(pyridine-2-yl-méthyl)amino]méthylène-amide d'acide 5-chloro-3-méthylbenzo[b]thiophène-2-sulfonique (48)
• *N*-{amino[(pyridine-2-yl-méthyl)amino]méthylène}-4-tertio-butylbenzosulfonamide (49)
• *N*-{amino[(pyridine-2-yl-méthyl)amino]méthylènel-4-butylbenzosulfonamide (50)
• *N*-{amino[(pyrimidine-2-yl-méthyl)amino]méthylène}-4-tertio-butylbenzosulfonamide (51)
• *N*-{amino[(furanne-2-yl-méthyl)amino]méthylène}-4-butylbenzosulfonamide (52)
• *N*-{amino[(furanne-2-yl-méthyl)amino]méthylène}-4-méthyl-benzosulfonamide (53)
• *N*-{amino[(furanne-2-yl-méthyl)amino]méthylène}-4-isopropyl-benzosulfonamide (54)
• *N*-{amino[(furanne-2-yl-méthyl)amino]méthylène}-4-propyl-benzosulfonamide (55)
• *N*-{amino[(furanne-2-yl-méthyl)amino]méthylène}-4-tertio-butylbenzosulfonamide (56)
• *N*-{amino[(thiophène-2-yl-méthyl)amino]méthylène}-4-butyl-benzosulfonamide (57)
• *N*-{amino[(thiophène-2-yl-méthyl)amino]méthylène}-4-propyl-benzosulfonamide (58)
• *N*-{amino[(thiophène-2-yl-méthyl)amino]méthylène)-4-isopropyl-benzosulfonamide (59)
• *N*-{amino[(thiophène-2-yl-méthyl)amino]méthylène}-4-tertio-butylbenzosulfonamide (60)
• *N*-{amino[(thiophène-2-yl-méthyl)amino]méthylène}-4-chloro-benzosulfonamide (61)
• *N*-{amino[(thiophène-2-yl-méthyl)amino)méthylène}-4-méthyl-benzosulfonamide (62)
• *N*-{amino[(furanne-2-yl-méthyl)amino]méthylène}-4-chloro-benzosulfonamide (63)
le cas échéant sous la forme de leurs racémates, de leurs stéréoisomères purs, ou sous la forme de mélanges des stéréoisomères selon un ratio de mélange quelconque ; sous une forme galénique ou sous la forme de leurs acides ou de leur bases ou sous la forme de leurs sels physiologiquement compatibles, ou sous la forme de leurs solvates ; sous leurs deux formes tautomères suivant les formules I et Ia, exclusivement sous une des formes tautomères suivant les formules I ou Ia ou également dans des mélanges des deux formes suivant les formules I et Ia, la forme tautomère préférée pouvant varier d'un composé à l'autre, par exemple en fonction de l'état des agrégats ou en fonction du solvant choisi.

13. Sulfonylguanidines selon la revendication 12, **caractérisées en ce qu'**une sulfonylguanidine est présente en totalité ou majoritairement sous la forme tautomère de formule I.

14. Sulfonylguanidines selon la revendication 12, **caractérisées en ce qu'**une sulfonylguanidine est présente en totalité ou majoritairement sous la forme tautomère de formule Ia.

15. Médicament contenant au moins une sulfonylguanidine selon l'une des revendications 12 à 14, ainsi que le cas échéant des additifs et/ou adjuvants appropriés et/ou le cas échéant d'autres substances actives.

16. Médicament selon la revendication 15, **caractérisé en ce qu'**une sulfonylguanidine selon l'une des revendications 12 à 14 qu'il contient est présente sous la forme d'un diastéréoisomère et/ou énantiomère pur, sous forme de racémate ou sous la forme d'un mélange non équimolaire ou équimolaire des diastéréoisomères et/ou énantiomères.
